(19) Europäisches Patentamt
European Patent Office
Office européen des brevets

(11) EP 3 058 423 B1

(12) EUROPÄISCHE PATENTSCHRIFT

(45) Veröffentlichungstag und Bekanntmachung des Hinweises auf die Patenterteilung:
**18.04.2018 Patentblatt 2018/16**

(21) Anmeldenummer: **14783851.0**

(22) Anmeldetag: **13.10.2014**

(51) Int Cl.:
*G03F 7/00* (2006.01) *G03F 7/029* (2006.01)
*G03F 7/035* (2006.01) *C07F 5/02* (2006.01)

(86) Internationale Anmeldenummer:
**PCT/EP2014/071877**

(87) Internationale Veröffentlichungsnummer:
**WO 2015/055576 (23.04.2015 Gazette 2015/16)**

# (54) PHOTOPOLYMER-FORMULIERUNG ZUR HERSTELLUNG HOLOGRAPHISCHER MEDIEN MIT BORATEN MIT NIEDRIGER TG

PHOTOPOLYMER FORMULATION FOR PRODUCING HOLOGRAPHIC MEDIA WITH LOW TG BORATES

FORMULATION À BASE DE PHOTOPOLYMÈRE POUR LA FABRICATION DE SUPPORTS HOLOGRAPHIQUES AVEC DES BORATES À BASSE TG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.10.2013 EP 13189138**

(43) Veröffentlichungstag der Anmeldung:
**24.08.2016 Patentblatt 2016/34**

(73) Patentinhaber: **Covestro Deutschland AG**
**51373 Leverkusen (DE)**

(72) Erfinder:
- **BERNETH, Horst**
  **51373 Leverkusen (DE)**
- **RÖLLE, Thomas**
  **51381 Leverkusen (DE)**
- **BRUDER, Friedrich-Karl**
  **47802 Krefeld (DE)**
- **HÖNEL, Dennis**
  **53909 Zülpich-Wichterich (DE)**
- **WEISER, Marc-Stephan**
  **51377 Leverkusen (DE)**
- **FÄCKE, Thomas**
  **51375 Leverkusen (DE)**
- **HAGEN, Rainer**
  **51377 Leverkusen (DE)**
- **WALZE, Günther**
  **51377 Leverkusen (DE)**

(74) Vertreter: **Levpat**
**c/o Covestro AG**
**Gebäude 4825**
**51365 Leverkusen (DE)**

(56) Entgegenhaltungen:
**EP-A1- 0 475 153** **EP-A1- 2 450 893**
**JP-A- 2000 267 273**

- **KULAPINA E G ET AL: "Physicochemical properties of tetraalkylammonium tetraphenylborates and tetraalkylammonium dodecylsulfates", RUSSIAN JOURNAL OF INORGANIC CHEMISTRY, NAUKA/INTERPERIODICA, MO, Bd. 58, Nr. 1, 8. Januar 2013 (2013-01-08) , Seiten 112-116, XP035161785, ISSN: 1531-8613, DOI: 10.1134/S0036023613010129**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 058 423 B1

## Beschreibung

Die Erfindung betrifft eine Photopolymer-Formulierung umfassend eine gegenüber Isocyanaten reaktive Komponente, eine Polyisocyanat-Komponente, ein Schreibmonomer und einen Photoinitiator, enthaltend mindestens einen Farbstoff und einen speziellen Coinitiator.

Weitere Gegenstände der Erfindung sind ein Verfahren zur Herstellung der speziellen Coinitiatoren sowie die nach diesem Verfahren erhältlichen Coinitiatoren, weiterhin ein Verfahren zur Herstellung eines holographischen Mediums unter Verwendung der speziellen Coinitiatoren sowie ein holographisches Medium erhältlich unter Verwendung der erfindungsgemäßen Photopolymer-Formulierung. Des Weiteren betrifft die Erfindung einen Schichtaufbau umfassend ein erfindungsgemäßes holographisches Medium und ebenfalls spezielle, als Coinitiatoren geeignete, Borate.

Ammoniumsalze von Alkyl-triarylboraten als Coinitiatoren sowie ihre Synthese sind bekannt. Bei der technisch auch im großen Maßstab gut durchführbaren Synthese nach folgender Route:

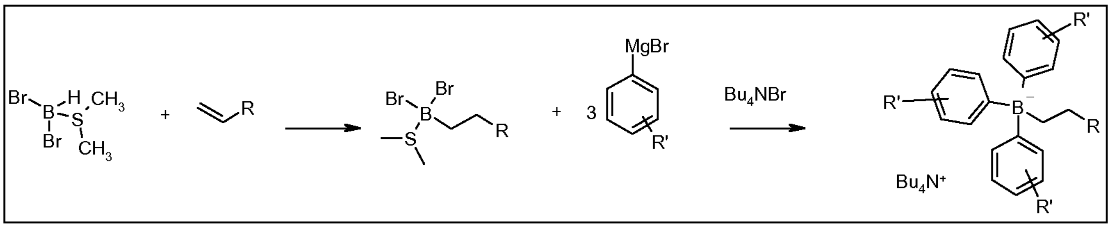


fällt stets eine Mischung aus Alkyltriarylboraten der Formel (A)

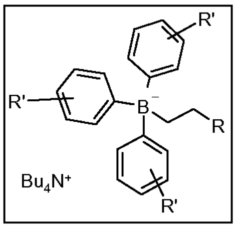
(A)

und Tetraarylboraten der Formel (B)

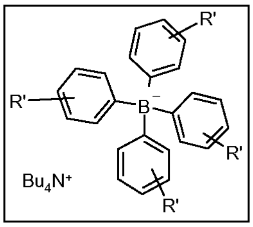
(B)

an, da die Hydroborierung Tribromboran als Katalysator benötigt. Verwendet man dagegen die bei der Synthese anfallende Mischung aus Alkyltriarylboraten und Tetraarylboraten als Coinitiatoren in holographischen Medien, so lassen sich ungenügende Langzeitstabilität und photochemische Bleichbarkeit feststellen. Solche Materialien sind daher für die technische Nutzung unbrauchbar. Die Trennung dieser Mischung ist jedoch aufwändig und führt zwangsläufig zu erheblichen Substanzverlusten.

Aufgabe der vorliegenden Erfindung war daher die Bereitstellung einer Photopolymerzusammensetzung, die einen einfach und kostengünstig herzustellenden Coinitiator umfasst und gleichzeitig dazu geeignet ist holographische Medien herzustellen, die eine gute Langzeitstabilität und photochemische Bleichbarkeit aufweisen. Ebenfalls sollte ein effizientes Verfahren zur Herstellung geeigneter Coinitiatoren bereitgestellt werden.

Diese Aufgabe wurde erfindungsgemäß gelöst durch eine Photopolymer-Formulierung umfassend eine gegenüber Isocyanaten reaktive Komponente, eine Polyisocyanat-Komponente, ein Schreibmonomer und einen Photoinitiator enthaltend mindestens einen Farbstoff und einen Coinitiator, dadurch gekennzeichnet, dass der Coinitiator wenigstens eine Substanz der Formel (Ia) enthält,

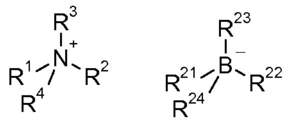

(Ia)

worin

$R^1$ für einen gegebenenfalls verzweigten $C_{14}$- bis $C_{22}$-Alkyl-Rest steht,

$R^2$ für einen gegebenenfalls verzweigten und/oder gegebenenfalls substituierten $C_8$- bis $C_{22}$-Alkyl-Rest, einen Cyclohexyl- oder Cycloheptylrest, einen $C_7$- bis $C_{10}$-Aralkyl-Rest oder einen durch nichtionische Reste substituierten Phenyl-Rest steht und

$R^3$ und $R^4$ unabhängig voneinander für einen gegebenenfalls verzweigten und/oder gegebenenfalls substituierten $C_1$- bis $C_5$-Alkyl-Rest stehen oder

$R^1$ für einen gegebenenfalls verzweigten $C_{14}$- bis $C_{22}$-Alkyl-Rest steht,

$R^2$ für einen gegebenenfalls verzweigten und/oder gegebenenfalls substituierten $C_8$- bis $C_{22}$-Alkyl-Rest oder einen $C_7$- bis $C_{10}$-Aralkyl-Rest steht und

$R^3$ und $R^4$ gemeinsam eine $-(CH_2)_4-$, $-(CH_2)_5-$ oder $-(CH_2)_2-O-(CH_2)_2-$Brücke bilden oder

$R^1$ für einen gegebenenfalls verzweigten $C_{14}$- bis $C_{22}$-Alkyl-Rest steht und

$R^2$, $R^3$ und $R^4$ gemeinsam mit dem $N^+$ einen Imidazol- oder Pyridin-Ring bilden, der mindestens durch einen Rest der Auswahl $C_1$- bis $C_8$-Alkyl, $C_1$- bis $C_8$-Alkoxy, $C_5$- bis $C_7$-Cycloalkyl, Benzyl oder Phenyl substituiert ist

und worin

$R^{21}$ für einen gegebenenfalls substituierten $C_1$- bis $C_{22}$-Alkyl-, $C_3$- bis $C_{22}$-Alkenyl-, $C_5$- bis $C_7$-Cycloalkyl- oder $C_7$- bis $C_{13}$-Aralkylrest steht und
$R^{22}$ bis $R^{24}$ unabhängig voneinander für einen gegebenenfalls durch mindestens einen Rest der Auswahl Halogen, $C_1$- bis $C_4$-Alkyl, Trifluormethyl, $C_1$-bis $C_4$-Alkoxy, Trifluormethoxy, Phenyl oder Phenoxy substituierten $C_6$- bis $C_{10}$-Arylrest stehen.

Es wurde nun überraschend festgestellt, dass die erfindungsgemäßen Photopolymer-Formulierungen zur Herstellung von holographischen Medien geeignet sind, die eine gute Langzeitstabilität und photochemische Bleichbarkeit aufweisen. Zudem sind die erfindungsgemäß eingesetzten Coinitiatoren einfach und kostengünstig herzustellen, da das bei der oben beschriebenen Synthese anfallende Gemisch aus Alkyltriarylboraten und Tetraarylboraten direkt durch einen einfachen Prozess in einen geeigneten Coinitiator umgewandelt werden kann. Die erhaltenen Coinitiatoren können so wie erhalten eingesetzt werden, ohne dass die hergestellten holographischen Medien die oben genannten Nachteile aufweisen. Bevorzugt weisen die Coinitiatoren dabei eine Glasübergangstemperatur $T_g \leq 0$ °C auf.
Die Herstellung solcher Kohlenstoff-substituierter Ammonium-triarylalkylborate, wie sie in bestimmten Ausführungsformen auch zur Verwendung als Coinitiatoren in den erfindungsgemäßen Photopolymer-Formulierungen geeignet sind, ist im Allgemeinen aus dem Stand der Technik, speziell aus der JP 2000267273 bekannt. Die Verwendung solcher Kohlenstoff-substituierten Ammonium-triarylalkylborate für holographische Anwendungen ist im Stand der Technik allerdings nicht beschrieben.
Vorzugsweise weisen die in der erfindungsgemäßen Photopolymer-Formulierung eingesetzten Coinitiatoren eine Glasübergangstemperatur $T_g$ von $\leq 0$ °C auf. Besonders bevorzugt liegt die $T_g \leq -10$ °C, besonders bevorzugt $\leq -15$ °C, ganz besonders bevorzugt $\leq -20$ °C.
In Rahmen dieser Erfindung wird die Glasübergangstemperatur $T_g$ mittels dynamischer Differenzkalorimetrie in Anlehnung an die DIN EN 61006, Verfahren A, bestimmt, wobei ein DSC Gerät verwendet wird, das zur Bestimmung der $T_g$ mit Indium und Blei kalibriert ist und wobei drei aufeinander folgende Durchläufe einer Aufheizungen von -100 °C bis +80 °C für den ersten und -100 °C bis +150 °C für den zweiten und dritten Durchlauf, bei einer konstanten Heizrate von 20 K/min, mit anschließender Abkühlung, bei einer Kühlrate von 50 K/min vorgenommen und die dritte Aufheizkurve zur Bestimmung der Werte verwendet wird. Die $T_g$ wird durch die Temperatur bei der halben Höhe einer Glasübergangsstufe gegeben.

Weitere Details zu bevorzugten Ausführungsformen dieser Messungen sind in den im Beispielteil genannten Methoden angegeben.

Bevorzugt enthalten die erfindungsgemäß eingesetzten Coinitiatoren wenigstens eine Substanz der Formel (Ia), worin

| | |
|---|---|
| $R^1$ | für einen gegebenenfalls verzweigten $C_{14}$- bis $C_{22}$-Alkyl-Rest steht, |
| $R^2$ | für einen gegebenenfalls verzweigten $C_8$- bis $C_{22}$-Alkyl-Rest, einen Cyclohexyl- oder Cycloheptylrest, einen Phenyl-$C_1$- bis $C_3$-alkyl-Rest oder einen durch mindestens einen Rest der Auswahl $C_1$- bis $C_8$-Alkyl, $C_1$- bis $C_8$-Alkoxy, Fluor, Chlor, Trifluormethyl, Trifluormethoxy, $C_5$- bis $C_7$-Cycloalkyl, Benzyl, Phenyl oder Phenoxy substituierten Phenyl-Rest steht und |
| $R^3$ und $R^4$ | unabhängig voneinander für Methyl, Ethyl, Propyl, Butyl, Chlorethyl, Hydroxyethyl oder Cyanethyl stehen oder |
| $R^1$ | für einen gegebenenfalls verzweigten $C_{14}$- bis $C_{22}$-Alkyl-Rest steht, |
| $R^2$ | für einen gegebenenfalls verzweigten $C_8$- bis $C_{22}$-Alkyl-Rest oder einen Phenyl-Ci- bis $C_3$-alkyl-Rest steht und |
| $R^3$ und $R^4$ | gemeinsam eine $-(CH_2)_4-$, $-(CH_2)_5-$ oder $-(CH_2)_2-O-(CH_2)_2-$Brücke bilden oder |
| $R^1$ | für einen gegebenenfalls verzweigten $C_{14}$- bis $C_{22}$-Alkyl-Rest steht und |
| $R^2$, $R^3$ und $R^4$ | gemeinsam mit dem $N^+$-Atom einen Imidazol- oder Pyridin-Ring bilden, der mindestens durch einen Rest der Auswahl $C_1$- bis $C_8$-Alkyl, $C_1$- bis $C_8$-Alkoxy, $C_5$- bis $C_7$-Cycloalkyl, Benzyl oder Phenyl substituiert ist. |

Besonders bevorzugt sind Substanzen der Formel (Ia), worin

| | |
|---|---|
| $R^1$ | für Tetradecyl, Hexadecyl, Octadecyl, Eicosyl oder Docosyl steht, |
| $R^2$ | für Octyl, 2-Ethylhexyl, Nonyl, Decyl, Dodecyl, Tetradecyl, Hexadecyl, Octadecyl, Eicosyl, Docosyl, Cyclohexyl, Cycloheptyl, Benzyl, 2-Phenylethyl, 2- oder 3-Phenylpropyl oder einen durch mindestens einen in 3-, 4- und/oder 5-Stellung stehenden Rest der Auswahl $C_4$- bis $C_8$-Alkyl, $C_4$- bis $C_8$-Alkoxy, Trifluormethyl, Trifluormethoxy, $C_5$- bis $C_7$-Cycloalkyl, Benzyl, Phenyl oder Phenoxy substituierten Phenyl-Rest steht und |
| $R^3$ und $R^4$ | unabhängig voneinander für Methyl oder Ethyl stehen oder |
| $R^1$ | für Tetradecyl, Hexadecyl, Octadecyl, Eicosyl oder Docosyl steht, |
| $R^2$ | für Octyl, 2-Ethylhexyl, Nonyl, Decyl, Dodecyl, Tetradecyl, Hexadecyl, Octadecyl, Eicosyl, Docosyl, Benzyl, 2-Phenylethyl, 2- oder 3-Phenylpropyl steht und |
| $R^3$ und $R^4$ | gemeinsam eine $-(CH_2)_4-$, $-(CH_2)_5-$ oder $-(CH_2)_2-O-(CH_2)_2-$Brücke bilden oder |
| $R^1$ | für Tetradecyl, Hexadecyl, Octadecyl, Eicosyl oder Docosyl steht und |
| $R^2$, $R^3$ und $R^4$ | gemeinsam mit dem $N^+$-Atom einen Imidazol- oder Pyridin-Ring bilden, der mindestens durch einen Rest der Auswahl Methyl, Ethyl, 1- oder 2-Propyl, 1- oder 2-Butyl, 1,1,-Dimethylethyl, 1-Pentyl, 1-Hexyl, 1-Octyl, Methoxy, Ethoxy, 1- oder 2-Propoxy, 1- oder 2-Butoxy, 1,1,-Dimethylethoxy, 1-Pentoxy, 1-Hexyloxy, Cyclopentyl, Cyclohexyl, Benzyl oder Phenyl substituiert ist. |

Ganz besonders bevorzugt sind Substanzen der Formel (Ia), worin

| | |
|---|---|
| $R^1$ | für Hexadecyl oder Octadecyl steht, |
| $R^2$ | für Octyl, 2-Ethylhexyl, Decyl, Dodecyl, Tetradecyl, Hexadecyl, Octadecyl, Benzyl, 3-Phenylpropyl oder einen durch mindestens einen in 3-, 4- und/oder 5-Stellung stehenden Rest der Auswahl 1- oder 2-Butyl, 1,1-Dimethylethyl, 1-Pentyl, 1-Hexyl, 1-Octyl, 1- oder 2-Butoxy, 1,1-Dimethylethoxy, 1-Pentoxy, 1-Hexoxy, 1-Octoxy, Trifluormethyl, Trifluormethoxy, Cyclohexyl, Benzyl, Phenyl oder Phenoxy substituierten Phenyl-Rest steht und |
| $R^3$ und $R^4$ | unabhängig voneinander für Methyl stehen oder |
| $R^1$ | für Hexadecyl oder Octadecyl steht, |
| $R^2$ | für Octyl, 2-Ethylhexyl, Decyl, Dodecyl, Tetradecyl, Hexadecyl, Octadecyl, Benzyl oder 3-Phenylpropyl steht und |
| $R^3$ und $R^4$ | gemeinsam eine $-(CH_2)_4-$, $-(CH_2)_5-$ oder $-(CH_2)_2-O-(CH_2)_2-$Brücke bilden oder |
| $R^1$ | für Hexadecyl oder Octadecyl steht und |
| $R^2$, $R^3$ und $R^4$ | gemeinsam mit dem $N^+$-Atom einen Imidazol-Ring, dessen zweites N-Atom durch 1- oder 2-Butyl, 1- |

Pentyl, 1-Hexyl, 1-Octyl, Cyclopentyl, Cyclohexyl, Benzyl oder Phenyl substituiert ist, oder Pyridin-Ring bilden, dessen Ring mindestens durch einen Rest der Auswahl 1- oder 2-Butyl, 1,1,-Dimethylethyl, 1-Pentyl, 1-Hexyl, 1- oder 2-Butoxy, 1,1,-Dimethylethoxy, 1-Pentoxy, 1-Hexyloxy, Cyclopentyl, Cyclohexyl, Benzyl oder Phenyl substituiert ist.

Bevorzugt werden in der erfindungsgemäßen Photopolymer-Formulierung Coinitiatoren enthaltend wenigstens eine Substanz der Formel (Ia) eingesetzt deren Reste $R^{22}$ bis $R^{24}$ gleich sind. Des Weiteren bevorzugt stehen die Substituenten in den Resten $R^{22}$ bis $R^{24}$ in 3-, 4- und/oder 5-Stellung, besonders bevorzugt in 3- oder 4- Stellung oder in 3,4-Stellung.

Bevorzugt sind außerdem Coinitiatoren enthaltend wenigstens eine Substanz der Formel (Ia) wobei

$R^{21}$ für einen gegebenenfalls verzweigten und gegebenenfalls durch Fluor, Chlor, Methoxy, Ethoxy oder Cyano substituierten $C_2$- bis $C_{18}$-Alkyl-, $C_3$- bis $C_{12}$-Alkenyl- oder $C_7$- bis $C_{10}$-Aralkylrest, Cyclopentyl oder Cyclohexyl steht und

$R^{22}$ bis $R^{24}$ unabhängig voneinander für einen durch mindestens einen Rest der Auswahl Fluor, Chlor, $C_1$-bis $C_4$-Alkyl, Trifluormethyl, $C_1$-bis $C_4$-Alkoxy, Trifluormethoxy, Phenyl oder Phenoxy substituierten $C_6$- bis $C_{10}$- Arylrest stehen.

Besonders bevorzugt sind Coinitiatoren enthaltend wenigstens eine Substanz der Formal (Ia) wobei

$R^{21}$ für 1- oder 2-Butyl, 1,1-Dimethylethyl, 1- oder 2-Pentyl, 1- oder 2-Hexyl, 1- oder 2-Heptyl, 1- oder 2-Octyl, 2-Ethylhexyl, 1- oder 2- Nonyl, 1- oder 2-Decyl, 1-Dodecyl, 1-Tetradecyl, 1-Hexadecyl, 1-Octadecyl, Allyl, 2-Buten-1-yl, Benzyl, 2-Phenylethyl, 2- oder 3-Phenylpropyl, Cyclopentyl oder Cyclohexyl steht und

$R^{22}$ bis $R^{24}$ unabhängig voneinander für einen gegebenenfalls durch mindestens einen Rest der Auswahl Fluor, Chlor, Methyl, tert.-Butyl, Trifluormethyl, Methoxy oder Trifluormethoxy in 3- und/oder 4-Stellung substituierten Phenylrest stehen.

Ganz besonders bevorzugt sind Coinitiatoren enthaltend wenigstens eine Substanz der Formel (Ia) wobei

$R^{21}$ für 1-Butyl, 1- Pentyl, 1-Hexyl, 1-Heptyl, 1-Octyl, 1-Nonyl, 1-Decyl, 1-Dodecyl, 1-Tetradecyl, 1-Hexadecyl, 1-Octadecyl, Allyl, 3-Phenylpropyl, Cyclopentyl oder Cyclohexyl steht und

$R^{22}$ bis $R^{24}$ für 4-Fluorphenyl, 4-Chlorphenyl, 4-tert.-Butylphenyl, 3-Fluor-4-methylphenyl oder 3-Chlor-4-methylphenyl stehen und

$R^{22}$ bis $R^{24}$ gleich sind.

Des Weiteren bevorzugt sind Coinitiatoren enthaltend wenigstens eine Substanz der Formel (Ia) wobei

$R^{21}$ für 1-Butyl, 1-Hexyl, 1-Octyl, 1-Dodecyl oder 3-Phenylpropyl steht,

$R^{22}$ bis $R^{24}$ für 4-Fluorphenyl, 4-Chlorphenyl, 3-Fluor-4-methylphenyl oder 3-Chlor-4-methylphenyl stehen und

$R^{22}$ bis $R^{24}$ gleich sind.

In einer bevorzugten Ausfiihrungsform der Erfindung enthalten die in der erfindungsgemäßen Photopolymer-Formulierungen eingesetzten Coinitiatoren, weiterhin wenigstens eine Substanz der Formel (Ib),

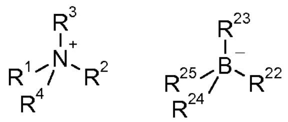

(Ib)

worin $R^1$ bis $R^4$ definiert sind wie in Anspruch 1 und

$R^{22}$ bis $R^{25}$ unabhängig voneinander für einen gegebenenfalls durch mindestens einen Rest der Auswahl Halogen, $C_1$-bis $C_4$-Alkyl, Trifluormethyl, $C_1$-bis $C_4$-Alkoxy, Trifluormethoxy, Phenyl oder Phenoxy substituierten $C_6$- bis $C_{10}$-Arylrest stehen.

Die erfindungsgemäß eingesetzten Coinitatoren enthalten die Substanzen der Formeln (Ia) und (Ib) bevorzugt im molaren Verhältnis 80:20 bis 99,99:0,01, besonders bevorzugt 90:10 bis 99,95:0,05, ganz besonders bevorzugt 95:5 bis 99,9:0,1 und des Weiteren bevorzugt 97:3 bis 99,9:0,1.

In einer bevorzugten Ausführungsform besteht der Coinitiator aus der Substanz der Formel (Ia) oder einem

Gemisch der Substanzen der Formeln (Ia) und (Ib).

Für die Reste $R^1$ bis $R^4$ sowie $R^{22}$ bis $R^{24}$ der Substanzen der Formel (Ib) gelten ebenfalls die oben für die Substanzen der Formel (Ia) angeführten bevorzugten Ausführungsformen. Für $R^{25}$ der Formel (Ib) gelten ebenfalls die oben bezüglich Substanzen der Formal (Ia) aufgeführten bevorzugten Ausführungsformen für $R^{22}$, $R^{23}$ oder $R^{24}$.

In einer weiteren bevorzugten Ausführungsform der Erfindung enthält der Coinitiator weiterhin auch Salze der Formel (II),

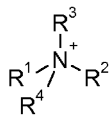 $An^-$ (II)

worin

$An^-$ für ein Anion mit einem AClogP im Bereich von 2 bis 8, vorzugsweise 3 bis 6, besonders bevorzugt 3,5 bis 5 steht und $R^1$ bis $R^4$ die oben für die Substanzen der Formel (Ia) angegebene Bedeutung besitzen.

Für $R^1$ bis $R^4$ der Salze der Formel (II) gelten ebenfalls die oben für Substanzen der Formel (Ia) angeführten bevorzugten Ausführungsformen bezüglich $R^1$ bis $R^4$.

Überraschenderweise wurde gefunden, dass sich besonders Anionen $An^-$ eignen, die einen AClogP im Bereich von 2 bis 8, vorzugsweise 3 bis 6, besonders bevorzugt 3,5 bis 5 aufweisen.

Der AClogP wird nach J. Comput. Aid. Mol. Des. 2005, 19, 453; Virtual Computational Chemistry Laboratory, http://www.vcclab.org berechnet.

Die erfindungsgemäß eingesetzten Coninitatoren können deshalb Mischungen aus Boraten und Salzen mit Anionen der Formel $An^-$ mit den erfindungsgemäßen Ammoniumkationen enthalten oder daraus bestehen. In einer bevorzugten Ausführungsform der Erfindung enthält der Coinitiator eine Mischung von Substanzen der Formeln (Ia), (Ib) und (II) oder Substanzen der Formeln (Ia) und (II), weiterhin bevorzugt besteht der Coinitiator aus einer Mischung von Substanzen der Formeln (Ia), (Ib) und (II) oder Substanzen der Formeln (Ia) und (II).

Der Coinitiator enthält bevorzugt 0,01 bis 10 Gew.-%, besonders bevorzugt 0,05 bis 7 Gew.-% und ganz besonders bevorzugt 1 bis 5 Gew.-% der Salze der Formel (II), bezogen auf die Gesamtmasse des Coinitiators.

Beispiele für geeignete Anionen $An^-$ sind:

| Anion | AClogP |
|---|---|
| 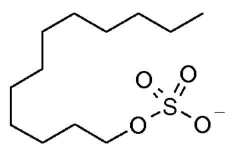 | 3,05 |
| 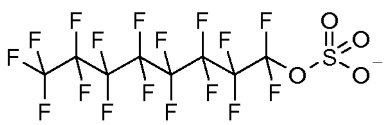 | 3,32 |
| 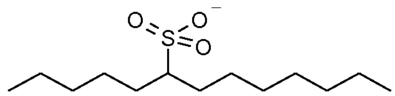 | 3,45 |
| 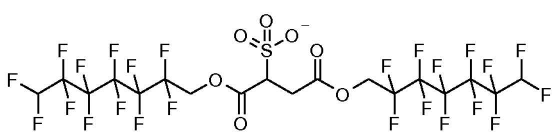 | 3,62 |

(fortgesetzt)

| Anion | AClogP |
|---|---|
| 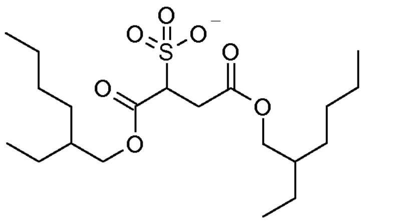 | 3,67 |
| 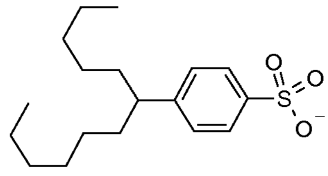 | 4,85 |
| 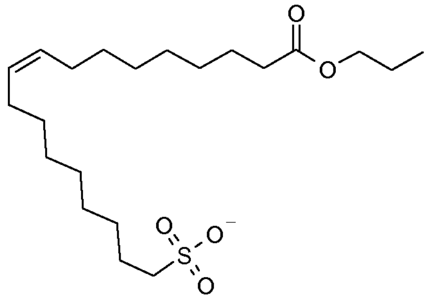 | 5,78 |

Bei einer weiteren bevorzugten Ausführungsform der Erfindung ist vorgesehen, dass das Anion $An^-$ eine Molmasse M ≥150 g/mol und besonders bevorzugt ≥ 250 g/mol aufweist.

Das Anion der Formel $An^-$ kann vorzugsweise wenigstens ein Phosphor- oder Schwefel-Atom, bevorzugt wenigstens ein Schwefel-Atom und besonders bevorzugt ein Schwefel-Atom in einer $SO_3$-Gruppierung umfassen.

Ebenfalls bevorzugt kann das Anion $An^-$ mindestens einen linearen oder verzweigten aliphatischen Rest, bevorzugt einen linearen oder verzweigten aliphatischen $C_8$ bis $C_{18}$-Rest aufweisen. Enthält das Anion mehr als einen linearen oder verzweigten aliphatischen Rest, so enthalten diese zusammen 8 bis 36, vorzugsweise 8 bis 24 C-Atome. Dieser aliphatische Rest kann Substituenten tragen wie Fluor, Methoxy oder Ethoxy.

Ganz besonders bevorzugte Anionen der Formel $An^-$ besitzen folglich eine Molmasse ≥ 250 g/mol und enthalten eine $SO_3^-$-Gruppierung sowie mindestens eine Alkylgruppe mit mindestens 8 C-Atomen und haben eine AClogP im Bereich 3 bis 6, vorzugsweise 3,5 bis 5.

Die erfindungsgemäßen Anionen der Formel $An^-$ umfassen insbesondere auch die folgenden Substanzen, sofern ihr AClogP-Wert im Bereich 2 bis 8 liegt:

$C_8$- bis $C_{25}$-Alkansulfonat, vorzugsweise $C_{13}$- bis $C_{25}$-Alkansulfonat, $C_6$- bis $C_{18}$-Perfluoralkansulfonat, vorzugsweise $C_6$- bis $C_{18}$-Perfluoralkansulfonat, $C_9$- bis $C_{25}$-Alkanoat, $C_9$- bis $C_{25}$-Alkenoat, $C_8$- bis $C_{25}$-Alkylsulfat, vorzugsweise $C_{13}$- bis $C_{25}$-Alkylsulfat, $C_8$- bis $C_{25}$-Alkenylsulfat, vorzugsweise $C_{13}$- bis $C_{25}$-Alkenylsulfat, $C_6$- bis $C_{18}$-Perfluoralkylsulfat, vorzugsweise $C_4$- bis $C_{18}$-Perfluoralkylsulfat, Polyethersulfate basierend auf mindestens 4 Äquivalenten Ethylenoxid und/oder Äquivalenten 4 Propylenoxid, Bis-$C_4$- bis $C_{25}$-Alkyl-, $C_5$- bis $C_7$-Cycloalkyl-, $C_3$- bis Cs-Alkenyl- oder $C_7$- bis $C_{11}$-Aralkyl-sulfosuccinat, durch mindestens 8 Fluoratome substituiertes Bis-$C_2$- bis $C_{10}$-Alkylsulfosuccinat, $C_8$- bis $C_{25}$-Alkyl-sulfoacetate, durch mindestens einen Rest der Gruppe $C_8$- bis $C_{25}$-Alkyl, Perfluor-$C_6$-bis $C_{12}$-Alkyl und/oder $C_8$- bis $C_{18}$-Alkoxycarbonyl substituiertes Benzolsulfonat, sulfonierte oder sulfatierte, ggf. mindestens einfach ungesättigte $C_8$- bis $C_{25}$-Fettsäureester von aliphatischen $C_1$- bis $C_8$-Alkoholen oder Glycerin, Bis-(sulfo-$C_2$- bis $C_6$-alkyl)-$C_3$- bis $C_{12}$-alkandicarbonsäureester, Bis-(sulfo-$C_2$-bis $C_6$-alkyl)-itaconsäureester, (Sulfo-$C_2$- bis $C_6$-alkyl)-$C_6$- bis $C_{18}$-alkancarbonsäureester, (Sulfo-$C_2$- bis $C_6$-alkyl)-acryl- oder methacrylsäureester,-wobei bei mehrwertigen Anionen An- für ein Äquivalent dieses Anions steht, und wobei die Alkan- und Alkylgruppen verzweigt sein können und/oder durch Halogen, Cyano, Methoxy, Ethoxy, Methoxycarbonyl oder Ethoxycarbonyl sustituiert sein können.

Besonders bevorzugt sind:

Sec-$C_{11}$- bis $C_{18}$-Alkansulfonat, $C_{13}$- bis $C_{25}$-Alkylsulfat, verzweigtes $C_8$- bis $C_{25}$-Alkylsulfat, ggf. verzweigtes Bis-$C_6$- bis $C_{25}$-Alkylsulfosuccinat, Bis-cyclopentyl- oder -cyclohexylsulfosuccinat, sec- oder tert.-$C_8$- bis $C_{25}$-Alkylbenzolsulfonat, sulfonierte oder sulfatierte, ggf. mindestens einfach ungesättigte $C_8$-bis $C_{25}$-Fettsäureester von aliphatischen $C_1$- bis $C_8$-Alkoholen oder Glycerin, Bis-(sulfo-$C_2$- bis $C_6$-alkyl)-$C_3$- bis $C_{12}$-alkandicarbonsäureester, (Sulfo-$C_2$- bis $C_6$-alkyl)-$C_6$- bis $C_{18}$-alkancarbonsäureester.

Ganz besonders bevorzugt sind: Bis-(1-hexyl)-sulfosuccinat, Bis-(1-octyl)-sulfosuccinat, Bis-(2-ethylhexyl)-sulfosuccinat, Biscyclohexylsulfosuccinat, 4-n-Dodecylbenzolsulfonat, 4-s-Dodecylbenzolsulfonat, 4-verzweigt-Dodecylbenzolsulfonat, Dodecylsulfat, Tetradecylsulfat, Hexadecylsulfat, Octadecylsulfat.

Photopolymer-Formulierungen in einer weiteren Ausführungsform sind dadurch gekennzeichnet, dass der Coinitiator weiterhin Substanzen der Formeln (IIIa) und gegebenenfalls (IIIb) enthält,

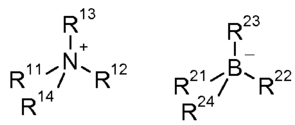

(IIIa)

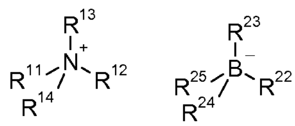

(IIIb),

worin

$R^{11}$ bis $R^{14}$ unabhängig voneinander für $C_1$- bis $C_4$-Alkyl stehen und
$R^{21}$ bis $R^{24}$ die oben für Formal (Ia) genannte Bedeutung und $R^{25}$ die oben für Formel (Ib) genannte Bedeutung besitzen.

Dabei gelten für $R^{21}$ bis $R^{25}$ ebenfalls die oben für $R^{21}$ bis $R^{25}$ bezüglich der Formeln (Ia) und (Ib) angegebenen bevorzugten Ausführungsformen.

Die erfindungsgemäß eingesetzten Coinitatoren enthalten die Substanzen der Formeln (IIIa) und (IIIb) bevorzugt im molaren Verhältnis 80:20 bis 99,99:0,01, besonders bevorzugt 90:10 bis 99,95:0,05, ganz besonders bevorzugt 95:5 bis 99,9:0,1 und des Weiteren bevorzugt 97:3 bis 99,9:0,1.

Dabei enthält der Coinitiator die Substanzen (IIIa) und (IIIb) bevorzugt im gleichen molaren Verhältnis zueinander in dem auch die Substanzen (Ia) und (Ib) zueinander vorliegen.

In einer bevorzugten Ausführungsform der Erfindung besteht der Coinitiator aus einer Mischung der Substanzen der Formeln (Ia), (Ib), (IIIa) und (IIIb) oder (Ia) und (IIIa), besonders bevorzugt aus einer Mischung der Substanzen (Ia) und (IIIa).

Das molare Verhältnis der Substanzen (IIIa) und gegebenenfalls (IIIb) zu der Summe der Substanzen (Ia) und gegebenenfalls (Ib) liegt bevorzugt ≤ 15:85, besonders bevorzugt ≤ 10:90, ganz besonders bevorzugt ≤ 5:95 und weiterhin bevorzugt ≤ 2:98. Vorzugsweise beträgt das molare Verhältnis der Substanzen (IIIa) und gegebenenfalls (IIIb) zu der Summe der Substanzen (Ia) und gegebenenfalls (Ib) aber ≥ 0,01:99,99, besonders bevorzugt ≥ 0,05:99,95 und ganz besonders bevorzugt ≥ 0,10:99,90.

Der erfindungsgemäß eingesetzte Coinitiator kann auch eine Mischung der Substanzen der Formeln (Ia), (Ib), (IIIa) und (IIIb) mit Salzen der Formel (II) enthalten oder daraus bestehen. Ebenso kann der erfindungsgemäß eingesetzte Coinitiator eine Mischung von Substanzen der Formeln (Ia) und (IIIa) mit Salzen der Formel (II) enthalten oder daraus bestehen. Auch diese Mischungen enthalten bevorzugt 0,01 bis 10 Gew.-%, besonders bevorzugt 0,05 bis 7 Gew.-% und ganz besonders bevorzugt 1 bis 5 Gew.-% der Salze der Formel (II) bezogen auf die Gesamtmasse des Coinitiators.

**Photopolymer-Formulierung**

Geeignete Photopolymer-Formulierungen, die für die Herstellung einer Photopolymerschicht geeignet sind, sind dem Fachmann ebenfalls bekannt und beispielsweise in WO-A 2011/054797 und WO 2011/067057 beschrieben. Dabei handelt es sich bei der Photopolymer-Formulierung für die Herstellung der Photopolymerschicht um eine solche

umfassend eine Polyisocyanat-Komponente a), eine isocyanatreaktive Komponente b), ein Schreibmonomer und einen Photoinitiator.

Die Polyisocyanat-Komponente a) umfasst wenigstens eine organische Verbindung mit wenigstens zwei NCO-Gruppen. Bei diesen organischen Verbindungen kann es sich insbesondere um monomere Di- und Triisocyanate, Polyisocyanate und / oder NCO-funktionelle Präpolymere handeln. Die Polyisocyanat-Komponente a) kann auch Mischungen monomerer Di- und Triisocyanate, Polyisocyanate und / oder NCO-funktioneller Präpolymere enthalten oder daraus bestehen.

Als monomere Di- und Triisocyanate können alle dem Fachmann an sich gut bekannten Verbindungen oder deren Mischungen eingesetzt werden. Diese Verbindungen können aromatische, araliphatische, aliphatische oder cycloaliphatische Strukturen aufweisen. In untergeordneten Mengen können die monomeren Di- und Triisocyanate auch Monoisocyanate, d.h. organische Verbindungen mit einer NCO-Gruppe umfassen.

Beispiele für geeignete monomere Di- und Triisocyanate sind 1,4-Butandiisocyanat, 1,5-Pentandiisocyanat, 1,6-Hexandiisocyanat (Hexamethylendiisocyanat, HDI), 2,2,4-Trimethylhexamethylendiisocyanat und / oder 2,4,4-Trimethylhexamethylendiisocyanat (TMDI), Isophorondiisocyanat (IPDI), 1,8-Diiso-cyanato-4-(isocyanatomethyl)-octan, Bis-(4,4'-isocyanatocyclohexyl)methan und / oder Bis-(2',4-isocyanatocyclohexyl)methan und / oder deren Mischungen beliebigen Isomerengehalts, 1,4-Cyclohexandiisocyanat, die isomeren Bis-(isocyanatomethyl)cyclohexane, 2,4- und / oder 2,6-Diisocyanato-1-methylcyclohexan (Hexahydro-2,4- und / oder 2,6-toluylendiisocyanat, $H_6$-TDI), 1,4-Phenylendiisocyanat, 2,4- und / oder 2,6-Toluylendiisocyanat (TDI), 1,5-Naphthylendiisocyanat (NDI), 2,4'- und / oder 4,4'-Diphenylmethandiisocyanat (MDI), 1,3-Bis(isocyanatomethyl)benzol (XDI) und / oder das analoge 1,4-Isomere oder beliebige Mischungen der vorgenannten Verbindungen.

Geeignete Polyisocyanate sind auch Verbindungen mit Urethan-, Harnstoff-, Carbodiimid-, Acylharnstoff-, Amid-, Isocyanurat-, Allophanat-, Biuret-, Oxadiazintrion-, Uretdion- und/oder Iminooxadiazindionstrukturen, die aus den vorgenannten Di- oder Triisocyanaten erhältlich sind.

Besonders bevorzugt handelt es sich bei den Polyisocyanaten um oligomerisierte aliphatische und / oder cycloaliphatische Di- oder Triisocyanate, wobei insbesondere die oben stehenden aliphatischen und / oder cycloaliphatischen Di- oder Triisocyanate verwendet werden können.

Ganz besonders bevorzugt sind Polyisocyanate mit Isocyanurat-, Uretdion- und / oder Iminooxadiazindion-Strukturen sowie Biurete basierend auf HDI oder deren Mischungen.

Geeignete Präpolymere enthalten Urethan- und / oder Harnstoff-Gruppen sowie gegebenenfalls weitere durch Modifizierung von NCO-Gruppen entstandene Strukturen wie oben genannt. Derartige Präpolymere sind beispielsweise durch Umsetzung der oben genannten monomeren Di- und Triisocyanate und / oder Polyisocyanaten a1) mit isocyanatreaktiven Verbindungen b1) erhältlich.

Als isocyanatreaktive Verbindungen b1) können Alkohole, Amino oder Mercapto-Verbindungen, bevorzugt Alkohole, verwendet werden. Dabei kann es sich insbesondere um Polyole handeln. Ganz besonders bevorzugt können als isocyanatreaktive Verbindung b1) Polyester-, Polyether-, Polycarbonat-, Poly(meth)acrylat- und/oder Polyurethan-Polyole verwendet werden.

Als Polyesterpolyole sind beispielsweise lineare Polyesterdiole oder verzweigte Polyesterpolyole geeignet, die in bekannter Weise durch Umsetzung von aliphatischen, cycloaliphatischen oder aromatischen Di- bzw. Polycarbonsäuren bzw. ihren Anhydriden mit mehrwertigen Alkoholen einer OH-Funktionalität $\geq 2$ erhalten werden können. Beispiele für geeignete Di- bzw. Polycarbonsäuren sind mehrwertige Carbonsäuren wie Bernstein-, Adipin-, Kork-, Sebacin-, Decandicarbon-, Phthal-, Terephthal-, Isophthal- Tetrahydrophthal- oder Trimellithsäure sowie Säureanhydride wie Phthal-, Trimellith- oder Bernsteinsäureanhydrid oder deren beliebige Gemische untereinander. Die Polyesterpolyole können auch auf natürlichen Rohstoffen wie Rizinusöl basieren. Es ist ebenfalls möglich, dass die Polyesterpolyole auf Homo- oder Mischpolymerisaten von Lactonen basieren, die bevorzugt durch Anlagerung von Lactonen bzw. Lactongemischen wie Butyrolacton, $\varepsilon$-Caprolacton und / oder Methyl-$\varepsilon$-caprolacton an hydroxyfunktionelle Verbindungen wie mehrwertige Alkohole einer OH-Funktionalität $\geq 2$ beispielsweise der nachstehend genannten Art erhalten werden können.

Beispiele für geeignete Alkohole sind alle mehrwertigen Alkohole wie z.B. die $C_2$ - $C_{12}$-Diole, die isomeren Cyclohexandiole, Glycerin oder deren beliebige Gemische untereinander.

Geeignete Polycarbonatpolyole sind in an sich bekannter Weise durch Umsetzung von organischen Carbonaten oder Phosgen mit Diolen oder Diol-Mischungen zugänglich.

Geeignete organische Carbonate sind Dimethyl-, Diethyl- und Diphenylcarbonat.

Geeignete Diole bzw. Mischungen umfassen die an sich im Rahmen der Polyestersegmente genannten mehrwertigen Alkohole einer OH-Funktionalität $\geq 2$, bevorzugt Butandiol-1,4, Hexandiol-1,6 und / oder 3-Methylpentandiol. Auch Polyesterpolyole können zu Polycarbonatpolyolen umgearbeitet werden.

Geeignete Polyetherpolyole sind gegebenenfalls blockweise aufgebaute Polyadditionsprodukte cyclischer Ether an OH- oder NH-funktionelle Startermoleküle.

Geeignete cyclische Ether sind beispielsweise Styroloxide, Ethylenoxid, Propylenoxid, Tetrahydrofuran, Butylenoxid, Epichlorhydrin sowie ihre beliebigen Mischungen.

Als Starter können die an sich im Rahmen der Polyesterpolyole genannten mehrwertigen Alkohole einer OH-Funktionalität ≥ 2 sowie primäre oder sekundäre Amine und Aminoalkohole verwendet werden.

Bevorzugte Polyetherpolyole sind solche der vorgenannten Art ausschließlich basierend auf Propylenoxid oder statistische oder Block-Copolymere basierend auf Propylenoxid mit weiteren 1-Alkylenoxiden. Besonders bevorzugt sind Propylenoxid-homopolymere sowie statistische oder Block-Copolymere, die Oxyethylen-, Oxypropylen- und / oder Oxybutyleneinheiten aufweisen, wobei der Anteil der Oxypropyleneinheiten bezogen auf die Gesamtmenge aller Oxyethylen-, Oxypropylen- und Oxybutyleneinheiten mindestens 20 Gew.-%, bevorzugt mindestens 45 Gew.-% ausmacht. Oxypropylen- und Oxybutylen umfasst hierbei alle jeweiligen linearen und verzweigten $C_3$- und $C_4$-Isomere.

Daneben sind als Bestandteile der Polyol-Komponente b1) als polyfunktionelle, isocyanatreaktive Verbindungen auch niedermolekulare, d.h. mit Molekulargewichten ≤ 500 g/mol, kurzkettige, d.h. 2 bis 20 Kohlenstoffatome enthaltende aliphatische, araliphatische oder cycloaliphatische di-, tri- oder polyfunktionelle Alkohole geeignet.

Dies können beispielsweise in Ergänzung zu den oben genannten Verbindungen Neopentylglykol, 2-Ethyl-2-butylpropandiol, Trimethylpentandiol, stellungsisomere Diethyloctandiole, Cyclohexandiol, 1,4-Cyclohexandimethanol, 1,6-Hexandiol, 1,2- und 1,4-Cyclohexandiol, hydriertes Bisphenol A, 2,2-Bis(4-hydroxy-cyclohexyl)-propan oder 2,2-Dimethyl-3-hydroxypropionsäure, 2,2-dimethyl-3-hydroxypropyl-ester sein. Beispiele geeigneter Triole sind Trimethylolethan, Trimethylolpropan oder Glycerin. Geeignete höherfunktionelle Alkohole sind Di-(trimethylolpropan), Pentaerythrit, Dipentaerythrit oder Sorbit.

Besonders bevorzugt ist, wenn die Polyolkomponente ein difunktioneller Polyether-, Polyester oder ein Polyether-polyester-block-copolyester oder ein Polyether-Polyester-Blockcopolymer mit primären OH-Funktionen ist.

Es ist ebenfalls möglich, als isocyanatreaktive Verbindungen b1) Amine einzusetzen. Beispiele geeigneter Amine sind Ethylendiamin, Propylendiamin, Diaminocyclohexan, 4,4'-Dicylohexylmethandiamin, Isophorondiamin (IPDA), difunktionelle Polyamine wie z.B. die Jeffamine®, aminterminierte Polymere, insbesondere mit zahlenmittleren Molmassen ≤ 10000 g/Mol. Mischungen der vorgenannten Amine können ebenfalls verwendet werden.

Es ist ebenfalls möglich, als isocyanatreaktive Verbindungen b1) Aminoalkohole einzusetzen. Beispiele geeigneter Aminoalkohole sind die isomeren Aminoethanole, die isomere Aminopropanole die isomeren Aminobutanole und die isomeren Aminohexanole oder deren beliebige Mischungen.

Alle vorgenannten isocyanatreaktiven Verbindungen b1) können untereinander beliebig vermischt werden.

Bevorzugt ist auch, wenn die isocyanatreaktiven Verbindungen b1) eine zahlenmittlere Molmasse von ≥ 200 und ≤ 10000 g/Mol, weiter bevorzugt ≥ 500 und ≤ 8000 g/Mol und ganz besonders bevorzugt ≥ 800 und ≤ 5000 g/Mol aufweisen. Die OH-Funktionalität der Polyole beträgt bevorzugt 1.5 bis 6.0, besonders bevorzugt 1.8 bis 4.0.

Die Präpolymere der Polyisocyanat-Komponente a) können insbesondere einen Restgehalt an freiem monomeren Di- und Triisocyanaten < 1 Gew.-%, besonders bevorzugt < 0.5 Gew.-% und ganz besonders bevorzugt < 0.3 Gew.-% aufweisen.

Es ist gegebenenfalls auch möglich, dass die Polyisocyanat-Komponente a) vollständig oder anteilsmäßig organische Verbindung enthält, deren NCO-Gruppen ganz oder teilweise mit aus der Beschichtungstechnologie bekannten Blockierungsmitteln umgesetzt sind. Beispiel für Blockierungsmittel sind Alkohole, Lactame, Oxime, Malonester, Pyrazole sowie Amine, wie z.B. Butanonoxim, Diisopropylamin, Malonsäurediethylester, Acetessigester, 3,5-Dimethylpyrazol, ε-Caprolactam, oder deren Mischungen.

Besonders bevorzugt ist, wenn die Polyisocyanat-Komponente a) Verbindungen mit aliphatisch gebundenen NCO-Gruppen umfasst, wobei unter aliphatisch gebundenen NCO-Gruppen derartige Gruppen verstanden werden, die an ein primäres C-Atom gebunden sind.

Die isocyanatreaktive Komponente b) umfasst bevorzugt wenigstens eine organische Verbindung, die im Mittel wenigstens 1.5 und bevorzugt 2 bis 3 isocyanatreaktive Gruppen aufweist. Im Rahmen der vorliegenden Erfindung werden als isocyanatreaktive Gruppen bevorzugt Hydroxy-, Amino- oder MercaptoGruppen angesehen.

Die isocyanatreaktive Komponente kann insbesondere Verbindungen umfassen, die im Zahlenmittel wenigstens 1.5 und bevorzugt 2 bis 3 isocyanatreaktive Gruppen aufweisen.

Geeignete polyfunktionelle, isocyanatreaktive Verbindungen der Komponente b) sind beispielsweise die oben beschriebenen Verbindungen b1) inklusive aller für die Komponente b1) genannten bevorzugten Ausführungsformen.

Weitere Beispiele geeigneter Polyether und Verfahren zu deren Herstellung sind in der EP 2 172 503 A1 beschrieben, auf deren diesbezügliche Offenbarung hiermit Bezug genommen wird.

Durch Reaktion der Polyisocyanat-Komponente a) mit der isocyanatreaktiven Komponente b) entsteht ein polymeres Matrixmaterial. Besonders bevorzugt ist dieses Matrixmaterial bestehend aus Additionsprodukten von Butyrolacton, ε-Caprolacton und/oder Methyl-ε-caprolacton an Polyetherpolyolen einer Funktionalität von ≥ 1.8 und ≤ 3.1 mit zahlenmittleren Molmassen von ≥ 200 und ≤ 4000 g/mol in Verbindung mit Isocyanuraten, Uretdionen, Iminooxadiazindionen und/oder anderen Oligomeren basierend auf HDI. Ganz besonders bevorzugt sind Additionsprodukte von ε -Caprolacton an Poly(tetrahydrofurane) mit einer Funktionalität von ≥ 1.9 und ≤ 2.2 und zahlenmittleren Molmassen von ≥ 500 und ≤ 2000 g/mol, insbesondere von ≥ 600 und ≤ 1400 g/mol, deren zahlenmittlere Gesamtmolmasse von ≥ 800

und ≤ 4500 g/Mol, insbesondere von ≥ 1000 und ≤ 3000 g/Mol liegt in Verbindung mit Oligomeren, Isocyanuraten und/oder Iminooxadiazindionen basierend auf HDI.

Bei einer weiteren bevorzugten Ausführungsform ist vorgesehen, dass das Schreibmonomer wenigstens ein mono- und / oder ein multifunktionales Schreibmonomer umfasst oder daraus besteht. Weiter bevorzugt kann das Schreibmonomer wenigstens ein mono- und / oder ein multifunktionelles (Meth)acrylat-Schreibmonomere umfassen oder daraus bestehen. Ganz besonders bevorzugt kann das Schreibmonomer wenigstens ein mono- und / oder ein multifunktionelles Urethan(meth)acrylat umfassen oder daraus bestehen.

Geeignete Acrylat-Schreibmonomere sind insbesondere Verbindungen der allgemeinen Formel (IV)

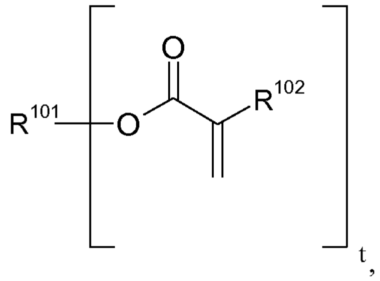

(IV)

bei denen t≥1 und t≤4 ist und $R^{101}$ ein linearer, verzweigter, cyclischer oder heterocyclischer unsubstituierter oder gegebenenfalls auch mit Heteroatomen substituierter organischer Rest und/oder $R^{102}$ Wasserstoff, ein linearer, verzweigter, cyclischer oder heterocyclischer unsubstituierter oder gegebenenfalls auch mit Heteroatomen substituierter organischer Rest ist. Besonders bevorzugt ist $R^{102}$ Wasserstoff oder Methyl und/oder $R^{101}$ ein linearer, verzweigter, cyclischer oder heterocyclischer unsubstituierter oder gegebenenfalls auch mit Heteroatomen substituierter organischer Rest.

Als Acrylate bzw. Methacrylate werden Ester der Acrylsäure bzw. Methacrylsäure bezeichnet. Beispiele bevorzugt verwendbarer Acrylate und Methacrylate sind Phenylacrylat, Phenylmethacrylat, Phenoxyethylacrylat, Phenoxyethylmethacrylat, Phenoxyethoxyethylacrylat, Phenoxyethoxyethylmethacrylat, Phenylthioethylacrylat, Phenylthioethylmethacrylat, 2-Naphthylacrylat, 2-Naphthylmethacrylat, 1,4-Bis-(2-thionaphthyl)-2-butylacrylat, 1,4-Bis-(2-thionaphthyl)-2-butylmethacrylat, Bisphenol A Diacrylat, Bisphenol A Dimethacrylat, sowie deren ethoxylierte Analogverbindungen, N-Carbazolylacrylate.

Als Urethanacrylate werden Verbindungen mit mindestens einer Acrylsäureestergruppe und mindestens eine Urethanbindung verstanden. Solche Verbindungen können beispielsweise durch Umsetzung eines Hydroxy-funktionellen Acrylats oder Methacrylats mit einer Isocyanat-funktionellen Verbindung erhalten werden.

Beispiele hierfür verwendbarer Isocyanat-funktionelle Verbindungen sind Monoisocyanate sowie die unter a) genannten monomeren Diisocyanate, Triisocyanate und / oder Polyisocyanate. Beispiele geeigneter Monoisocyanate sind Phenylisocyanat, die isomeren Methylthiophenylisocyanate. Di-, Tri- oder Polyisocyanate sind oben genannt sowie Triphenylmethan-4,4',4"-triisocyanat und Tris(p-isocyanatophenyl)thiophosphat oder deren Derivate mit Urethan-, Harnstoff-, Carbodiimid-, Acylharnstoff-, Isocyanurat-, Allophanat-, Biuret-, Oxadiazintrion-, Uretdion-, Iminooxadiazindionstruktur und Mischungen derselben. Bevorzugt sind dabei aromatische Di-, Tri- oder Polyisocyanate.

Als hydroxyfunktionelle Acrylate oder Methacrylate für die Herstellung von Urethanacrylaten kommen beispielsweise Verbindungen wie 2-Hydroxyethyl(meth)acrylat, Polyethylenoxid-mono(meth)acrylate, Polypropylenoxidmono(meth)acrylate, Polyalkylenoxidmono(meth)-acrylate, Poly(ε-caprolacton)mono-(meth)acrylate, wie z.B. Tone® M100 (Dow, Schwalbach, DE), 2-Hydroxypropyl(meth)acrylat, 4-Hydroxybutyl(meth)acrylat, 3 -Hydroxy-2,2-dimethylpropyl-(meth)acrylat, Hydroxypropyl(meth)acrylat, Acrylsäure-(2-hydroxy-3-phenoxypropylester), die hydroxyfunktionellen Mono-, Di- oder Tetraacrylate mehrwertiger Alkohole wie Trimethylolpropan, Glycerin, Pentaerythrit, Dipentaerythrit, ethoxyliertes, propoxyliertes oder alkoxyliertes Trimethylolpropan, Glycerin, Pentaerythrit, Dipentaerythrit oder deren technische Gemische. Bevorzugt sind 2-Hydroxyethylacrylat, Hydroxypropylacrylat, 4-Hydroxybutylacrylat und Poly(ε-caprolacton)mono(meth)acrylat.

Ebenfalls verwendet werden können die an sich bekannten hydroxylgruppenhaltigen Epoxy(meth)acrylate mit OH-Gehalten von 20 bis 300 mg KOH/g oder hydroxylgruppenhaltige Polyurethan(meth)acrylate mit OH-Gehalten von 20 bis 300 mg KOH/g oder acrylierte Polyacrylate mit OH-Gehalten von 20 bis 300 mg KOH/g sowie deren Mischungen untereinander und Mischungen mit hydroxylgruppenhaltigen ungesättigten Polyestern sowie Mischungen mit Polyester(meth)acrylaten oder Mischungen hydroxylgruppenhaltiger ungesättigter Polyester mit Polyester(meth)acrylaten.

Bevorzugt sind insbesondere Urethanacrylate erhältlich aus der Umsetzung von Tris(p-isocyanatophenyl)thiophosphat und / oder m-Methylthiophenylisocyanat mit alkoholfunktionellen Acrylaten wie Hydroxyethyl(meth)acrylat, Hydroxypropyl(meth)acrylat und / oder Hydroxybutyl(meth)-acrylat.

Ebenso ist es möglich, dass das Schreibmonomer weitere ungesättigte Verbindungen wie $\alpha$,$\beta$-ungesättigte Carbonsäurederivate wie beispielsweise Maleinate, Fumarate, Maleimide, Acrylamide, weiterhin Vinylether, Propenylether, Allylether und Dicyclopentadienyl-Einheiten enthaltende Verbindungen sowie olefinisch ungesättigte Verbindungen wie z.B. Styrol, $\alpha$-Methylstyrol, Vinyltoluol und / oder Olefine, umfasst oder daraus besteht.

Gemäß einer weiteren bevorzugten Ausführungsform ist vorgesehen, dass die Photopolymer-Formulierung zusätzlich Urethane als Additive enthält, wobei die Urethane insbesondere mit wenigstens einem Fluoratom substituiert sein können.

Bevorzugt können die Urethane die allgemeine Formel (V)

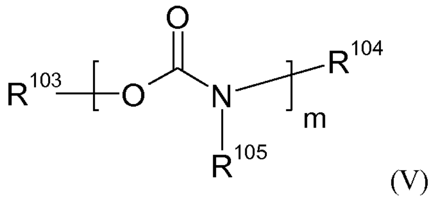

(V)

haben, in der m≥1 und m≤8 ist und $R^{103}$ ein linearer, verzweigter, cyclischer oder heterocyclischer unsubstituierter oder gegebenenfalls auch mit Heteroatomen substituierter organischer Rest und/oder $R^{104}$, $R^{105}$ unabhängig voneinander Wasserstoff sind, wobei bevorzugt mindestens einer der Reste $R^{103}$, $R^{104}$, $R^{105}$ mit wenigstens einem Fluoratom substituiert ist und besonders bevorzugt $R^{103}$ ein organischer Rest mit mindestens einem Fluoratom ist. Besonders bevorzugt ist $R^{105}$ ein linearer, verzweigter, cyclischer oder heterocyclischer unsubstituierter oder gegebenenfalls auch mit Heteroatomen wie beispielsweise Fluor substituierter organischer Rest.

Photoinitiatoren sind üblicherweise durch aktinische Strahlung aktivierbare Verbindungen, die eine Polymerisation der Schreibmonomere auslösen können. Bei den Photoinitiatoren kann zwischen unimolekularen (Typ I) und bimolekularen (Typ II) Initiatoren unterschieden werden. Des Weiteren werden sie je nach ihrer chemischen Natur in Photoinitiatoren für radikalische, anionische, kationische oder gemischte Art der Polymerisation unterschieden.

Typ I-Photoinitiatoren (Norrish-Typ-I) für die radikalische Photopolymerisation bilden beim Bestrahlen durch eine unimolekulare Bindungsspaltung freie Radikale. Beispiele für Typ I-Photoinitiatoren sind Triazine, Oxime, Benzoinether, Benzilketale, Bis-imidazole, Aroylphosphinoxide, Sulfonium- und Iodoniumsalze.

Typ II-Photoinitiatoren (Norrish-Typ-II) für die radikalische Polymerisation bestehen aus einem Farbstoff als Sensibilisator und einem Coinitiator und durchlaufen bei der Bestrahlung mit auf den Farbstoff angepasstem Licht eine bimolekulare Reaktion. Zunächst absorbiert der Farbstoff ein Photon und überträgt aus einem angeregten Zustand Energie auf den Coinitiator. Dieser setzt durch Elektronen- oder Protonentransfer oder direkte Wasserstoffabstraktion die polymerisationsauslösenden Radikale frei.

Im Sinne dieser Erfindung werden bevorzugt Typ II-Photoinitiatoren verwendet.

Solche Photoinitiatorsysteme sind prinzipiell in der EP 0 223 587 A beschrieben und bestehen bevorzugt aus einer Mischung von einem oder mehreren Farbstoffen mit Ammoniumalkylarylborat(en).

Geeignete Farbstoffe, die zusammen mit einem Ammoniumalkylarylborat einen Typ II-Photoinitiator bilden, sind die in der WO 2012062655 beschriebenen kationischen Farbstoffe in Kombination mit den eben dort beschriebenen Anionen.

Unter kationischen Farbstoffen werden bevorzugt solche der folgenden Klassen verstanden: AcridinFarbstoffe, Xanthen-Farbstoffe, Thioxanthen-Farbstoffe, Phenazin-Farbstoffe, Phenoxazin-Farbstoffe, Phenothiazin-Farbstoffe, Tri(het)arylmethan-Farbstoffe - insbesondere Diamino- und Triamino(het)-arylmethan-Farbstoffe, Mono-, Di-, Tri- und Pentamethincyanin-Farbstoffe, Hemicyanin-Farbstoffe, extern kationische Merocyanin-Farbstoffe, extern kationische Neutrocyanin-Farbstoffe, Nullmethin-Farbstoffe - insbesondere Naphtholactam-Farbstoffe, Streptocyanin-Farbstoffe. Solche Farbstoffe sind beispielsweise in H. Berneth in Ullmann's Encyclopedia of Industrial Chemistry, Azine Dyes, Wiley-VCH Verlag, 2008, H. Berneth in Ullmann's Encyclopedia of Industrial Chemistry, Methine Dyes and Pigments, Wiley-VCH Verlag, 2008, T. Gessner, U. Mayer in Ullmann's Encyclopedia of Industrial Chemistry, Triarylmethane and Diarylmethane Dyes, Wiley-VCH Verlag, 2000 beschrieben.

Besonders bevorzugt sind Phenazin-Farbstoffe, Phenoxazin-Farbstoffe, Phenothiazin-Farbstoffe, Tri(het)-arylmethan-Farbstoffe - insbesondere Diamino- und Triamino(het)arylmethan-Farbstoffe, Mono-, Di-, Tri- und Pentamethincyanin-Farbstoffe, Hemicyanin-Farbstoffe, Nullmethin-Farbstoffe - insbesondere Naphtholactam-Farbstoffe, Streptocyanin-Farbstoffe.

Beispiele für kationische Farbstoffe sind Astrazon Orange G, Basic Blue 3, Basic Orange 22, Basic Red 13, Basic Violett 7, Methylenblau, Neu Methylenblau, Azur A, 2,4-Diphenyl-6-(4-methoxyphenyl)-pyrylium, Safranin O, Astraphloxin, Brilliant Grün, Kristallviolett, Ethylviolett und Thionin.

Bevorzugte Anionen sind insbesondere $C_8$- bis $C_{25}$-Alkansulfonat, vorzugsweise $C_{13}$- bis $C_{25}$-Alkansulfonat, $C_3$- bis $C_{18}$-Perfluoralkansulfonat, $C_4$- bis $C_{18}$-Perfluoralkansulfonat, das in der Alkylkette mindestens 3 Wasserstoffatome trägt, $C_9$- bis $C_{25}$-Alkanoat, $C_9$- bis $C_{25}$-Alkenoat, $C_8$- bis $C_{25}$-Alkylsulfat, vorzugsweise $C_{13}$- bis $C_{25}$-Alkylsulfat, $C_8$- bis $C_{25}$-Alkenylsulfat, vorzugsweise $C_{13}$- bis $C_{25}$-Alkenylsulfat, $C_3$- bis $C_{18}$-Perfluoralkylsulfat, $C_4$- bis $C_{18}$-Perfluoralkylsulfat, das in der Alkylkette mindestens 3 Wasserstoffatome trägt, Polyethersulfate basierend auf mindestens 4 Äquivalenten Ethylenoxid und/oder 4 Äquivalenten Propylenoxid, Bis-$C_4$- bis $C_{25}$-Alkyl-, $C_5$- bis $C_7$-Cycloalkyl-, $C_3$- bis Cs-Alkenyl- oder $C_7$- bis $C_{11}$-Aralkyl-sulfosuccinat, durch mindestens 8 Fluoratome substituiertes Bis-$C_2$- bis $C_{10}$-alkylsulfosuccinat, $C_8$- bis $C_{25}$-Alkyl-sulfoacetate, durch mindestens einen Rest der Gruppe Halogen, $C_4$- bis $C_{25}$-Alkyl, Perfluor-Ci- bis $C_8$-Alkyl und/oder $C_1$- bis $C_{12}$-Alkoxycarbonyl substituiertes Benzolsulfonat, ggf. durch Nitro, Cyano, Hydroxy, $C_1$- bis $C_{25}$-Alkyl, $C_1$- bis $C_{12}$-Alkoxy, Amino, $C_1$- bis $C_{12}$-Alkoxycarbonyl oder Chlor substituiertes Naphthalin- oder Biphenylsulfonat, ggf. durch Nitro, Cyano, Hydroxy, $C_1$- bis $C_{25}$-Alkyl, $C_1$- bis $C_{12}$-Alkoxy, $C_1$- bis $C_{12}$-Alkoxycarbonyl oder Chlor substituiertes Benzol-, Naphthalin- oder Biphenyldisulfonat, durch Dinitro, $C_6$- bis $C_{25}$-Alkyl, $C_4$- bis $C_{12}$-Alkoxycarbonyl, Benzoyl, Chlorbenzoyl oder Toluoyl substituiertes Benzoat, das Anion der Naphthalindicarbonsäure, Diphenyletherdisulfonat, sulfonierte oder sulfatierte, ggf. mindestens einfach ungesättigte $C_8$- bis $C_{25}$-Fettsäureester von aliphatischen $C_1$- bis $C_8$-Alkoholen oder Glycerin, Bis-(sulfo-$C_2$- bis $C_6$-alkyl)-$C_3$- bis $C_{12}$-alkandicarbonsäureester, Bis-(sulfo-$C_2$- bis $C_6$-alkyl)-itaconsäureester, (Sulfo-$C_2$- bis $C_6$-alkyl)-$C_6$- bis $C_{18}$-alkancarbonsäureester, (Sulfo-$C_2$- bis $C_6$-alkyl)-acryl- oder methacrylsäureester, ggf. durch bis zu 12 Halogenreste substituiertes Triscatecholphosphat, ein Anion der Gruppe Tetraphenylborat, Cyanotriphenylborat, Tetraphenoxyborat, $C_4$- bis $C_{12}$-Alkyl-triphenylborat, deren Phenyl- oder Phenoxy-Reste durch Halogen, $C_1$- bis $C_4$-Alkyl und/oder $C_1$- bis $C_4$-Alkoxy substituiert sein können, $C_4$- bis $C_{12}$-Alkyl-trinaphthylborat, Tetra-$C_1$- bis $C_{20}$-alkoxyborat, 7,8- oder 7,9-Dicarba-nido-undecaborat(1-) oder (2-), die gegebenenfalls an den B- und/oder C-Atomen durch eine oder zwei $C_1$- bis $C_{12}$-Alkyl- oder Phenyl-Gruppen substituiert sind, Dodecahydro-dicarbadodecaborat$^{(2-)}$ oder B-$C_1$- bis $C_{12}$-Alkyl-C-phenyl-dodecahydro-dicarbadodecaborat$^{(-)}$ steht, wobei bei mehrwertigen Anionen wie Naphthalindisulfonat $A^-$ für ein Äquivalent dieses Anions steht, und wobei die Alkan- und Alkylgruppen verzweigt sein können und/oder durch Halogen, Cyano, Methoxy, Ethoxy, Methoxycarbonyl oder Ethoxycarbonyl substituiert sein können.

Bevorzugt ist auch, wenn das Anion $A^-$ des Farbstoffs einen AClogP im Bereich von 1 bis 30, besonders bevorzugt im Bereich von 1 bis 12 und insbesondere bevorzugt im Bereich von 1 bis 6,5 aufweist. Der AClogP wird nach J. Comput. Aid. Mol. Des. 2005, 19, 453; Virtual Computational Chemistry Laboratory, http://www.vcclab.org berechnet.

Geeignete Ammoniumalkylarylborate sind die erfindungsgemäßen Ammoniumalkylarylborate der Formel (Ia) sowie ihre ebenfalls erfindungsgemäßen Mischungen mit den Boraten der Formeln (Ib) und/oder (IIIa) und/oder (IIIb) und /oder den Salzen der Formel (II).

Es kann vorteilhaft sein, Gemische dieser Photoinitiatoren einzusetzen. Je nach verwendeter Strahlungsquelle muss Typ und Konzentration an Photoinitiator in dem Fachmann bekannter Weise angepasst werden. Näheres ist zum Beispiel in P. K. T. Oldring (Ed.), Chemistry & Technology of UV & EB Formulations For Coatings, Inks & Paints, Vol. 3, 1991, SITA Technology, London, S. 61 - 328 beschrieben.

Ganz besonders bevorzugt ist, wenn der Photoinitiator eine Kombination von Farbstoffen, deren Absorptionsspektren zumindest teilweise den Spektralbereich von 400 bis 800 nm abdecken, mit wenigstens einem auf die Farbstoffe abgestimmten Coinitiator umfasst.

Bevorzugt ist auch, wenn wenigstens ein für eine Laserlichtfarbe ausgewählt aus blau, grün und rot geeigneter Photoinitiator in der Photopolymer-Formulierung enthalten ist.

Weiter bevorzugt ist auch, wenn die Photopolymer-Formulierung für wenigstens zwei Laserlichtfarben ausgewählt aus blau, grün und rot je einen geeigneten Photoinitiator enthält.

Ganz besonders bevorzugt ist schließlich, wenn die Photopolymer-Formulierung für jede der Laserfarben blau, grün und rot jeweils einen geeigneten Photoinitiator enthält.

Aus der Photopolymer-Formulierung können Photopolymerfilme hergestellt werden, die bevorzugt eine Schichtdicke von $\geq 1$ $\mu$m und $\leq 100$ $\mu$m, besonders bevorzugt von $\geq 5$ $\mu$m und $\leq 30$ $\mu$m und ganz besonders bevorzugt von $\geq$ 7 $\mu$m und $\leq 25$ $\mu$m aufweisen.

## Holographische Medien

Unter Verwendung der erfindungsgemäßen Coinitiatoren enthaltend wenigstens eine Substanz der Formel (Ia) oder ihre Mischungen mit Substanzen der Formeln (Ib), (IIIa) und/oder (IIIb) sowie gegebenenfalls Salzen der Formel (II) konnten Photopolymerfilme hergestellt werden, die hohe Langzeitstabilität und photochemische Bleichbarkeit zeigen und in die helle Hologramme einbelichtet werden können.

Ein weiterer Gegenstand der Erfindung sind holographische Medien, die unter Verwendung der erfindungsgemäßen Photopolymer-Formulierung hergestellt werden können.

Die holographischen Medien können die oben genannten Photopolymerfilmen basierend auf der erfindungsgemäßen Photopolymer-Formulierung enthalten oder daraus bestehen.

Die holographischen Medien können belichtet oder in unbelichteter Form vorliegen.

Die erfindungsgemäßen holographischen Medien können durch entsprechende Belichtungsprozesse für optische Anwendungen im gesamten sichtbaren Bereich (400-800 nm) zu Hologrammen verarbeitet werden können. Visuelle Hologramme umfassen alle Hologramme, die nach dem Fachmann bekannten Verfahren aufgezeichnet werden können. Darunter fallen unter anderem In-Line (Gabor) Hologramme, Off-Axis Hologramme, Full-Aperture Transfer Hologramme, Weißlicht-Transmissionshologramme ("Regenbogenhologramme), Denisyukhologramme, Off-Axis Reflektionshologramme, Edge-Lit Hologramme sowie holographische Stereogramme. Bevorzugt sind Reflexionshologramme, Denisyukhologramme, Transmissionshologramme.

Mögliche optische Funktionen der Hologramme, die mit den erfindungsgemäßen Photopolymer-Formulierungen hergestellt werden können entsprechen den optische Funktionen von Lichtelementen wie Linsen, Spiegel, Umlenkspiegel, Filter, Streuscheiben, Beugungselemente, Lichtleiter, Lichtlenker (waveguides), Projektionsscheiben und/oder Masken. Häufig zeigen diese optischen Elemente eine Frequenzselektivität, je nachdem wie die Hologramme belichtet wurden und welche Dimensionen das Hologramm hat.

Zudem können mittels der erfindungsgemäßen Photopolymer-Formulierungen auch holographische Bilder oder Darstellungen hergestellt werden, wie zum Beispiel für persönliche Portraits, biometrische Darstellungen in Sicherheitsdokumenten, oder allgemein von Bilder oder Bildstrukturen für Werbung, Sicherheitslabels, Markenschutz, Markenbranding, Etiketten, Designelementen, Dekorationen, Illustrationen, Sammelkarten, Bilder und dergleichen sowie Bilder, die digitale Daten repräsentieren können u.a. auch in Kombination mit den zuvor dargestellten Produkten. Holographische Bilder können den Eindruck eines dreidimensionalen Bildes haben, sie können aber auch Bildsequenzen, kurze Filme oder eine Anzahl von verschiedenen Objekten darstellen, je nachdem aus welchem Winkel, mit welcher (auch bewegten) Lichtquelle etc. diese beleuchtet wird. Aufgrund dieser vielfältigen Designmöglichkeiten stellen Hologramme, insbesondere Volumenhologramme, eine attraktive technische Lösung für die oben genannte Anwendung dar.

Die Photopolymere-Formulierungen können insbesondere zur Herstellung holographischer Medien in Form eines Films verwendet werden. Dabei wird als Träger eine Lage eines für Licht im sichtbaren Spektralbereich (Transmission größer als 85% im Wellenlängenbereich von 400 bis 780 nm) transparenten Materials oder Materialverbunds ein- oder beidseitig beschichtet sowie ggf. eine Abdeckschicht auf der oder den Photopolymerlagen appliziert.

Gegenstand der Erfindung ist daher auch ein Verfahren zur Herstellung eines holographischen Mediums, bei dem

(I) eine erfindungsgemäße Photopolymer-Formulierung durch Vermischen aller Bestandteile hergestellt,

(II) die Photopolymer-Formulierung bei einer Verarbeitungstemperatur in die für das holographische Medium gewünschte Form gebracht und

(III) in der gewünschten Form unter Urethanbildung bei einer Vernetzungstemperatur oberhalb der Verarbeitungstemperatur ausgehärtet wird.

Bevorzugt wird die Photopolymer-Formulierung im Schritt I) durch Vermischen der einzelnen Bestandteile hergestellt. Dazu wird bevorzugt die gegenüber Isocyanaten reaktive Komponente schrittweise mit den Schreibmonomeren, den Additiven und dem Katalysator versetzt und gemischt. Anschließend wird bevorzugt eine Lösung der lichtsensitiven Photoinitiatorlösung der Mischung im Dunklen hinzugefügt und vermischt, so dass eine klare Lösung erhalten wird. Wenn nötig, wird die Formulierung für kurze Zeit bei 60 °C erwärmt, um die Einsatzstoffe schneller in Lösung zu bringen. Dieses Gemisch kann in einen von zwei Vorratsbehältern an einer dem Experten bekannten Folienbeschichtungsanlage eingebracht werden. In den zweiten Vorratsbehälter kann die Polyisocyanat-Komponente eingefüllt werden. Beide Komponenten werden dann bevorzugt vermischt. Hierbei können u.a dem Fachmann bekannten Dosier-, Filter- und Entgasungssysteme zum Einsatz kommen. Die erhaltene, flüssige Masse kann dann einer Beschichtungseinrichtung zugeführt werden.

Bevorzugt wird die Photopolymer-Formulierung im Schritt II) in die Form eines Films gebracht. Dazu kann die Photopolymer-Formulierung beispielsweise flächig auf ein Trägersubstrat aufgebracht werden, wobei beispielsweise die dem Fachmann bekannte Vorrichtungen wie eine Rakeleinrichtungen (Doctor Blade, knife-over-Roll, Commabar, u.a) oder eine Schlitzdüse zum Einsatz kommen können. Die Verarbeitungstemperatur liegt dabei im Bereich 20 bis 40°C, vorzugsweise im Bereich 20 bis 30°C.

Als Trägersubstrat kann eine Lage eines für Licht im sichtbaren Spektralbereich (Transmission größer als 85% im Wellenlängenbereich von 400 bis 800 nm) transparenten Materials oder Materialverbunds verwendet werden.

Bevorzugte Materialien oder Materialverbünde des Trägersubstrats basieren auf Polycarbonat (PC), Polyethylenterephthalat (PET), Polybutylenterephthalat, Polyethylen, Polypropylen, Celluloseacetat, Cellulosehydrat, Cellulosenitrat, Cycloolefinpolymere, Polystyrol, Polyepoxide, Polysulfon, Cellulosetriacetat (CTA), Polyamid, Polymethylmethacrylat, Polyvinylchlorid, Polyvinylbutyral oder Polydicyclopentadien oder deren Mischungen. Besonders bevorzugt basieren sie auf PC, PET und CTA. Materialverbünde können Folienlaminate oder Coextrudate sein. Bevorzugte Materialverbünde sind Duplex- und Triplexfolien aufgebaut nach einem der Schemata A/B, A/B/A oder A/B/C. Besonders bevorzugt sind PC/PET, PET/PC/PET und PC/TPU (TPU = Thermoplastisches Polyurethan).

Alternativ zu den vorgenannten Trägersubstraten können auch planare Glasplatten eingesetzt werden, die insbesondere für großflächige abbildungsgenaue Belichtungen Verwendung finden, z.B. für holographische Lithographie (Holographic interference lithography for integrated optics. IEEE Transactions on Electron Devices (1978), ED-25(10), 1193-1200, ISSN:0018-9383).

Die Materialien oder Materialverbünde des Trägersubstrats können einseitig oder beidseitig antihaftend, antistatisch, hydrophobiert oder hydrophiliert ausgerüstet sein. Die genannten Modifikationen dienen an der dem Photopolymer zugewandten Seite dem Zweck, dass das Photopolymer von dem Trägersubstrat zerstörungsfrei abgelöst werden kann. Eine Modifikation der dem Photopolymer abgewandten Seite des Trägersubstrats dient dazu, dass die erfindungsgemäßen Medien speziellen mechanischen Anforderungen genügen, die z.B. bei der Verarbeitung in Rollenlaminatoren, insbesondere bei Rolle-zu-Rolle-Verfahren, gefordert sind.

Das Trägersubstrat kann ein- oder beidseitig beschichtet sein.

Die Vernetzungstemperatur kann insbesondere ≥ 60° C und ≤ 110° C und bevorzugt ≥ 70° C und ≤ 105° C und besonders bevorzugt ≥ 75° C und ≤ 100° C sein.

Gegenstand der Erfindung ist auch ein nach dem erfindungsgemäßen Verfahren erhältliches holographisches Medium.

Ein weiter Gegenstand der Erfindung ist ein Schichtaufbau, umfassend ein Trägersubstrat, darauf aufgebracht ein erfindungsgemäßes holographisches Medium und gegebenenfalls eine auf der dem Trägersubstrat abgewandten Seite des holographischen Mediums aufgebrachte Abdeckschicht.

Medium aufweisen, um diesen vor Schmutz und Umwelteinflüssen zu schützen. Hierzu können Kunststofffolien oder Folienverbundsysteme, aber auch Klarlacke verwendet werden.

Als Abdeckschichten werden bevorzugt Folienmaterialien analog den in dem Trägersubstrat eingesetzten Materialien verwendet, wobei diese eine Dicke von typischerweise 5 bis 200 μm, bevorzugt 8 bis 125 μm, besonders bevorzugt 10 bis 50 μm aufweisen können.

Bevorzugt sind Abdeckschichten mit einer möglichst glatten Oberfläche. Als Maß gilt hier die Rauigkeit, bestimmt nach DIN EN ISO 4288 "Geometrische Produktspezifikation (GPS) - Oberflächenbeschaffenheit..." (engl. "Geometrical Product Specifications (GPS) - Surface texture..."), Prüfbedingung R3z Vorderseite und Rückseite. Bevorzugte Rauigkeiten liegen im Bereich kleiner oder gleich 2 μm, bevorzugt kleiner oder gleich 0,5 μm.

Als Abdeckschichten werden bevorzugt PE- oder PET-Folien einer Dicke von 20 bis 60 μm verwendet. Besonders bevorzugt kommt eine Polyethylenfolie mit einer Dicke von 40 μm zum Einsatz.

Es ist ebenfalls möglich, dass bei einem Schichtaufbau auf dem Trägersubstrat eine weitere Abdeckschicht als Schutzschicht aufgebracht ist.

**Syntheseverfahren**

Ein weiterer Gegenstand der Erfindung ist ein Verfahren zur Herstellung von Coinitiatoren umfassend eine Mischung von Substanzen der Formel (Ia) und Salzen der Formel (II), die gegebenenfalls auch Substanzen der Formel (Ib) und/oder der Formel (IIIa) und gegebenenfalls der Formel (IIIb) enthalten kann,

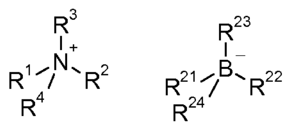

(Ia)

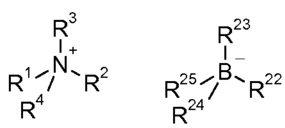

(Ib)

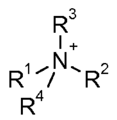

$An^-$ (II)

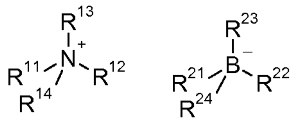

(IIIa)

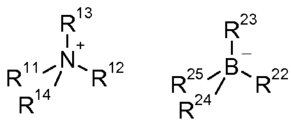

(IIIb),

worin

$R^1$ bis $R^4$, $R^{11}$ bis $R^{14}$, $R^{21}$ bis $R^{24}$, $R^{25}$ und $An^-$ die zuvor genannte Bedeutung besitzt,

dadurch gekennzeichnet, dass Borate der Formel (IIIa) oder eine Mischung aus Boraten der Formeln (IIIa) und (IIIb) mit einem Ammoniumsalz der Formel (VI)

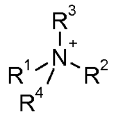

$X^-$ (VI),

worin

$R^1$ bis $R^4$ die oben genannte Bedeutung besitzen und $X^-$ für ein Anion, bevorzugt für ein Halogenid-Ion steht,

in Gegenwart eines Salzes der Formel (VII)

$M^+ An^-$ (VII),

worin

$M^+$ für ein Kation, bevorzugt für ein Alkali-Ion oder ein Ammonium-Ion steht und

$An^-$ die oben genannte Bedeutung besitzt,

in einem Zweiphasengemisch aus Wasser und einem Ester umgesetzt werden.

$M^+$ steht bevorzugt für $Na^+$ oder $K^+$, besonders bevorzugt für $Na^+$.

Ein effizientes Verfahrens zur Herstellung der als Coinitiatoren geeigneten Kohlenstoff-substituierten Ammonium-Alkyltriarylborate wurde überraschenderweise erreicht, indem diese in Gegenwart eines Anions $An^-$ synthetisiert wurden.

Überraschend wurde gefunden, dass sich besonders die bereits oben definierten Anionen $An^-$, die einen AClogP im Bereich von 2 bis 8, vorzugsweise 3 bis 6, besonders bevorzugt 3.5 bis 5 aufweisen, eignen.

Vorzugsweise wird das Verfahren so durchgeführt, dass man das Zweiphasengemisch trennt, die organische Phase zur Entfernung von Anionen $X^-$ mit Wasser wäscht und schließlich die organische Phase von enthaltenem Wasser befreit.

Die Anzahl der Wasserwäschen wird dadurch bestimmt, dass in der letzten Wasserwäsche das mit dem Ammoniumsalz der Formel (VI) eingebrachte Anion $X^-$ nicht mehr nachweisbar ist. Wenn, wie bevorzugt, $X^-$ für Chlorid oder Bromid steht, wird dieser Nachweis beispielsweise so geführt, dass man eine Probe der Wasserwäsche mit 10 Gew.-prozentiger Salpetersäure ansäuert und dann eine 5 Gew.-prozentige wässrige Lösung von Silbernitrat zugibt. Wenn dann höchstens eine kaum sichtbare schwache Trübung entsteht, kann auf weitere Wasserwäschen verzichtet werden.

Selbstverständlich hängt die Zahl der erforderlichen Wasserwäschen auch von deren Volumen relativ zum Volumen der organischen Phase ab. 3 bis 10, vorzugsweise 4 bis 8 Wasserwäschen sind ein guter Orientierungswert.

Ebenfalls vorzugsweise geht man dabei so vor, dass man die Salze der Formel (VII) relativ zu den Substanzen der Formel (Ia) oder der Summe der Substanzen der Formeln (Ia) und (Ib) im molaren Verhältnis 0.5 bis 10 zu 100, bevorzugt 1 bis 5 zu 100 einsetzt.

Des Weiteren bevorzugt werden die Salze der Formel (II) relativ zur Summe der Substanzen der Formeln (Ia), (Ib) und dem Salz der Formel (VII) im molaren Verhältnis 100 bis 110 zu 100, bevorzugt 100 bis 105 zu 100, besonders bevorzugt 100 bis 102 zu 100 eingesetzt.

Geeignete Ester sind die gegebenenfalls substituierten Alkylester der Ameisensäure, Essigsäure, Propionsäure oder Butansäure. Die Alkylgruppen können 1 bis 6 C-Atome enthalten und gegebenenfalls verzweigt sein. Sie können auch Reste wie Alkoxy tragen.

Beispiele sind n- oder i-Propylformiat, n-, -s- oder -i-Butylformiat, Methylacetat, Ethylacetat, n- oder -i-Propylacetat, n-, -s- oder -i-Butylacetat, Methoxypropylacetat, Diethylenglycolmonoethyletheracetat, Ethylpropionat, Methylbutyrat, Ethylbutyrat. Bevorzugt sind Ethylacetat, n-Butylacetat und Methoxypropylacetat, besonders bevorzugt ist n-Butylacetat.

Das Verfahren kann bei einer Temperatur von $\geq$ 10 und $\leq$ 100°C, vorzugsweise$\geq$ 20 und $\leq$ 70°C, besonders bevorzugt $\geq$ 25 und $\leq$ 50°C durchgeführt werden.

Nach dem erfindungsgemäßen Verfahren lassen sich stabile Lösungen der Coinitiatoren der Formel (Ia) sowie Ihrer Mischungen mit Tetraarylboraten der Formel (Ib) und/oder Tetraalkylammoniumboraten der Formeln (IIIa) und (IIIb) und mit Salzen der Formel (II) herstellen. Diese Lösungen basieren bevorzugt auf den oben angegebenen Lösungsmitteln, besonders bevorzugt Ethylacetat, n-Butylacetat und Methoxypropylacetat, ganz besonders bevorzugt n-Butylacetat. Ihre Konzentration kann in weiten Grenzen variieren. Beispielsweise liegt sie bei $\geq$ 1 und $\leq$ 90 Gew.-%, vorzugsweise bei $\geq$ 10 und $\leq$ 70 Gew.-%, besonders bevorzugt bei $\geq$ 30 und $\leq$ 60 Gew.-%, bezogen auf die Gesamtmasse der Lösung. Die Lösungen sind bevorzugt bis -78 °C stabil. Unterhalb dieser Temperatur erstarren sie ohne Entmischung zu einem klaren, sehr zähen Glas.

Ein weiterer Gegenstand der Erfindung sind daher auch Coinitiatoren herstellbar nach dem erfindungsgemäßen Verfahren. Diese sind besonders zum Einsatz in den oben genannten Photopolymer-Formulierungen geeignet. Des Weiteren eigenen sich die erhaltenen Photopolymer-Formulierungen dann zur Herstellung von holographischen Medien mit den zuvor bereits ausgeführten guten Eigenschaften. Die Coinitiatoren liegen dabei in einer bevorzugten Ausführungsform als Lösungen in den oben genannten Lösungsmitteln vor.

Ein weiterer Gegenstand der Erfindung sind Borate der Formel (VIII)

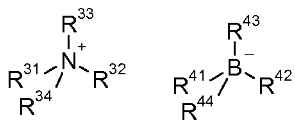

(VIII),

worin

$R^{31}$ für einen gegebenenfalls verzweigten $C_{14}$- bis $C_{22}$-Alkyl-Rest steht,
$R^{32}$ für einen $C_7$- bis $C_{10}$-Aralkyl-Rest steht,
$R^{33}$ und $R^{34}$ unabhängig voneinander für einen gegebenenfalls verzweigten und/oder gegebenenfalls substituierten $C_1$- bis Cs-Alkyl-Rest stehen
$R^{41}$ für einen $C_2$- bis $C_{22}$-Alkyl-, $C_3$- bis $C_{22}$-Alkenyl-, $C_5$- bis $C_7$-Cycloalkyl- oder $C_7$- bis $C_{13}$-Aralkylrest steht und
$R^{42}$ bis $R^{44}$ unabhängig voneinander für einen durch mindestens einen Rest der Auswahl Halogen, $C_1$- bis $C_4$-Alkyl, Trifluormethyl, $C_1$- bis $C_4$-Alkoxy, Trifluormethoxy, Phenyl oder Phenoxy substituierten $C_6$-bis $C_{10}$-Arylrest stehen, ausgenommen die Kombination $R^{31}$= Tetradecyl, $R^{32}$ = Benzyl, $R^{33}$ und $R^{34}$ = Methyl, $R^{41}$ = Butyl und $R^{42}$ bis $R^{44}$ = Phenyl,
oder worin
$R^{31}$ für einen gegebenenfalls verzweigten $C_{14}$- bis $C_{22}$-Alkyl-Rest steht,
$R^{32}$ für einen $C_7$- bis $C_{10}$-Aralkyl-Rest steht,
$R^{33}$ und $R^{34}$ unabhängig voneinander für einen gegebenenfalls verzweigten und/oder gegebenenfalls substituierten $C_1$- bis Cs-Alkyl-Rest stehen,
$R^{41}$ für einen $C_2$- bis $C_{22}$-Alkyl-, $C_3$- bis $C_{22}$-Alkenyl-, $C_5$- bis $C_7$-Cycloalkyl- oder $C_7$- bis $C_{13}$-Aralkylrest steht und
$R^{42}$ bis $R^{44}$ unabhängig voneinander für einen durch mindestens einen Rest der Auswahl Halogen, $C_1$- bis $C_4$-Alkyl, Trifluormethyl, $C_1$- bis $C_4$-Alkoxy, Trifluormethoxy, Phenyl oder Phenoxy substituierten $C_6$-bis $C_{10}$-Arylrest stehen
oder worin
$R^{31}$ für einen gegebenenfalls verzweigten $C_{14}$- bis $C_{22}$-Alkyl-Rest steht,
$R^{32}$ für einen gegebenenfalls verzweigten $C_8$- bis $C_{22}$-Alkyl-Rest oder einen $C_7$- bis $C_{10}$-Aralkyl-Rest steht,
$R^{33}$ und $R^{34}$ gemeinsam eine $-(CH_2)_4-$, $-(CH_2)_5-$ oder $-(CH_2)_2-O-(CH_2)_2-$Brücke bilden,
$R^{41}$ für einen $C_2$- bis $C_{22}$-Alkyl-, $C_3$- bis $C_{22}$-Alkenyl-, $C_5$- bis $C_7$-Cycloalkyl- oder $C_7$- bis $C_{13}$-Aralkylrest steht und
$R^{42}$ bis $R^{44}$ unabhängig voneinander für einen durch mindestens einen Rest der Auswahl Halogen, $C_1$- bis $C_4$-Alkyl,

Trifluormethyl, $C_1$- bis $C_4$-Alkoxy, Trifluormethoxy, Phenyl oder Phenoxy substituierten $C_6$-bis $C_{10}$-Arylrest stehen oder worin

$R^{31}$ für einen gegebenenfalls verzweigten $C_{14}$- bis $C_{22}$-Alkyl-Rest steht,

$R^{32}$ für einen durch ein bis drei Reste aus der Gruppe gegebenenfalls verzweigter $C_3$- bis $C_8$-Alkylrest, gegebenenfalls verzweigter $C_3$- bis $C_8$-Alkoxyrest, Trifluormethyl, Trifluormethoxy, Cyclopentyl, Cyclohexyl, Cycloheptyl, Phenyl oder Phenoxy substituierten Phenyl-Rest steht,

$R^{33}$ und $R^{34}$ unabhängig voneinander für einen gegebenenfalls verzweigten und/oder gegebenenfalls substituierten $C_1$- bis Cs-Alkyl-Rest stehen,

$R^{41}$ für einen $C_2$- bis $C_{22}$-Alkyl-, $C_3$- bis $C_{22}$-Alkenyl-, $C_5$- bis $C_7$-Cycloalkyl- oder $C_7$- bis $C_{13}$-Aralkylrest steht und

$R^{42}$ bis $R^{44}$ unabhängig voneinander für einen durch mindestens einen Rest der Auswahl Halogen, $C_1$- bis $C_4$-Alkyl, Trifluormethyl, $C_1$- bis $C_4$-Alkoxy, Trifluormethoxy, Phenyl oder Phenoxy substituierten $C_6$- bis $C_{10}$-Arylrest stehen oder worin

$R^{31}$ für einen gegebenenfalls verzweigten $C_{14}$- bis $C_{22}$-Alkyl-Rest steht und

$R^{32}$, $R^{33}$ und $R^{34}$ gemeinsam mit dem $N^+$-Atom einen Imidazol- oder Pyridin-Ring bilden, der mindestens durch einen Rest der Auswahl $C_1$- bis $C_8$-Alkyl, $C_1$- bis $C_8$-Alkoxy, $C_5$- bis $C_7$-Cycloalkyl, Benzyl oder Phenyl substituiert ist,

$R^{41}$ für einen $C_2$- bis $C_{22}$-Alkyl-, $C_3$- bis $C_{22}$-Alkenyl-, $C_5$- bis $C_7$-Cycloalkyl- oder $C_7$- bis $C_{13}$-Aralkylrest steht und

$R^{42}$ bis $R^{44}$ unabhängig voneinander für einen durch mindestens einen Rest der Auswahl Halogen, $C_1$- bis $C_4$-Alkyl, Trifluormethyl, $C_1$- bis $C_4$-Alkoxy, Trifluormethoxy, Phenyl oder Phenoxy substituierten $C_6$- bis $C_{10}$-Arylrest stehen.

Bevorzugte Borate der oben aufgeführten Formel (VII) sind solche, worin

$R^{31}$ für Tetradecyl, Hexadecyl, Octadecyl, Eicosyl oder Docosyl, besonders bevorzugt für Hexadecyl oder Octadecyl steht,

$R^{32}$ für Benzyl, 2-Phenylethyl, 2- oder 3-Phenylethyl, besonders bevorzugt für Benzyl oder 3-Phenylpropyl steht und

$R^{33}$ und $R^{34}$ gleich sind und für Methyl, Ethyl oder Hydroxyethyl, besonders bevorzugt für Methyl stehen

oder worin

$R^{31}$ für Tetradecyl, Hexadecyl, Octadecyl, Eicosyl oder Docosyl, besonders bevorzugt für Hexadecyl oder Octadecyl steht,

$R^{32}$ für einen durch mindestens einen in 3-, 4- und/oder 5-Stellung stehenden Rest aus der Gruppe 1- oder 2-Butyl, 1,1-Dimethylethyl, 1-Hexyl, 1-Octyl, 1- oder 2-Butoxy, 1,1-Dimethylethoxy, Trifluormethyl, Trifluormethoxy, Cyclopentyl, Cyclohexyl, Cycloheptyl, Phenyl oder Phenoxy substituierten Phenyl-Rest steht und

$R^{33}$ und $R^{34}$ gleich sind und für Methyl, Ethyl oder Hydroxyethyl, besonders bevorzugt für Methyl stehen

oder worin

$R^{31}$ für Tetradecyl, Hexadecyl, Octadecyl, Eicosyl oder Docosyl, besonders bevorzugt für Hexadecyl oder Octadecyl steht,

$R^{32}$ für Octyl, 2-Ethylhexyl, Nonyl, Decyl, Dodecyl, Tetradecyl, Hexadecyl, Octadecyl, Eicosyl, Docosyl, Benzyl, 2-Phenylethyl, 2- oder 3-Phenylethyl, besonders bevorzugt für Dodecyl, Tetradecyl, Hexadecyl, Octadecyl, Benzyl oder 3-Phenylpropyl steht und

$R^{33}$ und $R^{34}$ gemeinsam eine $-(CH_2)_4-$, $-(CH_2)_5-$ oder $-(CH_2)_2-O-(CH_2)_2-$Brücke bilden.

Bevorzugte Borate der oben aufgeführten Formel (VIII) sind solche, wobei

$R^{41}$ für einen gegebenenfalls verzweigten und gegebenenfalls durch Fluor, Chlor, Methoxy, Ethoxy oder Cyano substituierten $C_2$- bis $C_{18}$-Alkyl-, $C_3$- bis $C_{12}$-Alkenyl- oder $C_7$- bis $C_{10}$-Aralkylrest, Cyclopentyl oder Cyclohexyl steht und

$R^{42}$ bis $R^{44}$ unabhängig voneinander für einen gegebenenfalls durch mindestens einen Rest der Auswahl Fluor, Chlor, $C_1$-bis $C_4$-Alkyl, Trifluormethyl, $C_1$-bis $C_4$-Alkoxy, Trifluormethoxy, Phenyl oder Phenoxy substituierten $C_6$- bis $C_{10}$- Arylrest stehen.

Besonders bevorzugt sind solche, wobei

$R^{41}$ für 1- oder 2-Butyl, 1,1-Dimethylethyl, 1- oder 2-Pentyl, 1- oder 2-Hexyl, 1- oder 2-Heptyl, 1- oder 2-Octyl,

2-Ethylhexyl, 1- oder 2- Nonyl, 1- oder 2-Decyl, 1-Dodecyl, 1-Tetradecyl, 1-Hexadecyl, 1-Octadecyl, Allyl, 2-Buten-1-yl, Benzyl, 2-Phenylethyl, 2- oder 3-Phenylpropyl, Cyclopentyl oder Cyclohexyl steht und

$R^{42}$ bis $R^{44}$ unabhängig voneinander für einen gegebenenfalls durch mindestens einen Rest der Auswahl Fluor, Chlor, Methyl, Trifluormethyl, Methoxy oder Trifluormethoxy in 3- und/oder 4-Stellung substituierten Phenylrest stehen.

Ganz besonders bevorzugt sind solche, wobei

$R^{41}$ für 1-Butyl, 1- Pentyl, 1-Hexyl, 1-Heptyl, 1-Octyl, 1-Nonyl, 1-Decyl, 1-Dodecyl, 1-Tetradecyl, 1-Hexadecyl, 1-Octadecyl, Allyl, 3-Phenylpropyl, Cyclopentyl oder Cyclohexyl steht und

$R^{42}$ bis $R^{44}$ für 4-Fluorphenyl, 4-Chlorphenyl, 3-Fluor-4-methylphenyl oder 3-Chlor-4-methylphenyl stehen und

$R^{42}$ bis $R^{44}$ gleich sind.

Des Weiteren bevorzugt sind solche, wobei

$R^{41}$ für 1-Butyl, 1-Hexyl, 1-Octyl, 1-Dodecyl oder 3-Phenylpropyl steht,

$R^{42}$ bis $R^{44}$ für 4-Fluorphenyl, 4-Chlorphenyl, 3-Fluor-4-methylphenyl oder 3-Chlor-4-methylphenyl stehen und

$R^{42}$ bis $R^{44}$ gleich sind.

In einer speziellen Ausführungsform der erfindungsgemäßen Borate bilden die oben genannten Reste $R^{32}$, $R^{33}$ und $R^{34}$ gemeinsam mit dem $N^+$ einen Imidazol- oder Pyridin-Ring, der einer der Formeln

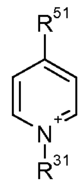 (IX), 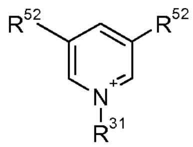 (X) oder 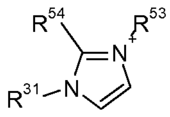 (XI)

entspricht, worin

$R^{31}$ für einen gegebenenfalls verzweigten $C_{14}$- bis $C_{22}$-Alkyl-Rest steht und

$R^{51}$ und $R^{53}$ unabhängig voneinander für einen gegebenenfalls verzweigten $C_3$- bis Cs-Alkylrest, Cyclopentyl, Cyclohexyl, Cycloheptyl oder Phenyl stehen und $R^{51}$ zusätzlich für einen gegebenenfalls verzweigten $C_3$- bis Cs-Alkoxyrest oder Phenoxy und $R^{53}$ zusätzlich für Phenyl oder Benzyl stehen,

$R^{52}$ für einen $C_1$- bis $C_4$-Alkylrest steht und

$R^{54}$ für Wasserstoff, einen $C_1$- bis $C_4$-Alkylrest oder Phenyl steht.

Besonders bevorzugt steht dabei

$R^{31}$ für Tetradecyl, Hexadecyl, Octadecyl, Eicosyl oder Docosyl, besonders bevorzugt für Hexadecyl oder Octadecyl,

$R^{51}$ und $R^{53}$ unabhängig voneinander für 1- oder 2-Propyl, 1- oder 2-Butyl, 1,1-Dimethylethyl, 1-Hexyl, 1-Octyl, 2-Ethylhexyl, Cyclopentyl, Cyclohexyl, Cycloheptyl oder Phenyl, besonders bevorzugt für 1- oder 2-Butyl, 1,1-Dimethylethyl, Cyclohexyl oder Phenyl stehen und $R^{31}$ zusätzlich für 1- oder 2-Propoxy, 1- oder 2-Butoxy, 1,1-Dimethylethoxy, 1-Hexoxy, 1-Octoxy, 2-Ethylhexoxy oder Phenoxy, besonders bevorzugt für 1- oder 2-Butoxy, 1,1-Dimethylethoxy oder Phenoxy, und $R^{33}$ zusätzlich für Phenyl oder Benzyl, besonders bevorzugt für Benzyl,

$R^{52}$ für Methyl, Ethyl, 1- oder 2-Propyl, 1- oder 2-Butyl oder 1,1-Dimethylethyl, besonders bevorzugt für 1- oder 2-Butyl oder 1,1-Dimethylethyl und

$R^{54}$ für Wasserstoff, Methyl, Ethyl oder Phenyl, besonders bevorzugt für Wasserstoff oder Methyl.

## Beispiele

### Ausgangssubstanzen:

Natrium-bis(2-ethylhexyl)sulfosuccinat (AClogP = 3.67)

Natrium-sec.-dodecylbenzolsulfonat (AClogP = 4.85)

### Methoden:

**Messung der Glastemperatur $T_g$ mittels DSC**

Die Messungen erfolgen in Anlehnung an die DIN EN 61006, Verfahren A. Die Kalibrierung des DSC Gerätes erfolgt mit Hilfe von Indium und Blei als Referenz.

10 mg Probenmenge wird mit Hilfe einer Mikrowaage (MT5 der Firma Mettler-Toledo) in einem geschlossenen, aber gelochten Aluminiumtiegel mit einem Volumen von 40 µl eingewogen. In einem Differential-Wärmestromkalorimeter (DSC 822 der Firma Mettler-Toledo) wird bei einer konstanten Aufheizrate von 20 K/min der Wärmestrom der Probe zu einer Referenz gemessen. Der Rezipient der DSC 822, der die Probe und die Referenz enthält, wird während der Messung mit einem Stickstoffstrom von einer Stärke von 20 ml/min durchspült.

Es wird folgender programmierter Temperaturzyklus durchfahren:

Erste Abkühlung. Die Starttemperatur beträgt 30°C, die Endtemperatur -100°C. Die Abkühlrate wird auf 50 K/min eingestellt. Bei Erreichen der Endtemperatur wird diese noch 7 min konstant gehalten.

Erstes Aufheizen. Die Starttemperatur beträgt -100°C die Endtemperatur 80°. Die Aufheizrate wird auf 20 K/min eingestellt. Dabei wird der Wärmestrom fortwährend aufgezeichnet.

Zweite Abkühlung. Die Starttemperatur beträgt 80°C die Endtemperatur -100°C. Die Abkühlrate wird auf 50 K/min eingestellt. Bei Erreichen der Endtemperatur wird diese noch 7 min konstant gehalten.

Zweites Aufheizen. Die Starttemperatur beträgt -100°C die Endtemperatur 150°. Die Aufheizrate wird auf 20 K/min eingestellt. Dabei wird der Wärmestrom fortwährend aufgezeichnet.

Dritte Abkühlung. Die Starttemperatur beträgt 150°C die Endtemperatur -100°C. Die Abkühlrate wird auf 50 K/min eingestellt. Bei Erreichen der Endtemperatur wird diese noch 7 min konstant gehalten.

Drittes Aufheizen. Die Starttemperatur beträgt -100°C die Endtemperatur 150°. Die Aufheizrate wird auf 20 K/min eingestellt. Dabei wird der Wärmestrom fortwährend aufgezeichnet.

Als Glasübergang wird eine stufenförmige Erhöhung der Wärmestromkurve beim Aufheizen identifiziert. Die Glastemperatur ist dann diejenige Temperatur, bei der die halbe Stufenhöhe in der Wärmestromkurve am Glasübergang erreicht wird.

Um die thermische Vorgeschichte der Proben zu eliminieren, werden nur Glastemperaturen des dritten Aufheizens angegeben.

**Bestimmung des Kationenverhältnisses über $^1$H-NMR:**

$^1$H-NMR wurden gemessen an einem Bruker DPX-400, 400 MHz und in $CDCl_3$ aufgenommen. Die Auswertung erfolgte über die Integrale charakteristischer Signale der einzelnen Bestandteile, siehe die Angaben bei den Beispielen.

**Ermittlung des Anteils der Borate an der Lösung mittels HPLC:**

Die HPLC Messungen wurden unter reversed phase Bedingungen unter Benutzung einer octadecylsilylmodifizierten Silicagel-Säule (endcapped) durchgeführt. Als mobile Phase wurde eine Mischung aus Acetonitril und Wasser (gepuffert, enthält ein Amin) verwendet. Die Eluierung erfolgte mit einem schrittweise geänderten Laufmittel-Gradienten. Die Detektion der Substanzen erfolgte durch UV Detektion bei 205 nm und die Quantifizierung erfolgte mit Hilfe eines externen Standards.

## Beispiele 1 bis 4: Herstellverfahren

### Beispiel 1

500 g einer Mischung aus 99,7 Gew.-% Tetrabutylammonium-hexyl-tris(3-chlor-4-methylphenyl)borat und 0,3 Gew.-% Tetrabutylammonium-tetrakis(3-chlor-4-methylphenyl)borat (Schmp. = 119-121 °C, keine $T_g$), 304 g Benzyl-dimethyl-hexadecyl-ammonium-chlorid-Hydrat (1,05 Moläquivalente) und 15,5 g Natrium-bis(2-ethylhexyl)sulfosuccinat (0,05 Moläquivalente) wurden in einer Mischung aus 1500 ml Butylacetat und 1750 ml Wasser in einer 6 1-Planschliffapparatur 2,5 h bei 25 °C gerührt. Diese Temperatur wurde bei allen Folgeschritten eingehalten. Der Rührer wurde abgestellt. Nach 30 min wurde die untere wässrige Phase über das Bodenventil abgelassen. Die organische Phase wurde 30 min mit 800 ml Wasser verrührt. Der Rührer wurde abgestellt. Nach 30 min wurde die untere wässrige Phase über das Bodenventil abgelassen. Die organische Phase wurde 30 min mit 800 ml Wasser verrührt. Diese Vorgehensweise wurde zweimal wiederholt. In der letzten Wasserphase konnten Chlorid-Ionen noch stark nachgewiesen werden. (3 ml-Probe + 0,5 ml 10-proz. $HNO_3$ + 0,5 ml 5-proz. $AgNO_3$-Lösung). Weitere viermal wurde die organische Phase mit 800 ml Wasser 30 min verrührt und schließlich nach 30 minütiger Rührpause die Wasserphase abgelassen. In der letzten Wasserphase konnten keine Chlorid-Ionen mehr nachgewiesen werden. Die organische Phase wurde am Rotationsverdampfer bei 60 °C Badtemperatur und einem Endvakuum von 60 mbar azeotrop vom gelösten und mitgerissenen Wasser befreit. Man erhielt 937,8 g einer klaren Lösung, die gemäß HPLC 53,9 Gew.-%ig an der Mischung der Borate der Formeln

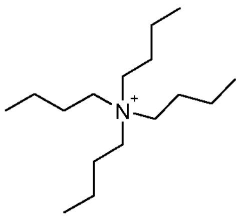
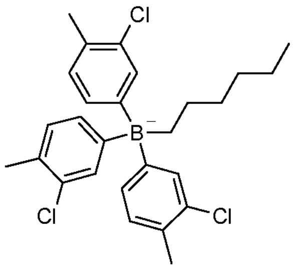
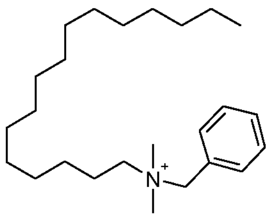
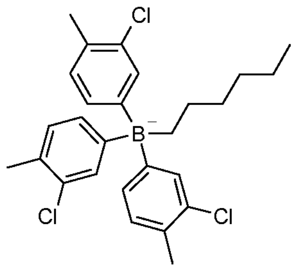

und 0,1 Gew.-%ig an der Mischung der Borate der Formeln

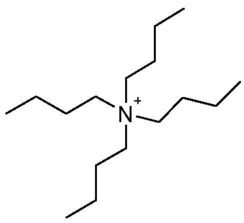
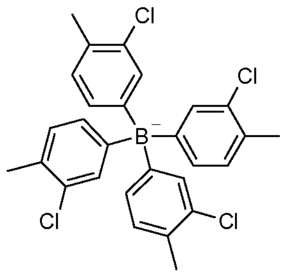
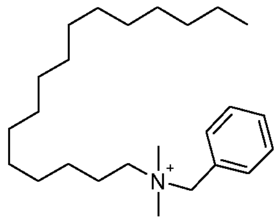
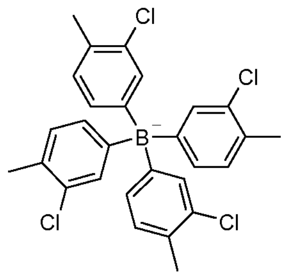

war. Die Ausbeute betrug also 94,2 % d. Th. Über $^1$H-NMR (in $CDCl_3$, charakteristische Signale für Tetrabutylammonium: $\delta$ = 2,55 ppm (m, 8H), für Benzyl-dimethyl-hexadecylammonium: $\delta$ = 6,95 ppm (d, 2H), $\delta$ = 3,40 ppm (s, 2H)) wurde das molare Verhältnis der Kationen Tetrabutyl-ammonium zu Benzyl-dimethyl-hexadecyl-ammonium zu 1,7 : 98,3 bestimmt. Ebenfalls über $^1$H-NMR (in $CDCl_3$, charakteristisches Signal für Bis(2-ethylhexyl)sulfosuccinat: $\delta$ = 3,25-3,1 ppm (m, 2H)) wurde ein Gehalt von 1,63 Gew.- % an dem Sulfosuccinat der Formel

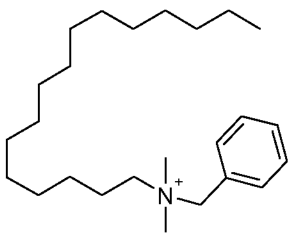
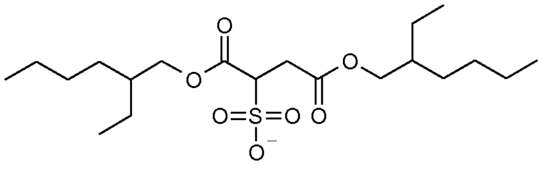

in der Lösung ermittelt, d. h. 3,02 Gew.-% bezogen auf die Mischung der Borate.

Wäschen insgesamt: 8

Zwischenphasen: 0

Nach Eindampfen eines Teils der Lösung im Vakuum und Trocken des Rückstands bei 80 °C im Vakuum erhielt man ein farbloses honigartiges Öl mit einer $T_g$ von - 27 °C.

Die obige 54 Gew.-%ige Lösung in Butylacetat ist bei -5°C und selbst bei -78 °C unbegrenzt stabil.

Zum Vergleich kristallisiert eine 30 Gew.-%ige Lösung des als Edukt benutzten Tetrabutylammoniumhexyl-tris(3-chlor-4-methylphenyl)borats in Butylacetat bereits nach 3 Tagen bei -5 °C stark aus. Nach 4 Wochen bei -5°C sind rund 75 Gew.-% dieses Ammoniumborats auskristallisiert. d. h. die Restlösung ist nur noch 7,5 Gew.-%ig.

**Beispiel 2**

Der Versuch des Beispiels 1 wurde wiederholt, jedoch wurde er bei 45-50 °C durchgeführt.

Das Edukt enthielt auch hier analog Beispiel 1 einen entsprechenden herstellungsbedingten Anteil des analogen Tetraarylborats. Dieser und auch die daraus entstehenden Produkte werden der Übersichtlichkeit halber nicht genauer angegeben.

Man erhielt 950,7 g einer klaren Lösung, die gemäß HPLC 54,6 Gew.-%ig an der Mischung der Borate der Formeln

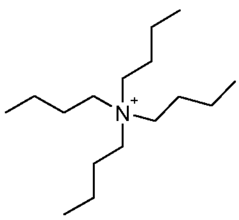
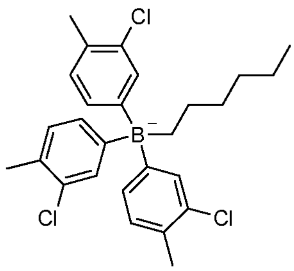
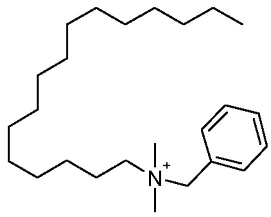
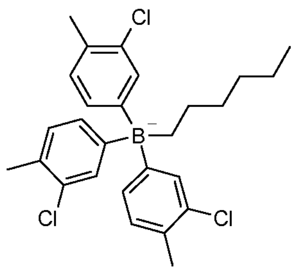

war. Die Ausbeute betrug also 96,6 % d. Th. Über $^1$H-NMR (in $CDCl_3$, Signale s. Beispiel 1) wurde das molare Verhältnis der Kationen Tetrabutyl-ammonium zu Benzyl-dimethyl-hexadecyl-ammonium zu 1,7 : 98,3 bestimmt. Ebenfalls über $^1$H-NMR (in $CDCl_3$, Signale s. Beispiel 1) wurde ein Gehalt von 1,63 Gew.-% an dem Sulfosuccinat der Formel

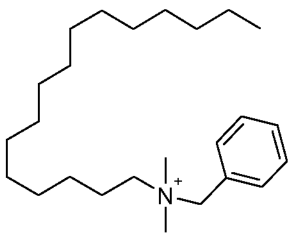
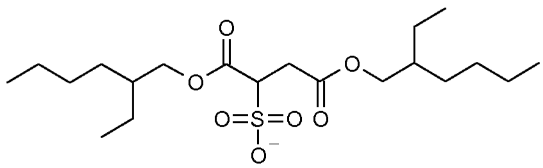

in der Lösung ermittelt, d. h. 2,98 Gew.-% bezogen auf die Mischung der Borate.

Wäschen insgesamt: 8

Zwischenphasen: 0

**Beispiel 3**

25 g Tetrabutylammonium-hexyl-tris(3-chlor-4-methylphenyl)borat, 14,8 g Benzyl-dimethyl-hexadecylammonium-chlorid-Hydrat (1,05 Moläquivalente) und 0,79 g Natrium-sec.-dodecylbenzolsulfonat (0,05 Moläquivalente, Isomerenmischung) wurden in einer Mischung aus 75 ml Butylacetat und 85 ml Wasser 2,5 h bei 25 °C gerührt. Das Edukt enthielt auch hier analog Beispiel 1 einen entsprechenden herstellungsbedingten Anteil des analogen Tetraarylborats. Dieser und auch die daraus entstehenden Produkte werden der Übersichtlichkeit halber nicht genauer angegeben. Die Temperatur wurde bei allen Folgeschritten eingehalten. Die Mischung wurde in einen Scheidetrichter überführt und die untere wässrige Phase wurde nach 30 min abgelassen. Die organische Phase wurde 30 min mit 40 ml Wasser verrührt

und wieder im Scheidetrichter nach 30 min von der wässrigen Phase getrennt. Diese Vorgehensweise wurde zweimal wiederholt. In der letzten Wasserphase konnten Chlorid-Ionen noch stark nachgewiesen werden. (3 ml-Probe + 0,5 ml 10-proz. $HNO_3$ + 0,5 ml 5-proz. $AgNO_3$-Lösung). Weitere viermal wurde die organische Phase mit 40 ml Wasser 30 min verrührt und schließlich die Wasserphase im Scheidetrichter abgetrennt. In der letzten Wasserphase konnten keine Chlorid-Ionen mehr nachgewiesen werden. Die organische Phase wurde am Rotationsverdampfer bei 60 °C Badtemperatur und einem Endvakuum von 60 mbar azeotrop vom gelösten und mitgerissenen Wasser befreit. Man erhielt 47,6 g einer klaren Lösung, die gemäß $^1$H-NMR (in $CDCl_3$, charakteristische Signale für Butylacetat: $\delta$ = 4,05 ppm (t, 2H), für Hexyl-tris(3-chlor-4-methylphenyl)borat: $\delta$ = 6,85 ppm (dd, 3H), $\delta$ = 2,20 ppm (s, 9H), für Benzyl-dimethyl-hexadecyl-ammonium: $\delta$ = 6,95 ppm (d, 2H), $\delta$ = 3,40 ppm (s, 2H)) 54,1 Gew.-%ig an der Mischung der Borate der Formeln

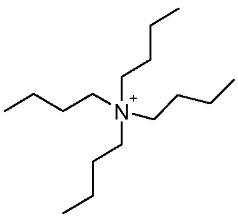 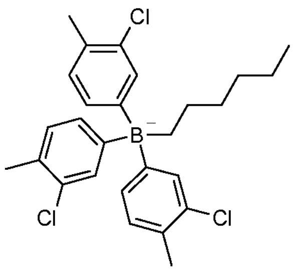 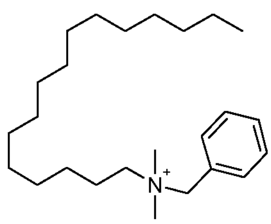 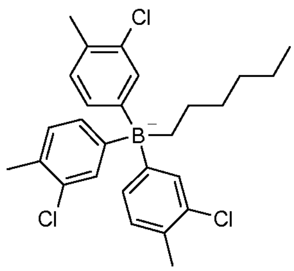

war. Die Ausbeute betrug also 96,0 % d. Th. Über $^1$H-NMR (in $CDCl_3$, Signale s. Beispiel 1) wurde das molare Verhältnis der Kationen Tetrabutyl-ammonium zu Benzyl-dimethyl-hexadecyl-ammonium zu 1,1 : 98,9 bestimmt. Ebenfalls über $^1$H-NMR (in $CDCl_3$, charakteristisches Signal für Dodecylbenzolsulfonat: $\delta$ = 7,77 ppm (dd, 2H) wurde ein Gehalt von 2,08 Gew.-% an dem sec.-Dodecylbenzolsulfonat der Formel

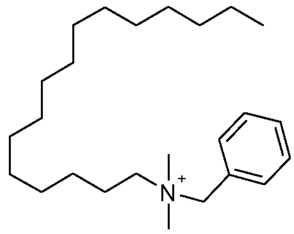 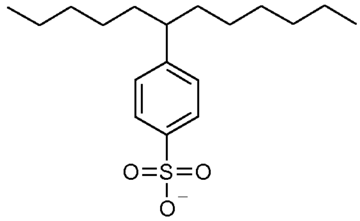

(nur 1 Isomer gezeichnet)
in der Lösung ermittelt, d. h. 3,84 Gew.-% bezogen auf die Mischung der Borate.

Wäschen insgesamt: 7

Zwischenphasen: 0

Nach Eindampfen eines Teils der Lösung im Vakuum und Trocken des Rückstands bei 80 °C im Vakuum erhielt man ein farbloses honigartiges Öl mit einer $T_g$ von -26 °C.

## Beispiel 4

5,00 g einer Mischung aus 99 Gew.-% Tetrabutylammonium-hexyl-tris(3-chlor-4-methylphenyl)borat und 1 Gew.-% Tetrabutylammonium-tetrakis(3-chlor-4-methylphenyl)borat, 3,04 g Benzyl-dimethyl-hexadecyl-ammonium-chlorid-Hydrat (1,05 Moläquivalente) und 0,155 g Natrium-bis(2-ethylhexyl)sulfosuccinat (0,05 Moläquivalente) wurden in einer Mischung aus 15 ml Butylacetat und 20 ml Wasser 2,5 h bei 25 °C gerührt. Das Edukt enthielt auch hier analog Beispiel 1 einen entsprechenden herstellungsbedingten Anteil des analogen Tetraarylborats. Dieser und auch die daraus entstehenden Produkte werden der Übersichtlichkeit halber nicht genauer angegeben. Die Temperatur wurde bei allen Folgeschritten eingehalten. Die Mischung wurde in einen Scheidetrichter überführt und die untere wässrige Phase wurde nach 10 min abgelassen. Die organische Phase wurde mit 10 ml Wasser gut geschüttelt und wieder nach 10 min von der wässrigen Phase getrennt. Diese Vorgehensweise wurde siebenmal wiederholt. In der letzten Wasserphase konnten keine Chlorid-Ionen mehr nachgewiesen werden (3 ml-Probe + 0,5 ml 10-proz. $HNO_3$ + 0,5 ml 5-proz. $AgNO_3$-Lösung). Die organische Phase wurde am Rotationsverdampfer bei 60 °C Badtemperatur und einem Endvakuum von 60 mbar azeotrop vom gelösten und mitgerissenen Wasser sowie dem Lösungsmittel befreit. Der erhaltene Honig wurde bis zur

Massenkonstanz imVakuum bei 80 °C getrocknet. Man erhielt 5,60 g eines gelblichen honigartigen Öls, das gemäß HPLC 92,0 Gew.-%ig an der Mischung der Borate der Formeln

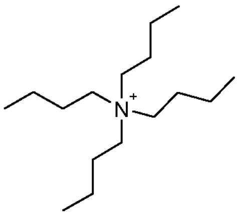
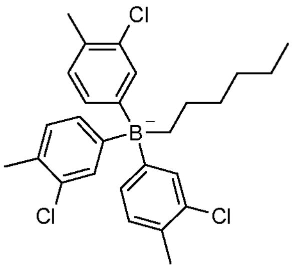
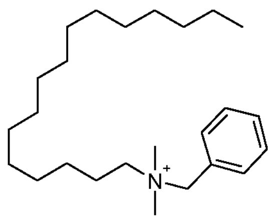
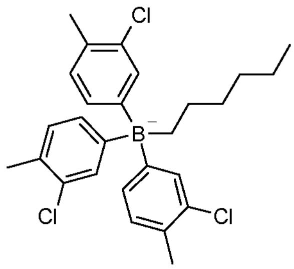

und 0,7 Gew.-%ig an der Mischung der Borate der Formeln

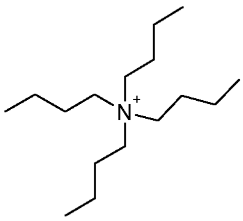
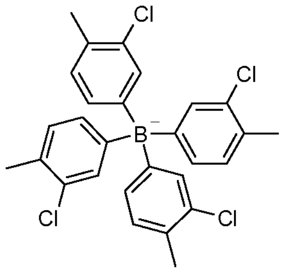
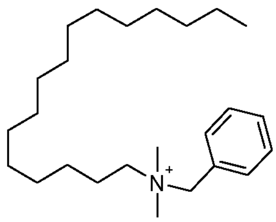
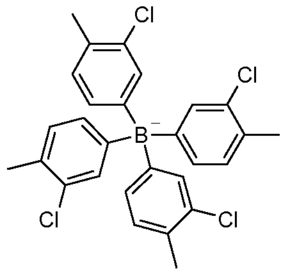

war. Die Ausbeute betrug also 88,9 % d. Th. Über $^1$H-NMR ($CDCl_3$, Signale s. Beispiel 1) wurde das molare Verhältnis der Kationen Tetrabutyl-ammonium zu Benzyl-dimethyl-hexadecyl-ammonium zu 0,3 : 99,7 bestimmt. Ebenfalls über $^1$H-NMR ($CDCl_3$, Signale s. Beispiel 1) wurde ein Gehalt von 4,34 Gew.-% an dem Sulfosuccinat der Formel

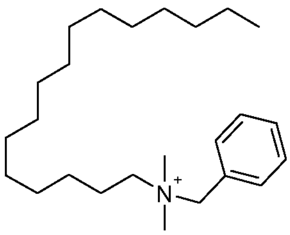
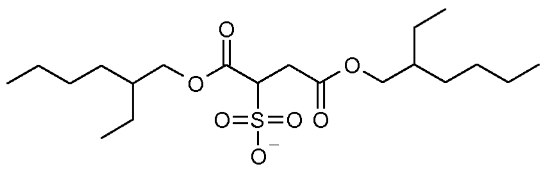

in dem honigartigen Öl ermittelt, d. h. 4,68 Gew.-% bezogen auf die Mischung der Borate.

$T_g$: - 27 °C.

Wäschen insgesamt: 8

Zwischenphasen: 0

Das Produkt wurde in 10 g Butylacetat gelöst und somit eine lagerstabile Lösung mit einer Konzentration von 34,1 Gew.-% hergestellt.

**Beipiele 5 bis 9: Variation von Ammonium und/oder Borat**

**Beispiel 5**

Es wurde analog Beispiel 1 gearbeitet, jedoch wurden 10,0 g Tetrabutylammonium-hexyl-tris(4-fluorphenyl)borat, 6,97 g Benzyl-dimethyl-hexadecyl-ammonium-chlorid-Hydrat (1,05 Moläquivalente) und 0,36 g Natrium-bis(2-ethylhexyl)sulfosuccinat (0,05 Moläquivalente) in einer Mischung aus 50 ml Butylacetat und 60 ml Wasser eingesetzt. Das Edukt enthielt auch hier analog Beispiel 1 einen entsprechenden herstellungsbedingten Anteil des analogen Tetraarylborats. Dieser und auch die daraus entstehenden Produkte werden der Übersichtlichkeit halber nicht genauer angegeben.

Gewaschen wurde mit 30 ml Wasser-Portionen. Man erhielt 27,5 g einer klaren Lösung, die gemäß $^1$H-NMR (in $CDCl_3$, charakteristische Signale für Butylacetat: $\delta$ = 4,05 ppm (t, 2H), für Hexyl-tris(4-fluorphenyl)borat: $\delta$ = 6,65 ppm (t, 6H), $\delta$ = 2,20 ppm (s, 9H), für Benzyl-dimethyl-hexadecylammonium: $\delta$ = 6,95 ppm (d, 2H), $\delta$ = 3,40 ppm (s, 2H)) 41,3-Gew.-%ig an der Mischung der Borate der Formeln

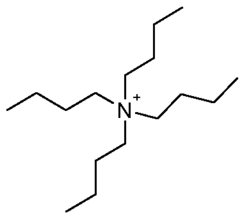
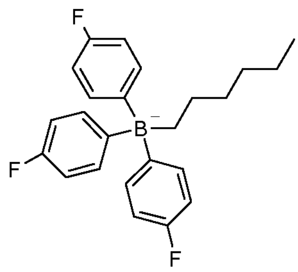
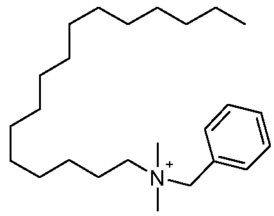
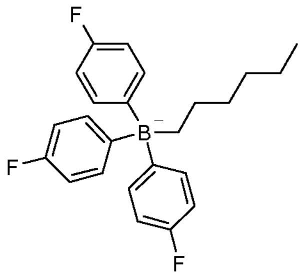

war. Die Ausbeute betrug also 96,8 % d. Th. Über $^1$H-NMR (in $CDCl_3$, Signale s. Beispiel 1) wurde das molare Verhältnis der Kationen Tetrabutyl-ammonium zu Benzyl-dimethyl-hexadecyl-ammonium zu 1,4 : 98,6 bestimmt. Ebenfalls über $^1$H-NMR (in $CDCl_3$, Signale s. Beispiel 1) wurde ein Gehalt von 1,45 Gew.-% an dem Sulfosuccinat der Formel

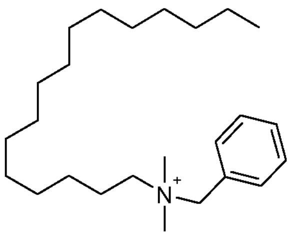
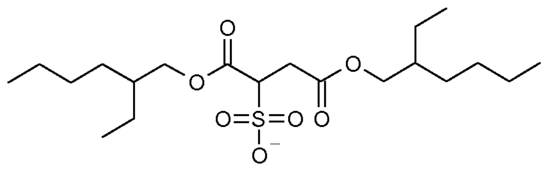

in der Lösung ermittelt, d. h. 3,51 Gew.-% bezogen auf die Mischung der Borate.

Wäschen insgesamt: 7

Zwischenphasen: 0

Nach Eindampfen eines Teils der Lösung im Vakuum und Trocken des Rückstands bei 80 °C im Vakuum erhielt man ein farbloses honigartiges Öl mit einer $T_g$ von -33 °C.

**Beispiel 6**

Es wurde analog Beispiel 1 gearbeitet, jedoch wurden 9,00 g Tetrabutylammonium-3-phenylpropyl-tris(4-fluorphenyl)borat, 5,95 g Benzyl-dimethyl-hexadecyl-ammonium-chlorid-Hydrat (1,05 Moläquivalente) und 0,31 g Natrium-bis(2-ethylhexyl)sulfosuccinat (0,05 Moläquivalente) in einer Mischung aus 60 ml Butylacetat und 75 ml Wasser eingesetzt. Das Edukt enthielt auch hier analog Beispiel 1 einen entsprechenden herstellungsbedingten Anteil des analogen Tetraarylborats. Dieser und auch die daraus entstehenden Produkte werden der Übersichtlichkeit halber nicht genauer angegeben.

Gewaschen wurde mit 50 ml Wasser-Portionen. Man erhielt 13,1 g einer klaren Lösung, die gemäß $^1$H-NMR (in $CDCl_3$, charakteristische Signale für Butylacetat: $\delta$ = 4,05 ppm (t, 2H), für Phenylpropyl-tris(4-fluorphenyl)borat: $\delta$ = 6,65 ppm (t, 6H), für Benzyl-dimethyl-hexadecyl-ammonium: $\delta$ = 6,95 ppm (d, 2H), $\delta$ = 3,40 ppm (s, 2H)) 26,2-Gew.-%ig an der Mischung der Borate der Formeln

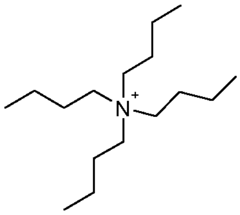
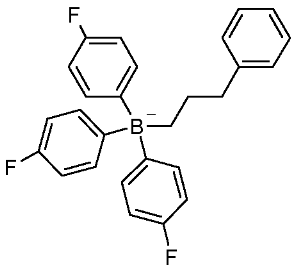
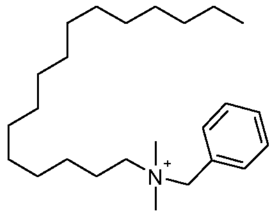
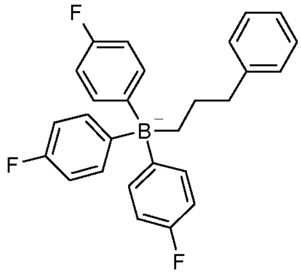

war. Die Ausbeute betrug also 97,0 % d. Th. Über $^1$H-NMR (in $CDCl_3$, Signale s. Beispiel 1) konnte Tetrabutyl-ammonium in Spurenmenge (< 0,3%) nachgewiesen werden. Das molare Verhältnis der Kationen Tetrabutyl-ammonium zu Benzyl-dimethyl-hexadecyl-ammonium wurde also zu <1 : >99 bestimmt. Ebenfalls über $^1$H-NMR (in $CDCl_3$, Signale s. Beispiel 1) wurde ein Gehalt von 0,7 Gew.-% an dem Sulfosuccinat der Formel

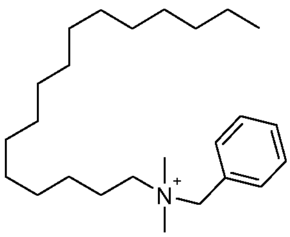
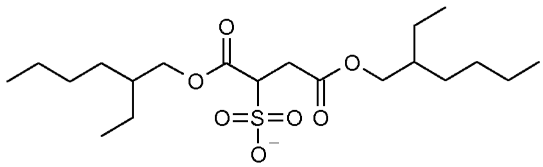

in der Lösung ermittelt, d. h. 2,67 Gew.-% bezogen auf die Mischung der Borate.

Wäschen insgesamt: 6

Zwischenphasen: 0

Nach Eindampfen eines Teils der Lösung im Vakuum und Trocken des Rückstands bei 80 °C im Vakuum erhielt man ein farbloses honigartiges Öl mit einer $T_g$ von -22 °C.

## Beispiel 7

Es wurde analog Beispiel 1 gearbeitet, jedoch wurden 9,00 g Tetrabutylammonium-dodecyl-tris(3-chlor-4-methylphenyl)borat, 4,89 g Benzyl-dimethyl-hexadecyl-ammonium-chlorid-Hydrat (1,05 Moläquivalente) und 0,25 g Natrium-bis(2-ethylhexyl)sulfosuccinat (0,05 Moläquivalente) in einer Mischung aus 60 ml Butylacetat und 75 ml Wasser eingesetzt. Das Edukt enthielt auch hier analog Beispiel 1 einen entsprechenden herstellungsbedingten Anteil des analogen Tetraarylborats. Dieser und auch die daraus entstehenden Produkte werden der Übersichtlichkeit halber nicht genauer angegeben.

Gewaschen wurde mit 50 ml Wasser-Portionen. Man erhielt 39,6 g einer klaren Lösung, die gemäß $^1$H-NMR (in $CDCl_3$, charakteristische Signale für Butylacetat: $\delta$ = 4,05 ppm (t, 2H), für Dodecyl-tris(3-chlor-4-methylphenyl)borat: $\delta$ = 6,85 ppm (dd, 3H), $\delta$ = 2,20 ppm (s, 9H), für Benzyl-dimethyl-hexadecylammonium: $\delta$ = 6,95 ppm (d, 2H), $\delta$ = 3,40 ppm (s, 2H)) 25,0-Gew.-%ig an der Mischung der Borate der Formeln

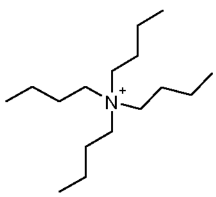
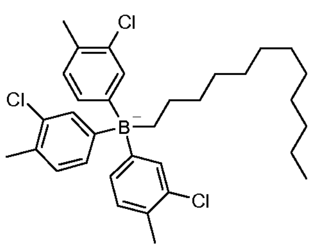
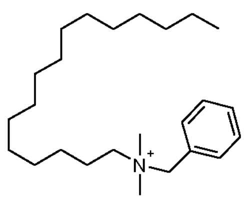
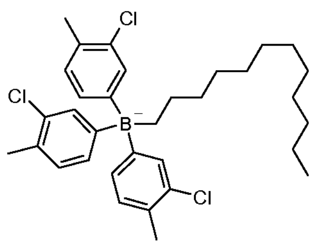

war. Die Ausbeute betrug also 95,8 % d. Th. Über $^1$H-NMR (in $CDCl_3$, Signale s. Beispiel 1) konnte Tetrabutyl-ammonium in Spurenmenge (< 0,3%) nachgewiesen werden. Das molare Verhältnis der Kationen Tetrabutyl-ammonium zu Benzyl-dimethyl-hexadecyl-ammonium wurde also zu <1 : >99 bestimmt. Ebenfalls über $^1$H-NMR (in $CDCl_3$, Signale s. Beispiel

1) wurde ein Gehalt von 1,25 Gew.-% an dem Sulfosuccinat der Formel

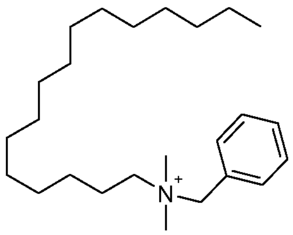

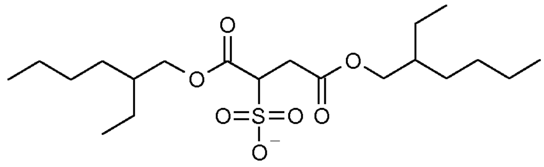

in der Lösung ermittelt, d. h. 5,00 Gew.-% bezogen auf die Mischung der Borate.

Wäschen insgesamt: 6

Zwischenphasen: 0

Nach Eindampfen eines Teils der Lösung im Vakuum und Trocken des Rückstands bei 80 °C im Vakuum erhielt man ein gelbliches honigartiges Öl mit einer $T_g$ von -47 °C.

## Beispiel 8

5,00 g Tetrabutylammonium-hexyl-tris(3-chlor-4-methylphenyl)borat, 2,91 g 1-Hexadecyl-4-tert.-butylpyridinium-chlorid (1,05 Moläquivalente) und 0,15 g Natrium-bis(2-ethylhexyl)sulfosuccinat (0,05 Moläquivalente) wurden in einer Mischung aus 20 ml Butylacetat und 30 ml Wasser 2,5 h bei 20 °C gerührt. Das Edukt enthielt auch hier analog Beispiel 1 einen entsprechenden herstellungsbedingten Anteil des analogen Tetraarylborats. Dieser und auch die daraus entstehenden Produkte werden der Übersichtlichkeit halber nicht genauer angegeben. Die Temperatur wurde bei allen Folgeschritten eingehalten. Die Mischung wurde in einen Scheidetrichter überführt und die untere wässrige Phase wurde nach 10 min abgelassen. Die organische Phase wurde mit 20 ml Wasser gut geschüttelt und wieder nach 10 min von der wässrigen Phase getrennt. Diese Vorgehensweise wurde fünfmal wiederholt. In der letzten Wasserphase konnten keine Chlorid-Ionen mehr nachgewiesen werden (3 ml-Probe + 0,5 ml 10-proz. $HNO_3$ + 0,5 ml 5-proz. $AgNO_3$-Lösung). Die organische Phase wurde am Rotationsverdampfer bei 60 °C Badtemperatur und einem Endvakuum von 60 mbar azeotrop vom gelösten und mitgerissenen Wasser sowie dem Lösungsmittel befreit. Die erhaltene honigartige Substanz wurde bis zur Massenkonstanz imVakuum bei 80 °C getrocknet. Man erhielt 5,71 g eines bräunlichen honigartigen Öls, das gemäß $^1$H-NMR (in $CDCl_3$, charakteristische Signale für Butylacetat: $\delta$ = 4,05 ppm (t, 2H), für Hexyl-tris(3-chlor-4-methylphenyl)borat: $\delta$ = 6,85 ppm (dd, 3H), $\delta$ = 2,20 ppm (s, 9H), für 1-Hexadecyl-4-tert.-butylpyridinium: $\delta$ = 7,10 ppm (d, 2H), $\delta$ = 3,50 ppm (m, 2H)) zu 96,5 Gew.-% aus einer Mischung der Borate der Formeln

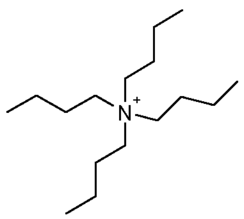

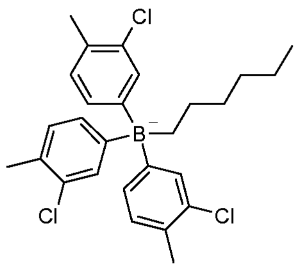

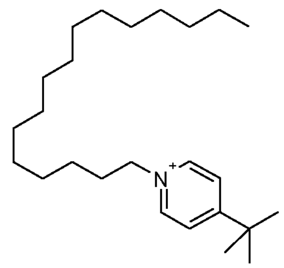

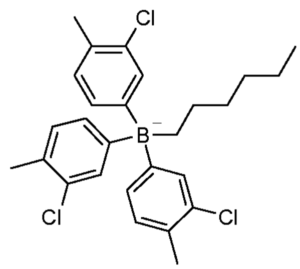

bestand. Die Ausbeute betrug also 94,6 % d. Th. Über $^1$H-NMR (in $CDCl_3$, charakteristische Signale für Tetrabutylammonium: $\delta$ = 2,55 (m, 8H), für 1-Hexadecyl-4-tert.-butylpyridinium: $\delta$ = 7,10 ppm (d, 2H), $\delta$ = 3,50 ppm (m, 2H)) wurde das molare Verhältnis der Kationen Tetrabutyl-ammonium zu 1-Hexadecyl-4-tert.-butylpyridinium zu 3,5 : 96,5 bestimmt. Ebenfalls über $^1$H-NMR (in $CDCl_3$, Signale s. Beispiel 1) wurde ein Gehalt von 3,5 Gew.-% an dem Bis(2-ethylhexyl)sulfosuccinat der Formel

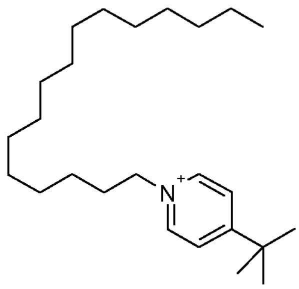

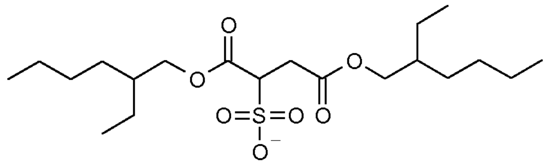

ermittelt, d. h. 3,63 Gew.-% bezogen auf die Mischung der Borate.

$T_g$: -22 °C.

Wäschen insgesamt: 6

Zwischenphasen: 0

Das als Edukt benötigte 1-Hexadecyl-4-tert.-butylpyridinium-chlorid wurde folgendermaßen hergestellt:

4,00 g 4-tert.-Butylpyridin und 19,7 g 1-Chlorhexadecan wurden mit einer Mikrospatelspitze Tetrabutylammoniumiodid versetzt und 24 h bei 130-135 °C gerührt. Nach dem Abkühlen wurde die erhaltene pastöse Kristallmasse mit 30 ml Cyclohexan aufgekocht, wobei sie teilweise in Lösung ging, und wieder kalt gerührt. Es wurde abgesaugt, mit 40 ml Cyclohexan gewaschen. Das pastöse Produkt wurde schließlich in 50 ml Diethylether verrührt, bis sich eine gut rührbare Suspension ergab. Diese wurde abgesaugt und mit 30 ml Diethylether gewaschen. Der beige Feststoff wurde bei 50 °C im Vakuum getrocknet. Man erhielt 5,85 g (29,2 % d. Th.) 1-Hexadecyl-4-tert.-butylpyridinium-chlorid.

## Beispiel 9

3,50 g Tetrabutylammonium-hexyl-tris(3-chlor-4-methylphenyl)borat, 2,15 g 1-Benzyl-3-hexadecyl-imidazolimchlorid (1,05 Moläquivalente, hergestellt nach Heterocycles **2010,** *80,* 989) und 0,11 g Natrium-bis(2-ethylhexyl)sulfosuccinat (0,05 Moläquivalente) wurden in einer Mischung aus 20 ml Butylacetat und 30 ml Wasser 2,5 h bei 20 °C gerührt. Das Edukt enthielt auch hier analog Beispiel 1 einen entsprechenden herstellungsbedingten Anteil des analogen Tetraarylborats. Dieser und auch die daraus entstehenden Produkte werden der Übersichtlichkeit halber nicht genauer angegeben.Die Temperatur wurde bei allen Folgeschritten eingehalten. Die Mischung wurde in einen Scheidetrichter überführt und die untere wässrige Phase wurde nach 10 min abgelassen. Die organische Phase wurde mit 10 ml Wasser gut geschüttelt und wieder nach 10 min von der wässrigen Phase getrennt. Diese Vorgehensweise wurde dreimal wiederholt. In der letzten Wasserphase konnten keine Chlorid-Ionen mehr nachgewiesen werden (3 ml-Probe + 0,5 ml 10-proz. $HNO_3$ + 0,5 ml 5-proz. $AgNO_3$-Lösung). Die organische Phase wurde am Rotationsverdampfer bei 60 °C Badtemperatur und einem Endvakuum von 60 mbar azeotrop vom gelösten und mitgerissenen Wasser sowie dem Lösungsmittel befreit. Die erhaltene honigartige Substanz wurde bis zur Massenkonstanz imVakuum bei 80 °C getrocknet. Man erhielt 4,14 g eines gelblichen honigartigen Öls, das gemäß $^1$H-NMR (in $CDCl_3$, charakteristische Signale für Butylacetat: $\delta$ = 4,05 ppm (t, 2H), für Hexyl-tris(3-chlor-4-methylphenyl)borat: $\delta$ = 6,85 ppm (dd, 3H), $\delta$ = 2,20 ppm (s, 9H), für 1-Benzyl-3-hexadecylimidazolium: $\delta$ = 4,60 ppm (s, 2H)) zu 95,7 Gew.-% aus einer Mischung der Borate der Formeln

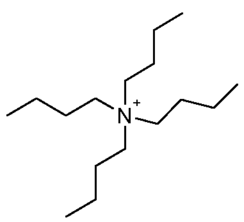

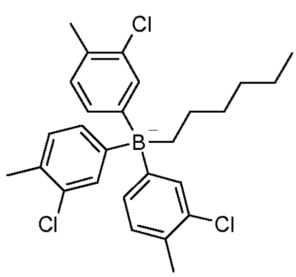

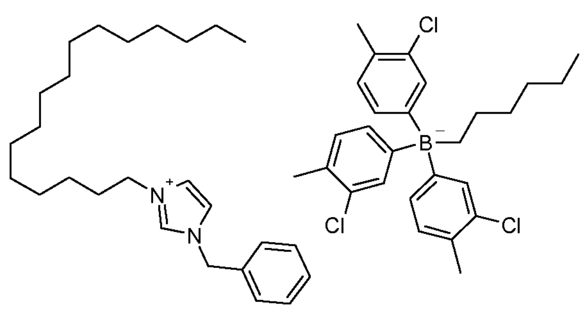

bestand. Die Ausbeute betrug also 94,6 % d. Th. Über $^1$H-NMR (in $CDCl_3$, charakteristische Signale für Tetrabutylammonium: $\delta$ = 2,55 (m, 8H), für 1-Benzyl-3-hexadecylimidazolium: $\delta$ = 4,60 ppm (s, 2H)) wurde das molare Verhältnis der Kationen Tetrabutyl-ammonium zu 1-Benzyl-3-hexadecyl-imidazolim zu 2,4 : 97,6 bestimmt. Ebenfalls über $^1$H-NMR (in $CDCl_3$, Signale s. Beispiel 1) wurde ein Gehalt von 3,5 Gew.-% an dem Bis(2-ethylhexyl)sulfosuccinat der Formel

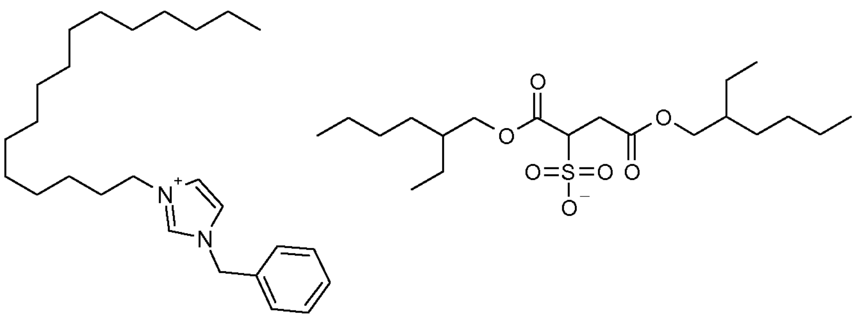

sowie 0,8 Gew.-% Butylacetat ermittelt, d. h. 3,66 Gew.-% bezogen auf die Mischung der Borate.

$T_g$: -37 °C.

Wäschen insgesamt: 4

Zwischenphasen: 0

**Beispiele 10 bis 11: Verwendung von Ammoniumsalzen mit zwei langkettigen Resten $R^1$ und $R^2$, bei denen der Zusatz des Salzes der Formel (II) nicht nötig ist**

**Beispiel 10**

4,00 g Tetrabutylammonium-hexyl-tris(3-chlor-4-methylphenyl)borat, 3,28 g Distearyl-dimethylammonium-chlorid (1,00 Moläquivalente) wurden in einer Mischung aus 50 ml Butylacetat und 30 ml Wasser 5 h bei 25 °C gerührt. Das Edukt enthielt auch hier analog Beispiel 1 einen entsprechenden herstellungsbedingten Anteil des analogen Tetraarylborats. Dieser und auch die daraus entstehenden Produkte werden der Übersichtlichkeit halber nicht genauer angegeben. Die Temperatur wurde bei allen Folgeschritten eingehalten. Die Mischung wurde in einen Scheidetrichter überführt und die untere wässrige Phase wurde nach 10 min abgelassen. Die organische Phase wurde mit 20 ml Wasser gut geschüttelt und wieder nach 10 min von der wässrigen Phase getrennt. Diese Vorgehensweise wurde fünfmal wiederholt. In der letzten Wasserphase konnten keine Chlorid-Ionen mehr nachgewiesen werden (3 ml-Probe + 0,5 ml 10-proz. $HNO_3$ + 0,5 ml 5-proz. $AgNO_3$-Lösung). Die organische Phase wurde am Rotationsverdampfer bei 60 °C Badtemperatur und einem Endvakuum von 60 mbar azeotrop vom gelösten und mitgerissenen Wasser sowie dem Lösungsmittel befreit. Die erhaltene honigartige Substanz wurde bis zur Massenkonstanz imVakuum bei 80 °C getrocknet. Man erhielt 5,10 g eines gelblichen honigartiges Öls, das gemäß $^1$H-NMR (in $CDCl_3$, charakteristische Signale für Butylacetat: $\delta$ = 4,05 ppm (t, 2H), für Hexyl-tris(3-chlor-4-methylphenyl)borat: $\delta$ = 6,85 ppm (dd, 3H), $\delta$ = 2,20 ppm (s, 9H), Signal für Distearyl-dimethylammonium: $\delta$ = 1,85 ppm (s, 6H)) ausschließlich aus dem Borat der Formel

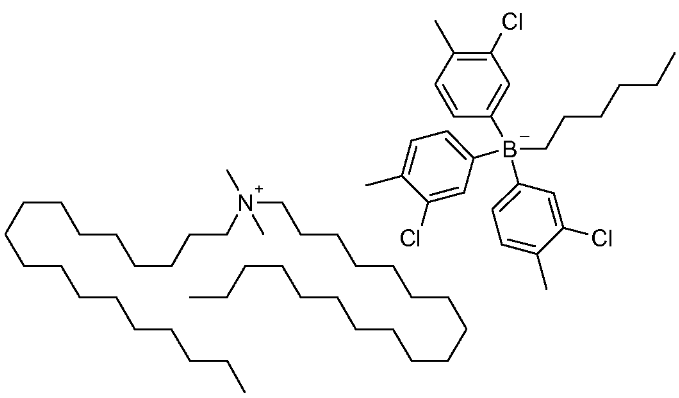

bestand. Die Ausbeute betrug also 89,1 % d. Th. Das molare Verhältnis der Kationen Tetrabutylammonium zu Distearyl-dimethyl-ammonium betrug also 0 : 100 ($^1$H-NMR in $CDCl_3$, Signale s. Beispiel 1).

$T_g$:_ -39 °C.

Wäschen insgesamt: 6

Zwischenphasen: 0

Das Produkt wurde in 9 g Butylacetat gelöst und somit eine lagerstabile Lösung mit einer Konzentration von 36 Gew.-% hergestellt.

**Beispiel 11**

3,32 g Tetrabutylammonium-hexyl-tris(3-chlor-4-methylphenyl)borat, 2,91 g N,N-Dioctadecylpiperidinium-chlorid (hergestellt nach J. Amer. Chem. Soc. 1955, 77, 485) (1,00 Moläquivalente) wurden in einer Mischung aus 15 ml Butylacetat und 20 ml Wasser 5 h bei 25 °C gerührt. Das Edukt enthielt auch hier analog Beispiel 1 einen entsprechenden herstellungsbedingten Anteil des analogen Tetraarylborats. Dieser und auch die daraus entstehenden Produkte werden der Übersichtlichkeit halber nicht genauer angegeben. Die Temperatur wurde bei allen Folgeschritten eingehalten. Die Mischung wurde in einen Scheidetrichter überführt und die untere wässrige Phase wurde nach 10 min abgelassen. Die organische Phase wurde mit 20 ml Wasser gut geschüttelt und wieder nach 10 min von der wässrigen Phase getrennt. Diese Vorgehensweise wurde fünfmal wiederholt. In der letzten Wasserphase konnten keine Chlorid-Ionen mehr nachgewiesen werden (3 ml-Probe + 0,5 ml 10-proz. $HNO_3$ + 0,5 ml 5-proz. $AgNO_3$-Lösung). Die organische Phase wurde am Rotationsverdampfer bei 60 °C Badtemperatur und einem Endvakuum von 60 mbar azeotrop vom gelösten und mitgerissenen Wasser sowie dem Lösungsmittel befreit. Der erhaltene Honig wurde bis zur Massenkonstanz imVakuum bei 80 °C getrocknet. Man erhielt 4,47 g eines gelblichen honigartigen Öls, das gemäß $^1$H-NMR ($CDCl_3$, charakteristische Signale für Butylacetat: $\delta$ = 4,05 ppm (t, 2H), für Hexyl-tris(3-chlor-4-methylphenyl)borat: $\delta$ = 6,85 ppm (dd, 3H), $\delta$ = 2,20 ppm (s, 9H), für Doctadecylpiperidinium: $\delta$ = 2,40 ppm (m, 4H)) ausschließlich aus dem Borat der Formel

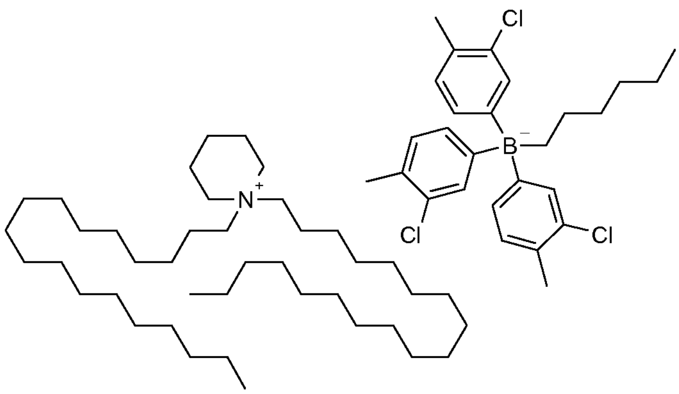

bestand. Die Ausbeute betrug also 90,5 % d. Th. Das molare Verhältnis der Kationen Tetrabutylammonium zu N,N-Dioctadecylpiperidinium betrug also 0:100 ($^1$H-NMR in $CDCl_3$, Signale s. Beispiel 1).

$T_g$: -34 °C._

Wäschen insgesamt: 6

Zwischenphasen: 0

**Beispiel 12**

2,50 g Tetrabutylammonium-tetrakis(3-chlor-4-methylphenyl)borat und 2,05 g Benzyl-dimethyl-hexadecyl-ammonium-chlorid-Hydrat (1,5 Moläquivalente) wurden in einer Mischung aus 100 ml Butylacetat und 100 ml Wasser 2,5 h bei 70 °C gerührt. Diese Temperatur wurde bei allen Folgeschritten eingehalten. Die Mischung wurde in einen Scheidetrichter überführt und die untere trübe wässrige Phase wurde nach 10 min abgelassen. Die organische Phase wurde

mit 20 ml Wasser gut geschüttelt und wieder nach 10 min von der wässrigen Phase getrennt. Diese Vorgehensweise wurde siebenmal wiederholt. In der letzten Wasserphase konnten keine Chlorid-Ionen mehr nachgewiesen werden (3 ml-Probe + 0,5 ml 10-proz. $HNO_3$ + 0,5 ml 5-proz. $AgNO_3$-Lösung). Die organische Phase wurde mit wasserfreiem Magnesiumsulfat getrocknet, filtriert und am Rotationsverdampfer bei 60 °C Badtemperatur und einem Endvakuum von 60 mbar vom Lösungsmittel befreit. Die erhaltene honigartige Substanz wurde bis zur Massenkonstanz imVakuum bei 80 °C getrocknet. Man erhielt 1,35 g eines gelblichen honigartigen Öls, das gemäß $^1$H-NMR (in $CDCl_3$, charakteristische Signale für Butylacetat: $\delta$ = 4,05 ppm (t, 2H), für Tetrakis(3-chlor-4-methylphenyl)borat: $\delta$ = 6,85 ppm (dd, 4H), $\delta$ = 2,20 ppm (s, 12H), für Benzyl-dimethyl-hexadecyl-ammonium: $\delta$ = 6,95 ppm (d, 2H), $\delta$ = 3,40 ppm (s, 2H)) 96,4 Gew.-%ig an dem Borat der Formel

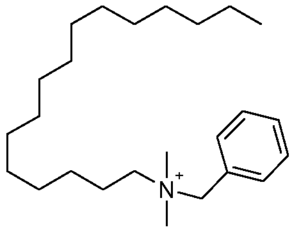 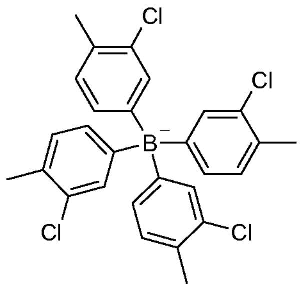

war. Die Ausbeute betrug also 46,7 % d. Th. Das molare Verhältnis Tetrabutyl-ammonium zu Benzyl-dimethyl-hexadecyl-ammonium betrug also 0:100 ($^1$H-NMR in $CDCl_3$, Signale s. Beispiel 1).

$T_g$: - 2,3 °C.

Wäschen insgesamt: 8

Zwischenphasen: 0

Es wird hier gezeigt, dass selbst das reine Tetraarylborat als Salz eines erfindungsgemäßen Kations eine $T_g$ < 0 °C aufweist.

Weitere Borate der Formel (Ia) sind in der folgenden Tabelle zusammengestellt. Sie lassen sich analog zu Beispiel 1 herstellen.

| Beispiel | 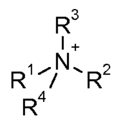 | 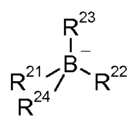 |
|---|---|---|
| 13 | 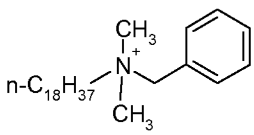 | 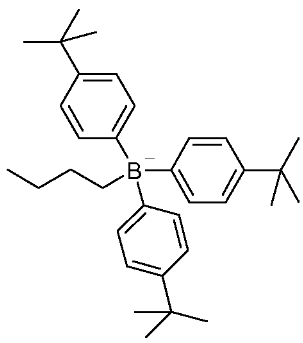 |

(fortgesetzt)

| Beispiel | 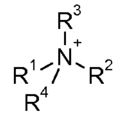 | 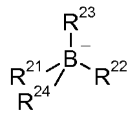 |
|---|---|---|
| 14 | 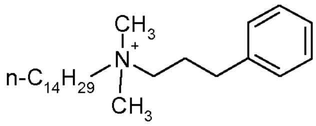 | 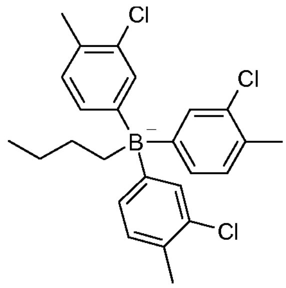 |
| 15 | 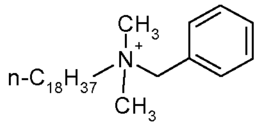 | 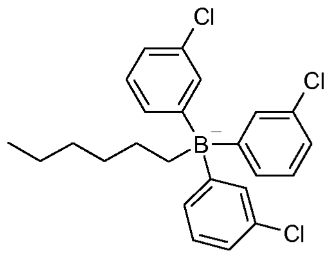 |
| 16 | 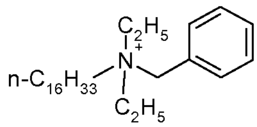 | 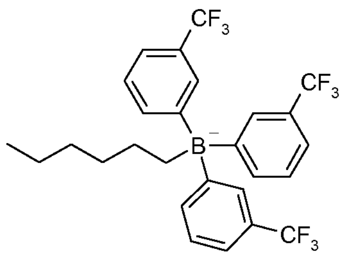 |
| 17 | 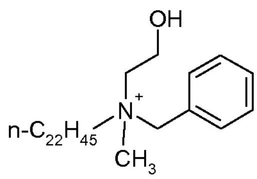 | 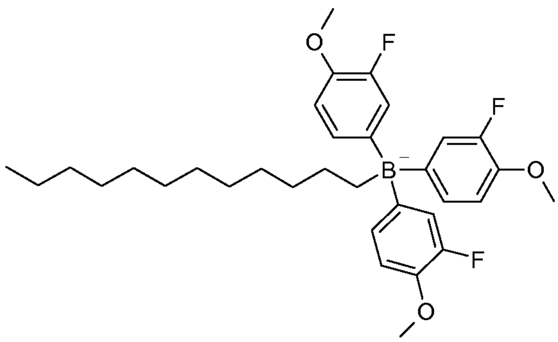 |
| 18 | 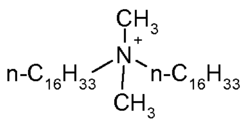 | 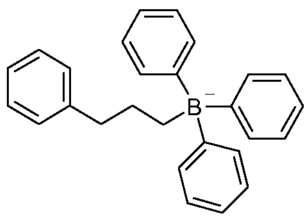 |

(fortgesetzt)

| Beispiel | 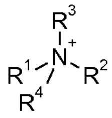 | 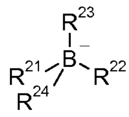 |
|---|---|---|
| 19 | 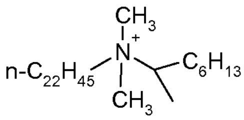 | 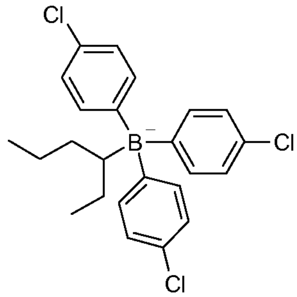 |
| 20 | 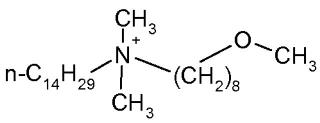 | 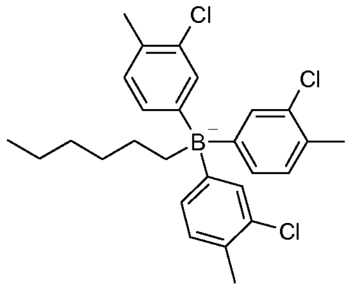 |
| 21 | 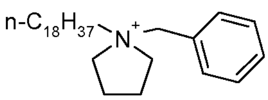 | 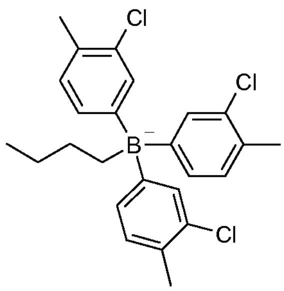 |
| 22 | 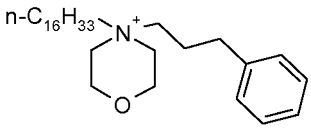 | 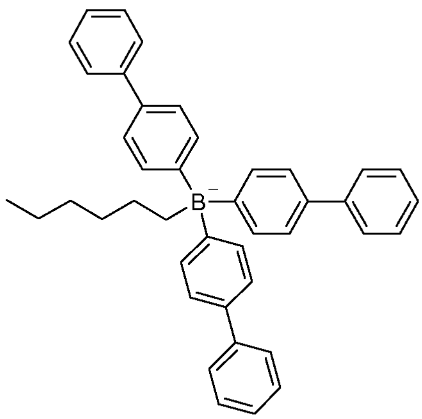 |

(fortgesetzt)

| Beispiel | 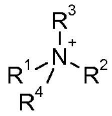 | 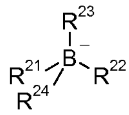 |
|---|---|---|
| 23 | 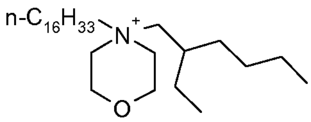 | 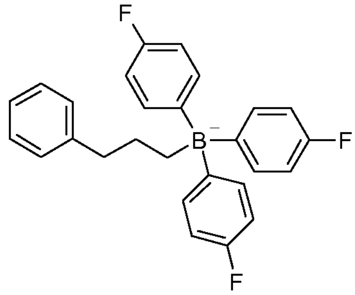 |
| 24 | 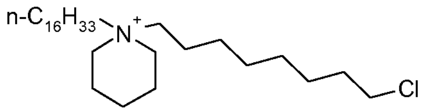 | 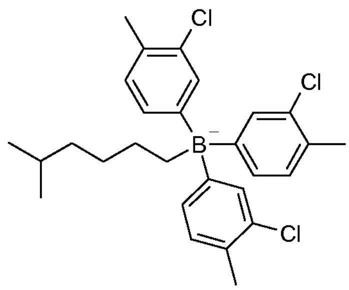 |
| 25 | 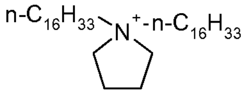 | 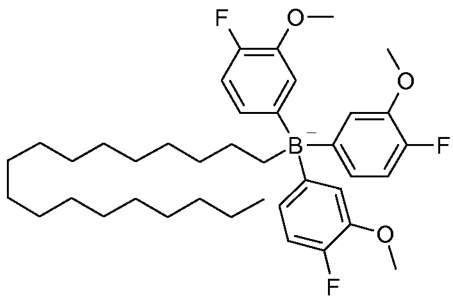 |
| 26 | 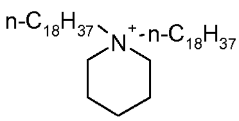 | 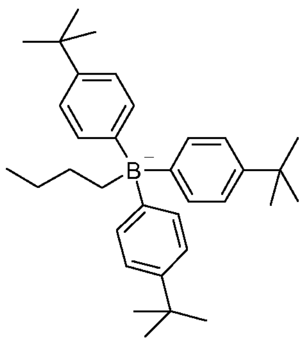 |
| 27 | 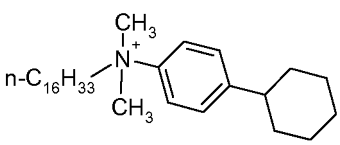 | 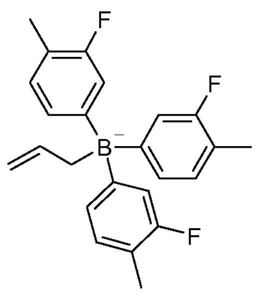 |

(fortgesetzt)

| Beispiel | 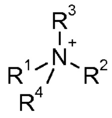 | 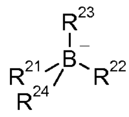 |
|---|---|---|
| 28 | 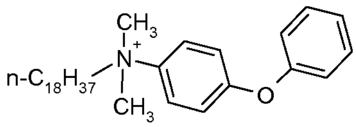 | 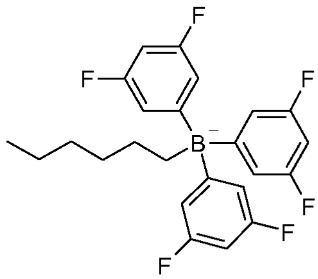 |
| 29 | 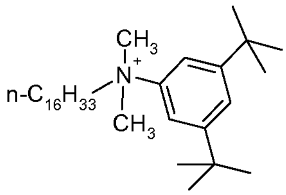 | 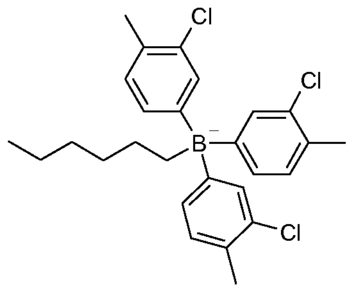 |
| 30 | 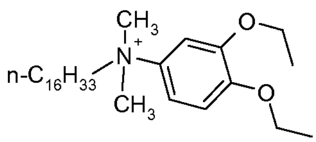 | 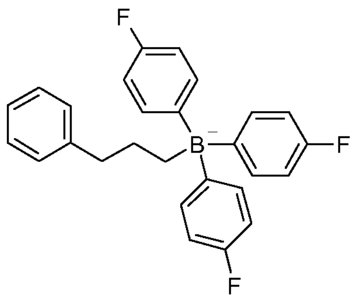 |
| 31 | 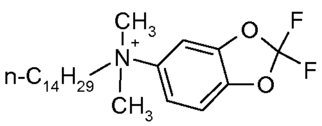 | 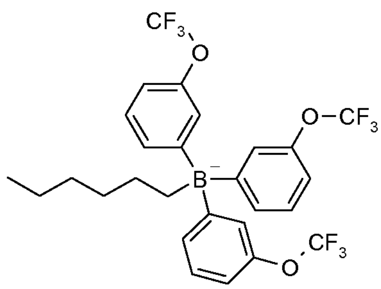 |
| 32 | 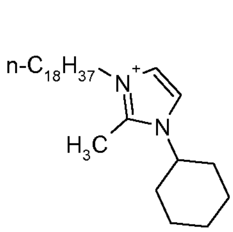 | 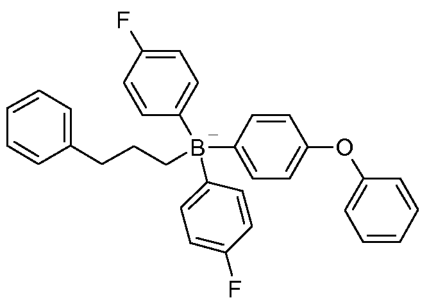 |

(fortgesetzt)

| Beispiel | 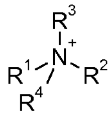 | 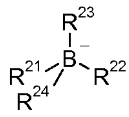 |
|---|---|---|
| 33 | 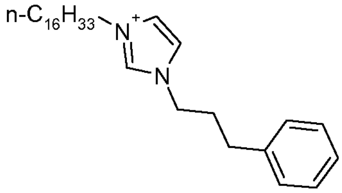 | 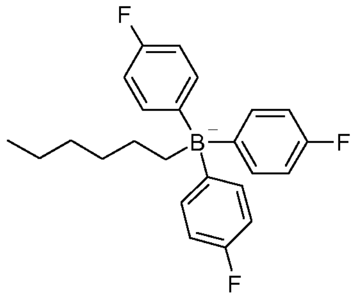 |
| 34 | 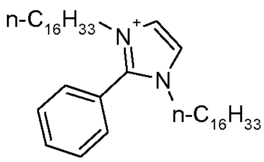 | 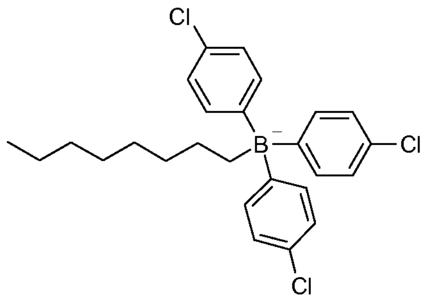 |
| 35 | 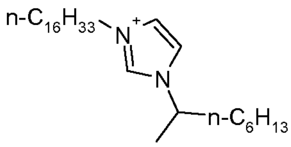 | 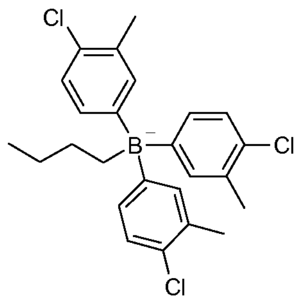 |
| 36 | 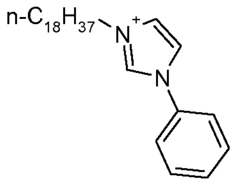 | 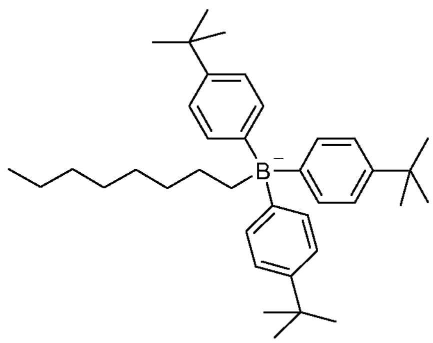 |

(fortgesetzt)

| Beispiel | 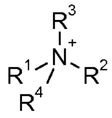 | 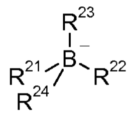 |
|---|---|---|
| 37 | 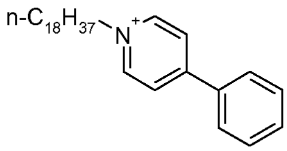 | 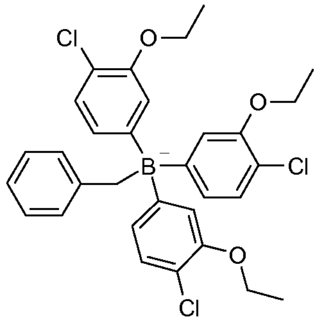 |
| 38 | 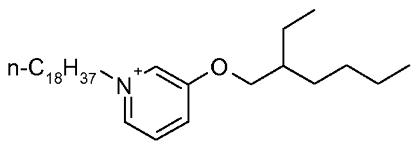 | 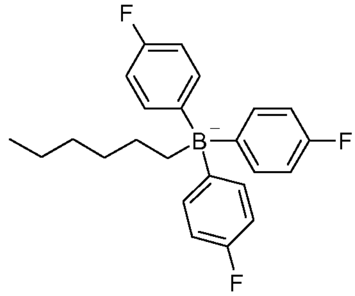 |
| 39 | 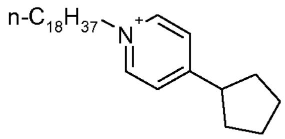 | 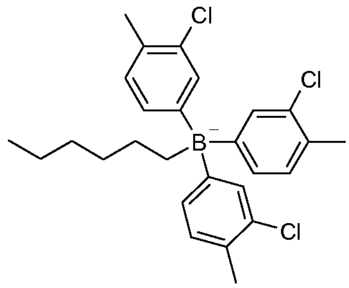 |
| 40 | 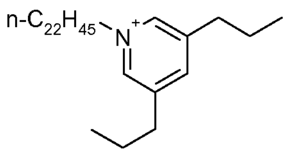 | 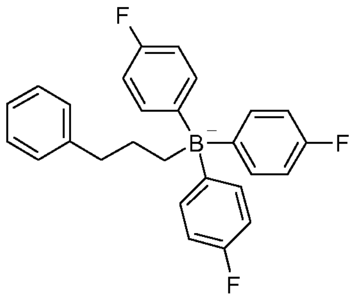 |

Die eventuell enthaltenen Ammoniumionen der Formel

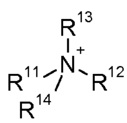

entsprechend einem Teil der Formel (IIIa) oder (IIIb) stehen für Tetramethylammonium, Tetraethylammonium,. Tetrapropylammonium oder Tetrabutylammonium; die eventuell enthaltenen Borate der Formel

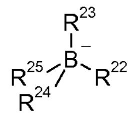

entsprechend einem Teil der Formel (Ib)

stehen für die in der Tabelle aufgeführten Borate mit der Maßgabe, dass auch der vierte Reste ein Arylrest ist. Beispielsweise für Beispiel 14 bedeutet dies dann ein Borat der Formel

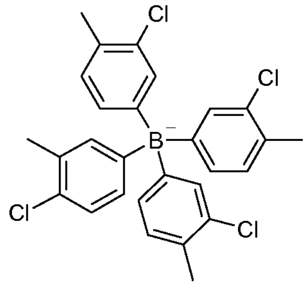

;

die eventuell enthaltenen Anionen $An^-$ entsprechend einem Teil der Formel (II) stehen vorzugsweise für Bis(2-ethylhexyl)sulfosuccinat und sec-Dodecylbenzolsulfonat.

**Vergleichsbeispiele 1 bis 5: Herstellungsverfahren**

**Vergleichsbeispiel 1**

50,0 g Tetrabutylammonium-hexyl-tris(3-chlor-4-methylphenyl)borat und 30,4 g Benzyl-dimethyl-hexadecyl-ammonium-chlorid-Hydrat (1,05 Moläquivalente) wurden in einer Mischung aus 150 ml Butylacetat und 175 ml Wasser 3 h bei Raumtemperatur gerührt. Das Edukt enthielt auch hier analog Beispiel 1 einen entsprechenden herstellungsbedingten Anteil des analogen Tetraarylborats. Dieser und auch die daraus entstehenden Produkte werden der Übersichtlichkeit halber nicht genauer angegeben. Die Mischung wurde in einen Scheidetrichter überführt und die untere wässrige Phase wurde nach 30 min abgelassen. Die organische Phase wurde 30 min mit 80 ml Wasser verrührt und wieder im Scheidetrichter nach 30 min von der wässrigen Phase getrennt. Diese Vorgehensweise wurde dreimal wiederholt. In der letzten Wasserphase konnten Chlorid-Ionen noch stark nachgewiesen werden. (3 ml-Probe + 0,5 ml 10-proz. $HNO_3$ + 0,5 ml 5-proz. $AgNO_3$-Lösung). Deshalb wurde die organische Phase wieder 30 min mit 80 ml Wasser verrührt und in den Scheidetrichter überführt. Die Phasentrennung verlief jetzt aber nur sehr langsam und unvollständig. Zwischen den Phasen hatte sich eine galertige dritte Phase gebildet. Dieser Vorgang wurde fünfmal wiederholt, wobei das Volumen der galertigen Phase bei den ersten Wäschen noch zunahm, schließlich aber wieder abnahm, so dass bei der letzten Wäsche die Wasserphase wieder komplett abgetrennt werden konnte. Diese letzte Wasserphase war auch die erste, in der keine Chlorid-Ionen mehr nachweisbar waren. Die organische Phase wurde am Rotationsverdampfer bei 60 °C Badtemperatur und einem Endvakuum von 60 mbar azeotrop vom gelösten und mitgerissenen Wasser befreit. Man erhielt 109,8 g einer klaren Lösung, die gemäß HPLC 47,5 Gew.-%ig an dem Borat der Formel

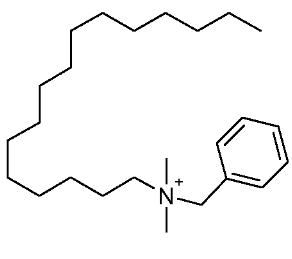

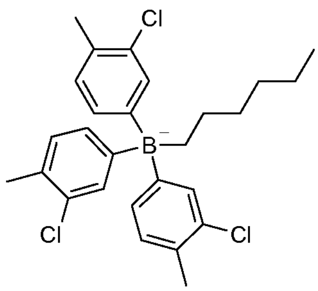

und 0,1 Gew.-%ig an dem Borat der Formel

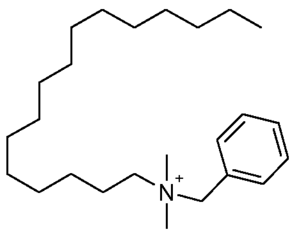
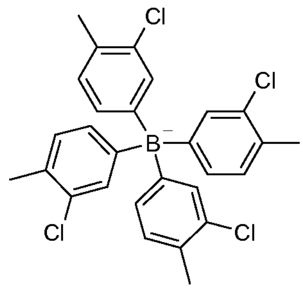

war. Die Ausbeute betrug also 96,2 % d. Th. Über $^1$H-NMR (in $CDCl_3$, Signale s. Beispiel 1) konnte kein Tetrabutylammoniumion nachgewiesen werden.

Wäschen insgesamt: 10

Zwischenphasen: 5

Im Vergleich zu Beispiel 1 wird hier die schlechte Reaktionsführung mit ungenügenden Phasentrennungen (Zwischenphasen) gezeigt, wenn man auf den Zusatz eines erfindungsgemäßen Salzes der Formel (II) verzichtet.
Auch bei allen folgenden Beispielen enthielt das Edukt Tetrabutylammonium-hexyl-tris(3-chlor-4-methylphenyl)borat einen entsprechenden herstellungsbedingten Anteil des analogen Tetraarylborats. Dieses wird aber der Übersichtlichkeit halber im Folgenden nicht mehr angegeben.

## Vergleichsbeispiel 2

100 g Tetrabutylammonium-hexyl-tris(3-chlor-4-methylphenyl)borat und 57,9 g Benzyl-dimethyl-hexadecyl-ammonium-chlorid-Hydrat (1,00 Moläquivalente) wurden in einer Mischung aus 450 ml Butylacetat und 300 ml Wasser 3 h bei Raumtemperatur gerührt. Das Edukt enthielt auch hier analog Beispiel 1 einen entsprechenden herstellungsbedingten Anteil des analogen Tetraarylborats. Dieser und auch die daraus entstehenden Produkte werden der Übersichtlichkeit halber nicht genauer angegeben.
2 g Natriumsulfat wurde zugesetzt und eine weitere Stunde gerührt. Die Mischung wurde in einen Scheidetrichter überführt und die untere wässrige Phase wurde nach 30 min abgelassen. Die organische Phase wurde 30 min mit einer Lösung von 2 g Natriumsulfat in 250 ml Wasser verrührt und wieder im Scheidetrichter nach 30 min von der wässrigen Phase getrennt. Diese Vorgehensweise wurde wiederholt, wobei jetzt eine dritte Phase zwischen der organischen und der wässrigen Phase auftrat, die aber nicht zusammen mit der Wasserphase abgelassen wurde. Zweimal wurde die organische Phase mit 250 ml Wasser 30 min verrührt und schließlich die Wasserphase abgelassen. In der letzten Wasserphase konnten Chlorid- und Sulfat-Ionen noch deutlich nachgewiesen werden. (3 ml-Probe + 0,5 ml 10-proz. $HNO_3$ + 0,5 ml 5-proz. $AgNO_3$-Lösung bzw. 3 ml-Probe + 0,5 ml 10-proz. $BaCl_2$-Lösung). Weitere viermal wurde die organische Phase mit 250 ml Wasser 30 min verrührt und schließlich die Wasserphase abgelassen. In der letzten Wasserphase konnten keine Chlorid- und Sulfat-Ionen mehr nachgewiesen werden. Die organische Phase wurde am Rotationsverdampfer bei 60 °C Badtemperatur und einem Endvakuum von 60 mbar azeotrop vom gelösten und mitgerissenen Wasser befreit. Man erhielt 412,6 g einer klaren Lösung, die gemäß HPLC 26,8 Gew.-%ig an der Mischung der Borate der Formeln

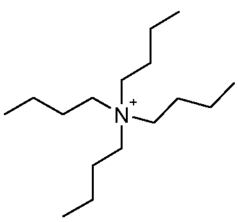
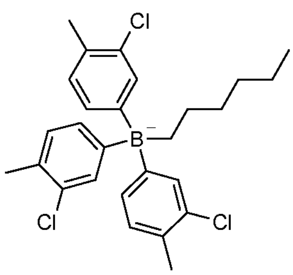
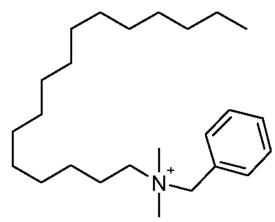
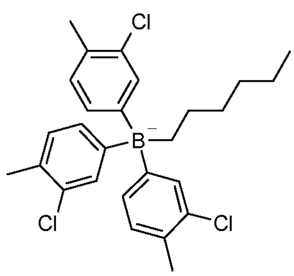

war. Die Ausbeute betrug also 99,4 % d. Th. Über $^1$H-NMR (in $CDCl_3$, Signale s. Beispiel 1) wurde das molare Verhältnis der Kationen Tetrabutyl-ammonium zu Benzyl-dimethyl-hexadecyl-ammonium zu 6 : 94 bestimmt.

Wäschen insgesamt: 8

Zwischenphasen: 1

Im Vergleich zu Beispiel 1 werden hier die schlechte Reaktionsführung mit ungenügenden Phasentrennungen (Zwischenphase) sowie der ungewünscht hohe Anteil des Tetrabutylammonium-Ions im Endprodukt gezeigt, wenn man statt des erfindungsgemäßen Salzes der Formel (II) ein anorganisches Salz einsetzt.

## Vergleichsbeispiel 3

100 g Tetrabutylammonium-hexyl-tris(3-chlor-4-methylphenyl)borat und 60,8 g Benzyl-dimethyl-hexadecyl-ammonium-chlorid-Hydrat (1,05 Moläquivalente) wurden in einer Mischung aus 300 ml Butylacetat und 350 ml Wasser 2 h bei Raumtemperatur gerührt. 55 ml einer 10 Gew.-% wässrigen Natriumchloridlösung wurden zugesetzt und eine weitere Stunde gerührt. Das Edukt enthielt auch hier analog Beispiel 1 einen entsprechenden herstellungsbedingten Anteil des analogen Tetraarylborats. Dieser und auch die daraus entstehenden Produkte werden der Übersichtlichkeit halber nicht genauer angegeben. Die Mischung wurde in einen Scheidetrichter überführt und die untere wässrige Phase wurde nach 30 min abgelassen. Die organische Phase wurde 30 min mit einer Mischung aus 170 ml Wasser und 25 ml 10 Gew.-% wässrigen Natriumchloridlösung verrührt und wieder im Scheidetrichter nach 30 min von der wässrigen Phase getrennt. Diese Vorgehensweise wurde wiederholt, wobei im Gegensatz zu Vergleichsbeispiel 2 keine dritte Phase zwischen der organischen und der wässrigen Phase auftrat. Zweimal wurde die organische Phase mit 170 ml Wasser 30 min verrührt und schließlich die Wasserphase abgelassen. In der letzten Wasserphase konnten Chlorid-Ionen noch deutlich nachgewiesen werden. (3 ml-Probe + 0,5 ml 10-proz. $HNO_3$ + 0,5 ml 5-proz. $AgNO_3$-Lösung). Weitere viermal wurde die organische Phase mit 170 ml Wasser 30 min verrührt und schließlich die Wasserphase abgelassen. In der letzten Wasserphase konnten keine Chlorid-Ionen mehr nachgewiesen werden. Die organische Phase wurde am Rotationsverdampfer bei 60 °C Badtemperatur und einem Endvakuum von 60 mbar azeotrop vom gelösten und mitgerissenen Wasser befreit. Man erhielt 207,0 g einer klaren Lösung, die gemäß HPLC 45,8 Gew.-%ig an der Mischung der Borate der Formeln

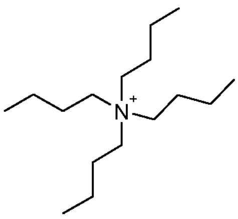 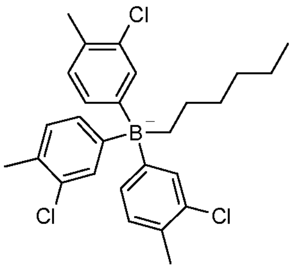 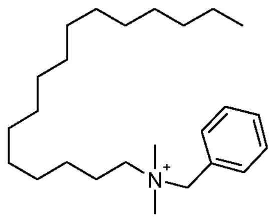 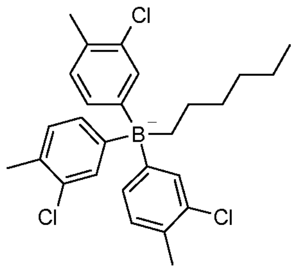

war. Die Ausbeute betrug also 87,4 % d. Th. Über $^1H$-NMR (in $CDCl_3$, Signale s. Beispiel 1) wurde das molare Verhältnis der Kationen Tetrabutyl-ammonium zu Benzyl-dimethyl-hexadecyl-ammonium zu 4 : 96 bestimmt.

Wäschen insgesamt: 8

Zwischenphasen: 0

Im Vergleich zu Beispiel 1 werden hier der ungewünscht hohe Anteil des Tetrabutylammonium-Ions im Endprodukt sowie die relativ geringe Ausbeute gezeigt, wenn man statt des erfindungsgemäßen Salzes der Formel (II) ein anorganisches Salz einsetzt.

## Vergleichsbeispiel 4

750 g Tetrabutylammonium-hexyl-tris(3-chlor-4-methylphenyl)borat und 455 g Benzyl-dimethyl-hexadecyl-ammonium-chlorid-Hydrat (1,05 Moläquivalente) wurden in einer Mischung aus 2300 ml Butylacetat und 2500 ml Wasser in einer 6 1-Planschliffapparatur gerührt. Das Edukt enthielt auch hier analog Beispiel 1 einen entsprechenden herstellungsbedingten Anteil des analogen Tetraarylborats. Dieser und auch die daraus entstehenden Produkte werden der Übersichtlichkeit halber nicht genauer angegeben.Während 45 min wurde auf 45-50 °C geheizt und 2 h bei dieser Temperatur gerührt. Die Temperatur von 45-50 °C wurde bei allen folgenden Schritten beibehalten. 400 ml 10 Gew.-% wässriger Natriumsulfatlösung wurden zugesetzt. Nach 30 min Rühren wurde der Rührer abgestellt. Nach 30 min wurde

die untere wässrige Phase über das Bodenventil abgelassen. Die organische Phase wurde 30 min mit einer Mischung aus 1000 ml Wasser und 150 ml 10 Gew.-% wässriger Natriumsulfatlösung verrührt. Der Rührer wurde abgestellt und nach 30 min wurde die untere wässrige Phase abgelassan. Der Vorgang wurde zweimal wiederholt, allerdings wurde hierbei eine Mischung aus 1000 ml Wasser und 75 ml 10 Gew.-% wässriger Natriumsulfatlösung verwendet. In beiden Fällen trat eine dritte Phase (Volumen ca. 650 ml) zwischen der organischen und der wässrigen Phase auf, die aber nicht zusammen mit der Wasserphase abgelassen wurde. Zweimal wurde die organische Phase mit 1000 ml Wasser 30 min verrührt und schließlich nach 30 minütiger Rührpause die Wasserphase abgelassen. In der letzten Wasserphase konnten Chlorid- und Sulfat-Ionen noch deutlich nachgewiesen werden. (3 ml-Probe + 0,5 ml 10-proz. $HNO_3$ + 0,5 ml 5-proz. $AgNO_3$-Lösung bzw. 3 ml-Probe + 0,5 ml 10-proz. $BaCl_2$-Lösung). Weitere viermal wurde die organische Phase mit 1000 ml Wasser 30 min verrührt und schließlich nach 30 minütiger Rührpause die Wasserphase abgelassen. In der letzten Wasserphase konnten keine Chlorid- und Sulfat-Ionen mehr nachgewiesen werden. Die organische Phase wurde in zwei Portionen am Rotationsverdampfer bei 60 °C Badtemperatur und einem Endvakuum von 60 mbar azeotrop vom gelösten und mitgerissenen Wasser befreit. Man erhielt 1462 g einer klaren Lösung, die gemäß HPLC 54,1 Gew.-%ig an der Mischung der Borate der Formeln

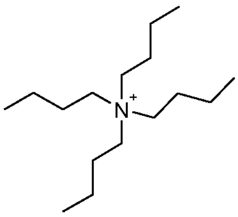 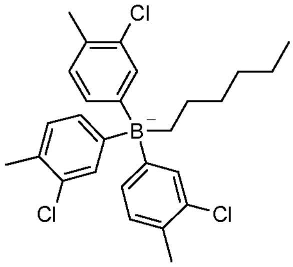 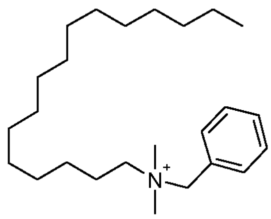 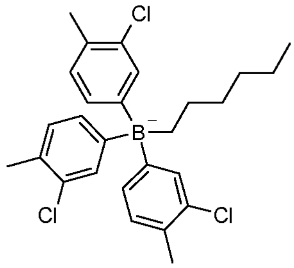

war. Die Ausbeute betrug also 98,2 % d. Th. Über $^1$H-NMR (in $CDCl_3$, Signale s. Beispiel 1) wurde das molare Verhältnis der Kationen Tetrabutyl-ammonium zu Benzyl-dimethyl-hexadecyl-ammonium zu 5 : 95 bestimmt.

Wäschen insgesamt: 9

Zwischenphasen: 2

Im Vergleich zu Beispiel 1 werden hier die schlechte Reaktionsführung mit ungenügenden Phasentrennungen (Zwischenphasen) sowie der ungewünscht hohe Anteil des Tetrabutylammonium-Ions im Endprodukt gezeigt, wenn man statt des erfindungsgemäßen Salzes der Formel (II) ein anorganisches Salz einsetzt und bei höherer Temperatur arbeitet.

**Vergleichsbeispiel 5**

500 g Tetrabutylammonium-hexyl-tris(3-chlor-4-methylphenyl)borat und 304 g Benzyl-dimethyl-hexadecyl-ammonium-chlorid-Hydrat (1,05 Moläquivalente) wurden in einer Mischung aus 1170 ml Butylacetat und 1700 ml Wasser in einer 6 1-Planschliffapparatur gerührt. Das Edukt enthielt auch hier analog Beispiel 1 einen entsprechenden herstellungsbedingten Anteil des analogen Tetraarylborats. Dieser und auch die daraus entstehenden Produkte werden der Übersichtlichkeit halber nicht genauer angegeben. Während 45 min wurde auf 45-50 °C geheizt und 2 h bei dieser Temperatur gerührt. Die Temperatur von 45-50 °C wurde bei allen folgenden Schritten beibehalten. 270 ml 10 Gew.-% wässriger Natriumchloridlösung wurden zugesetzt. Nach 30 min Rühren wurde der Rührer abgestellt. Nach 30 min wurde die untere wässrige Phase über das Bodenventil abgelassen. Die organische Phase wurde 30 min mit einer Mischung aus 415 ml Wasser und 50 ml 10 Gew.-% wässriger Natriumchloridlösung verrührt. Der Rührer wurde abgestellt und nach 30 min wurde die untere wässrige Phase abgelassan. Der Vorgang wurde zweimal wiederholt, allerdings wurde hierbei eine Mischung aus 415 ml Wasser und 25 ml 10 Gew.-% wässriger Natriumchloridlösung verwendet. In beiden Fällen trat keine dritte Phase zwischen der organischen und der wässrigen Phase auf. Viermal wurde die organische Phase mit 415 ml Wasser 30 min verrührt und schließlich nach 30 minütiger Rührpause die Wasserphase abgelassen. In der letzten Wasserphase konnten Chlorid-Ionen noch deutlich nachgewiesen werden. (3 ml-Probe + 0,5 ml 10-proz. $HNO_3$ + 0,5 ml 5-proz. $AgNO_3$-Lösung). Weitere viermal wurde die organische Phase mit 415 ml Wasser 30 min verrührt und schließlich nach 30 minütiger Rührpause die Wasserphase abgelassen. In der letzten Wasserphase konnten keine Chlorid-Ionen mehr nachgewiesen werden. Die organische Phase wurde am Rotationsverdampfer bei 60 °C Badtemperatur und einem Endvakuum von 60 mbar azeotrop vom gelösten und mitgerissenen Wasser befreit. Man erhielt 856,3 g einer klaren Lösung, die gemäß HPLC 59,2 Gew.-%tig an der Mischung der Borate der Formeln

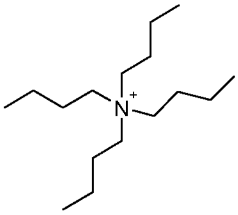
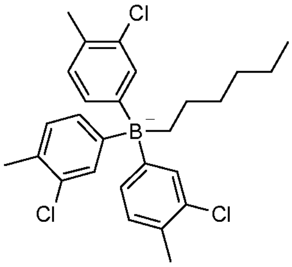
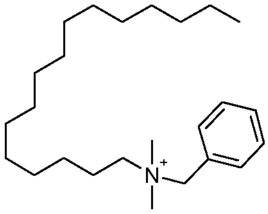
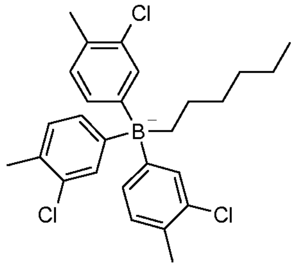

war. Die Ausbeute betrug also 95,6 % d. Th. Über $^1H$-NMR (in $CDCl_3$, Signale s. Beispiel 1) wurde das molare Verhältnis Tetrabutyl-ammonium zu Benzyl-dimethyl-hexadecyl-ammonium zu 10 : 90 bestimmt.

Wäschen insgesamt: 11

Zwischenphasen: 0

Im Vergleich zu Beispiel 1 werden hier die schlechte Reaktionsführung mit den deutlich mehr Wasserwäschen sowie der ungewünscht hohe Anteil des Tetrabutylammonium-Ions im Endprodukt gezeigt, wenn man statt des erfindungsgemäßen Salzes der Formel (II) ein anorganisches Salz einsetzt und bei höherer Temperatur arbeitet.

**Vergleichsbeispiel 6 bis 7: mit nicht erfindungsgemäßen Ammonium- und Pyrdinium-Ionen**

**Vergleichsbeispiel 6**

5,00 g Tetrabutylammonium-hexyl-tris(3-chlor-4-methylphenyl)borat und 2,75 g 1-Hexadecyl-pyridinium-chlorid (1,10 Moläquivalente) wurden in einer Mischung aus 40 ml Butylacetat und 25 ml Wasser 3 h bei 20 °C gerührt. Das Edukt enthielt auch hier analog Beispiel 1 einen entsprechenden herstellungsbedingten Anteil des analogen Tetraarylborats. Dieser und auch die daraus entstehenden Produkte werden der Übersichtlichkeit halber nicht genauer angegeben. Die Temperatur wurde bei allen Folgeschritten eingehalten. Die Mischung wurde in einen Scheidetrichter überführt und die untere wässrige Phase wurde nach 10 min abgelassen. Die organische Phase wurde mit 25 ml Wasser gut geschüttelt und wieder nach 10 min von der wässrigen Phase getrennt. Diese Vorgehensweise wurde sechsmal wiederholt. Bereits nach der vierten Wasserwäsche konnten in der Wasserphase keine Chlorid-Ionen mehr nachgewiesen werden (3 ml-Probe + 0,5 ml 10-proz. $HNO_3$ + 0,5 ml 5-proz. $AgNO_3$-Lösung). Die organische Phase wurde am Rotationsverdampfer bei 60 °C Badtemperatur und einem Endvakuum von 60 mbar azeotrop vom gelösten und mitgerissenen Wasser sowie dem Lösungsmittel befreit. Die erhaltene wachsartige Substanz wurde bis zur Massenkonstanz imVakuum bei 80 °C getrocknet. Man erhielt 4,96 g eines gelblichen Wachses, das gemäß $^1H$-NMR (in $CDCl_3$, charakteristische Signale für Butylacetat: $\delta$ = 4,05 ppm (t, 2H), für Hexyl-tris(3-chlor-4-methylphenyl)borat: $\delta$ = 6,85 ppm (dd, 3H), $\delta$ = 2,20 ppm (s, 9H), für 1-Hexadecyl-pyridinium: $\delta$ = 6,98 ppm (d, 2H), $\delta$ = 3,50 ppm (m, 2H)) 6,4% Butylacetat enthielt und somit zu 93,6 Gew.-% aus einer Mischung der Borate der Formeln

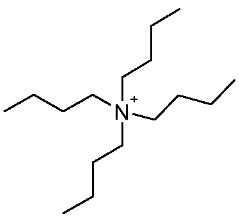
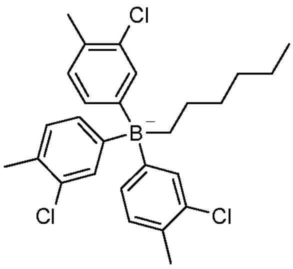
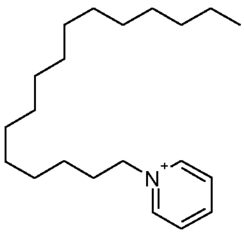
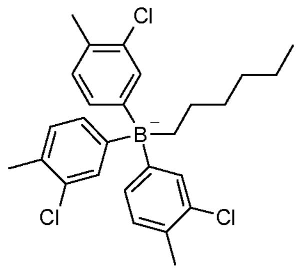

bestand. Die Ausbeute betrug also 85,4 % d. Th. Über $^1H$-NMR (in $CDCl_3$, charakteristische Signale für Tetrabutylammonium: $\delta$ = 2,55 (m, 8H), für 1-Hexadecyl-pyridinium: $\delta$ = 6,98 ppm (d, 2H), $\delta$ = 3,50 ppm (m, 2H)) wurde das molare Verhältnis Tetrabutyl-ammonium zu 1-Hexadecyl-pyridinium zu 43 : 57 bestimmt.

Wäschen insgesamt: 7

Zwischenphasen: 0

Ein vergleichbares schlechtes Resultat wird erreicht, wenn statt 1-Hexadecyl-pyridinium-chlorid 1-Docosyl-pyridinium-bromid eingesetzt wird.

Im Vergleich zu Beispiel 8 wird hier gezeigt, dass der Kationaustausch nicht in der geforderten Weise stattfindet, wenn das langkettig substituierte Pyridiniumsalz keinen erfindungsgemäßen zusätzlichen Substituenten im Ring trägt. Dies ist umso überraschender, als hier im Vergleich zu Beispiel 7 sogar ein noch größerer Überschuss des langkettig substituierten Pyridiniumsalzes eingesetzt wurde.

**Vergleichsbeispiel 7**

25,0 g Tetrabutylammonium-hexyl-tris(3-chlor-4-methylphenyl)borat, 14,8 g Methyl-trioctylammoniumchlorid (1,05 Moläquivalente) und 0,78 g Natrium-bis(2-ethylhexyl)sulfosuccinat (0,05 Moläquivalente) wurden in einer Mischung aus 75 ml Butylacetat und 85 ml Wasser 3 h bei 20 °C gerührt. Das Edukt enthielt auch hier analog Beispiel 1 einen entsprechenden herstellungsbedingten Anteil des analogen Tetraarylborats. Dieser und auch die daraus entstehenden Produkte werden der Übersichtlichkeit halber nicht genauer angegeben. Die Temperatur wurde bei allen Folgeschritten eingehalten. Die Mischung wurde in einen Scheidetrichter überführt und die untere wässrige Phase wurde nach 20 min abgelassen. Die organische Phase wurde mit 40 ml Wasser gut geschüttelt und wieder nach 20 min von der wässrigen Phase getrennt. Diese Vorgehensweise wurde viermal wiederholt. In der letzten Wasserphase konnten keine Chlorid-Ionen mehr nachgewiesen werden (3 ml-Probe + 0,5 ml 10-proz. $HNO_3$ + 0,5 ml 5-proz. $AgNO_3$-Lösung). Die organische Phase wurde am Rotationsverdampfer bei 60 °C Badtemperatur und einem Endvakuum von 60 mbar azeotrop vom gelösten und mitgerissenen Wasser befreit. Man erhielt 46,7 g einer Lösung, die gemäß $^1$H-NMR (in $CDCl_3$, charakteristische Signale für Butylacetat: $\delta$ = 4,05 ppm (t, 2H), für Hexyl-tris(3-chlor-4-methylphenyl)borat: $\delta$ = 6,85 ppm (dd, 3H), $\delta$ = 2,20 ppm (s, 9H), für Methyltrioctylammonium: $\delta$ = 1,90 ppm (s, 3H)) zu 55,7 Gew.-% aus einer Mischung der Borate der Formeln

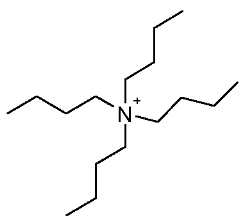
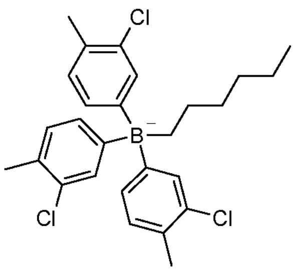
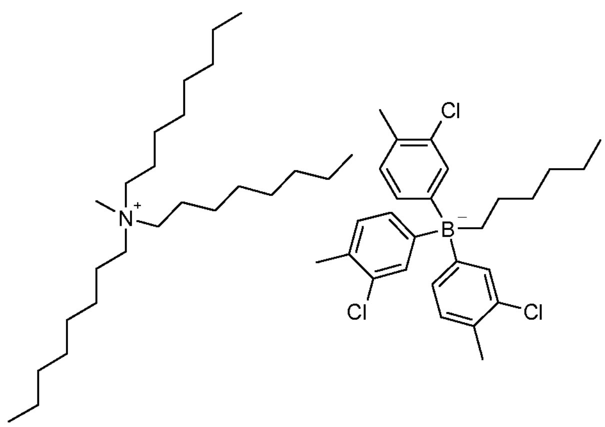

bestand. Die Ausbeute betrug also 86,6 % d. Th. Über $^1$H-NMR (in $CDCl_3$, charakteristische Signale für Tetrabutylammonium: $\delta$ = 2,55 (m, 8H), für Methyltrioctylammonium: $\delta$ = 1,90 ppm (s, 3H)) wurde das molare Verhältnis Tetrabutylammonium zu Methyl-trioctylammonium zu 19 : 81 bestimmt. Ebenfalls über $^1$H-NMR (in $CDCl_3$, Signale s. Beispiel 1) wurde ein Gehalt von 2,5 Gew.-% an dem Sulfosuccinat der Formel

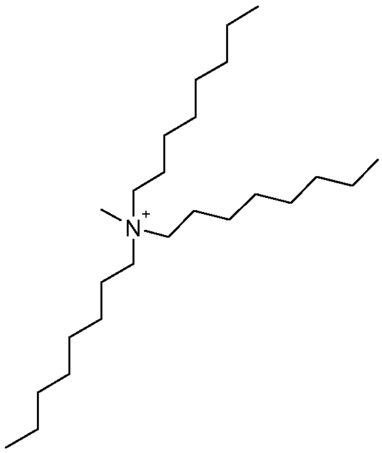

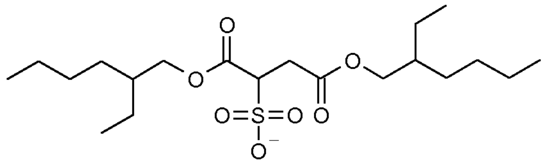

in der Lösung ermittelt, d. h. 4,5 Gew-% bezogen auf die Mischung der Borate.

Wäschen insgesamt: 5

Zwischenphasen: 0

Im Vergleich zu Beispiel 1 wird hier gezeigt, dass der Kationaustausch nicht in der geforderten Weise stattfindet, wenn das Ammoniumsalz trotz gleicher Anzahl von C-Atomen (25) nicht wenigstens einen erfindungsgemäßen $C_{14}$- bis $C_{22}$-Alkylrest trägt

**Einsatzstoffe:**

**Einsatzstoffe für die Photopolymerschichten:**

Komponente A:

experimentelles Produkt der Bayer MaterialScience AG, Leverkusen, Deutschland, die Herstellung ist unten beschrieben.

Komponente B1 (Phosphorthioyltris(oxy-4,1-phenyleniminocarbonyloxyethan-2,1-diyl)-triacrylat):

experimentelles Produkt der Bayer MaterialScience AG, Leverkusen, Deutschland, die Herstellung ist unten beschrieben.

Komponente B2 (2-({[3-(Methylsulfanyl)phenyl]carbamoyl}oxy)ethylprop-2-enoat):

experimentelles Produkt der Bayer MaterialScience AG, Leverkusen, Deutschland, die Herstellung ist unten beschrieben.

Komponente C (Bis(2,2,3,3,4,4,5,5,6,6,7,7-dodecafluorheptyl)-(2,2,4-trimethylhexan-1,6-diyl) biscarbamat):

experimentelles Produkt der Bayer MaterialScience AG, Leverkusen, Deutschland, die Herstellung ist unten beschrieben.

Komponente D:

Fascat 4102 0,07 %, Urethanisierungskatalysator, Butylzinn-tris(2-ethylhexanoat), Produkt der Arkema GmbH, Düsseldorf, Deutschland.

BYK® 310:

silikonbasiertes Oberflächenadditiv der Firma BYK-Chemie GmbH, Wesel, 25%ige Lösung in Xylol

Komponente E1:

Farbstoff 1 = C. I. Basic Blue 3 (als Bis-(2-ethylhexyl)sulfosuccinat) 0,26 %, Farbstoff 2 = Safranin O (als Bis-(2-ethylhexyl)sulfosuccinat) 0,13 % und Farbstoff 3 = Astrazon Orange G (als Bis-(2-ethylhexyl)sulfosuccinat) 0,13% mit einem der Borate 1,5%, als Lösung in 5,8% Ethylacetat gelöst. Prozentgehalte beziehen sich auf die Gesamtformulierung des Mediums.

Komponente E2:

Farbstoff 2 = Safranin O (als Bis-(2-ethylhexyl)sulfosuccinat) 0,2 % mit einem der Borate 1,5%, als Lösung in 5,8% Ethylacetat gelöst. Prozentgehalte beziehen sich auf die Gesamtformulierung des Mediums.

Die drei Farbstoffe wurden nach dem aus WO2012062655 bekannten Verfahren hergestellt.

Borate:

Borat 1 (erfindungsgemäß)

hergestellt gemäß Beispiel 1.

Borat 2 (nicht erfindungsgemäß):

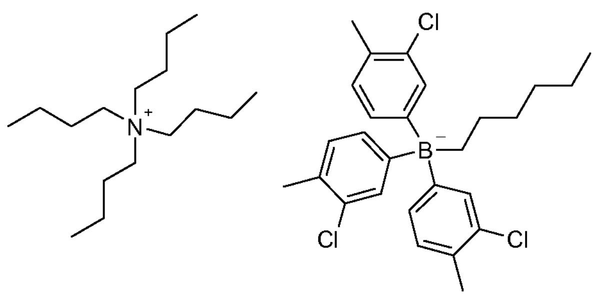

,

enthält 0,3% Tetraarylborat der Formel

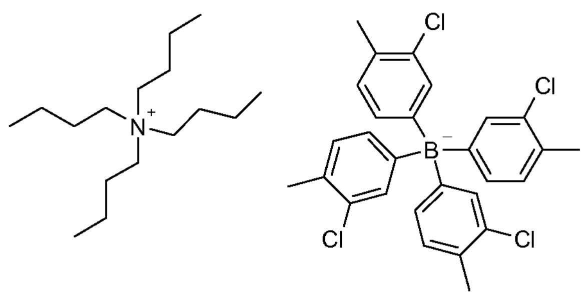

,

Schmp. = 119-121 °C, keine $T_g$

Diese Verbindung ist bekannt aus US 6,919,159, Beispiel b-5, und wurde hergestellt analog zu DE 196 48 282, Beispiel 2, Methode B, unter Verwendung von 1-Brom-3-chlor-4-methylbenzol anstelle von 1-Brom-4-chlorbenzol.

Komponente F:

Ethylacetat (CAS-Nr. 141-78-6).

Komponente G:

Desmodur® N 3900, Handelsprodukt der Bayer MaterialScience AG, Leverkusen, Deutschland, Hexandiisocyanat-basiertes Polyisocyanat, Anteil an Iminooxadiazindion mindestens 30 %, NCO-Gehalt: 23,5 %.

**Messmethoden:**

**Messung der Trockenschichtdicke der Photopolymere**

Die physikalische Schichtdicke wurde mit marktgängigen Weisslichtinterferometem ermittelt, wie z.B. das Gerät FTM-Lite NIR Schichtdickenmessgerät der Firma Ingenieursbüro Fuchs.

Die Bestimmung der Schichtdicke beruhte im Prinzip auf Interferenzerscheinungen an dünnen Schichten. Dabei überlagerten sich Lichtwellen, die an zwei Grenzflächen unterschiedlicher optischer Dichte reflektiert worden sind. Die ungestörte Überlagerung der reflektierten Teilstrahlen führte nun zur periodischen Aufhellung und Auslöschung im Spektrum eines weißen Kontinuumstrahlers (z.B. Halogenlampe). Diese Überlagerung nennt der Fachmann Interferenz. Die Interferenzspektren wurden gemessen und mathematisch ausgewertet.

**Festgehalt**

Ca. 1g der Probe wurde in einem unlackierten Dosendeckel aufgetragen und mittels einer Büroklammer gut verteilt. Dosendeckel und Büroklammer wurden zuvor gewogen. Die Probe mit Büroklammer und Dosendeckel wurden eine Stunde bei 125°C in einem Ofen getrocknet. Der Festgehalt ergab sich: (Auswaage-Tara)*100 / (Einwaage-Tara).

**Viskosität**

Die angegebenen Viskositäten wurden nach DIN EN ISO 3219/A.3 bei 23°C und einer Scherrate von 40 $s^{-1}$ bestimmt.

**Isocyanatgehalt (NCO-Gehalt)**

Die angegebenen NCO-Werte (Isocyanat-Gehalte) wurden gemäß DIN EN ISO 11909 bestimmt.

**Herstellungsvorschriften weiterer Einsatzstoffe für die holografischen Medien:**

Herstellung von Polyol-Komponente A:

In einem 1 L Kolben wurden 0,18 g Zinnoktoat, 374.8 g ε-Caprolacton und 374.8 g eines difunktionellen Polytetrahydrofuranpolyetherpolyols (Equivalentgewicht 500 g/Mol OH) vorgelegt und auf 120 °C aufgeheizt und so lange auf dieser Temperatur gehalten, bis der Festgehalt (Anteil der nicht-flüchtigen Bestandteile) bei 99.5 Gew.-% oder darüber lag. Anschließend wurde abgekühlt und das Produkt als wachsartiger Feststoff erhalten.

Herstellung von Komponente B1 (Phosphorthioyltris(oxy-4,1-phenyleniminocarbonyloxyethan-2,1-diyl)-triacrylat):

In einem 500 mL Rundkolben wurden 0,1 g 2,6-Di-tert.-butyl-4-methylphenol, 0,05 g Dibutylzinndilaurat (Desmorapid® Z, Bayer MaterialScience AG, Leverkusen, Deutschland) sowie und 213.07 g einer 27 %-igen Lösung von Tris(p-isocyanatophenyl)thiophosphat in Ethylacetat (Desmodur® RFE, Produkt der Bayer MaterialScience AG, Leverkusen, Deutschland) vorgelegt und auf 60 °C erwärmt. Anschließend wurden 42.37 g 2-Hydroxyethylacrylat zugetropft und die Mischung weiter auf 60 °C gehalten, bis der Isocyanatgehalt unter 0,1 % gesunken war. Danach wurde abgekühlt und im Vakuum das Ethylacetat vollständig entfernt. Das Produkt wurde als teilkristalliner Feststoff erhalten.

Herstellung von Komponente B2 (2-({[3-(Methylsulfanyl)phenyl]carbamoyl} oxy)ethylprop-2-enoat):

In einem 100 mL Rundkolben wurden 0,02 g 2,6-Di-tert.-butyl-4-methylphenol, 0,01 g Desmorapid® Z, 11.7 g 3-(Methylthio)phenylisocyanat vorgelegt und vorgelegt und auf 60 °C erwärmt. Anschließend wurden 8.2 g 2-Hydroxyethylacrylat zugetropft und die Mischung weiter auf 60 °C gehalten, bis der Isocyanatgehalt unter 0,1 % gesunken war. Danach wurde abgekühlt. Das Produkt wurde als hellgelbe Flüssigkeit erhalten.

Herstellung des Additives C (Bis(2,2,3,3,4,4,5,5,6,6,7,7-dodecafluorheptyl)-(2,2,4-trimethylhexan-1,6-diyl)biscarbamat):

In einem 25 mL Rundkolben wurden 0,02 g Desmorapid® Z und 3,60 g 2,4,4-Trimethylhexane-1,6-diisocyanat (TMDI) vorgelegt und auf 70 °C erwärmt. Anschließend wurden 11,39 g 2,2,3,3,4,4,5,5,6,6,7,7-Dodecafluorheptan-1-ol

zugetropft und die Mischung weiter auf 70 °C gehalten, bis der Isocyanatgehalt unter 0,1 % gesunken war. Danach wurde abgekühlt. Das Produkt wurde als farbloses Öl erhalten.

**Herstellung von holographischen Medien auf einer Folienbeschichtungsanlage**

Im Folgenden wird die kontinuierliche Herstellung von holographischen Medien in der Form von Filmen aus erfindungsgemäßen und nicht erfindungsgemäßen Photopolymer-Formulierungen beschrieben.

Im Folgenden wird die kontinuierliche Herstellung von holographischen Medien in der Form von Filmen aus erfindungsgemäßen und nicht erfindungsgemäßen Photopolymer-Formulierungen beschrieben.

Zur Herstellung wurde die in Fig. 1 dargestellte Folienbeschichtungsanlage verwendet, wobei den einzelnen Bauteilen die folgenden Bezugszeichen zugeordnet sind. Figur 1 zeigt den schematischen Aufbau der verwendeten Beschichtungsanlage. In der Figur haben die einzelnen Bauteile die folgenden Bezugszeichen:

1 Vorratsbehälter
2 Dosiereinrichtung
3 Vakuumentgasungseinrichtung
4 Filter
5 Statischer Mischer
6 Beschichtungseinrichtung
7 Umlufttrockner
8 Trägersubstrat
9 Abdeckschicht

Zur Herstellung der Photopolymer-Formulierung wurden 304,3 g der Polyol-Komponente A schrittweise mit einer Mischung aus 138 g des Schreibmonomeren B1 und 138 g des Schreibmonomeren B2, 191 g des Additivs C, 0,6 g des Katalysators D und 2,55 g des oberflächenaktiven Additivs BYK® 310 und 101 g der Komponente F versetzt und gemischt. Anschließend wurden 66,5 g einer Lösung der Komponente E1 oder E2 der Mischung im Dunklen hinzugefügt und vermischt, so dass eine klare Lösung erhalten wurde. Wenn nötig, wurde die Formulierung für kurze Zeit bei 60 °C erwärmt, um die Einsatzstoffe schneller in Lösung zu bringen. Dieses Gemisch wurde in einen der beiden Vorratsbehälter 1 der Beschichtungsanlage eingebracht. In den zweiten Vorratsbehälter 1' wurde die Polyisocyanat-Komponente G eingefüllt. Beide Komponenten wurden dann jeweils durch die Dosiereinrichtungen 2 im Verhältnis von 942,2 (Komponenten A bis F) zu 57,8 (Komponente G) zur Vakuumentgasungseinrichtung 3 gefördert und entgast. Von hier aus wurden sie dann jeweils durch die Filter 4 in den statischen Mischer 5 geleitet, in dem die Vermischung der Komponenten zur Photopolymer-Formulierung erfolgte. Die erhaltene, flüssige Masse wurde dann der Beschichtungseinrichtung 6 zugeführt.

Bei der Beschichtungseinrichtung handelte 6 handelte es sich im vorliegenden Fall um ein dem Fachmann bekanntes Rakelsystem (Doctor Blade). Alternativ kann aber auch eine Schlitzdüse zum Einsatz kommen. Mit Hilfe der Beschichtungseinrichtung 6 wurde die Photopolymer-Formulierung bei einer Verarbeitungstemperatur von 20 °C auf ein Trägersubstrat 8 in der Form einer 36 μm dicken Polyethylenterephthalatfolie appliziert und für 5,8 Minuten bei einer Vernetzungstemperatur von 80°C in einem Umlufttrockner 7 getrocknet. Dabei wurde ein Medium in der Form eines Films erhalten, der dann mit einer 40 μm dicken Polyethylenfolie als Abdeckschicht 9 versehen und aufgewickelt wurde.

Die gewünschte Zielschichtdicke des Films lag vorzugsweise zwischen 10 bis 60 μm.

Die Herstellgeschwindigkeit lag bevorzugt im Bereich von 0,2 m/min bis 300 m/min und besonders bevorzugt im Bereich vom 1,0 m/min bis 50 m/min.

Die erzielte Schichtdicke des Films lag bei 18 μm ± 1 μm.

**Messmethoden**

Messung der holographischen Eigenschaften Beugungseffizienz DE und Brechungsindexkontrast $\Delta n$ der holographischen Medien mittels Zweistrahlinterferenz in Reflexionsanordnung.

Mit einem holographischen Versuchsaufbau wie in Figur 2 dargestellt, wurden die Beugungseffizienz (DE) der Medien gemessen. Der Strahl eines DPSS Lasers (Emissionswellenlänge 532 nm) wurde mit Hilfe des Raumfilter (SF) und zusammen mit der Kollimationslinse (CL) in einen parallelen homogenen Strahl umgewandelt. Die finalen Querschnitte des Signal und Referenzstrahls werden durch die Irisblenden (I) festgelegt. Der Durchmesser der Irisblendenöffnung beträgt 0.4 cm. Die polarisationsabhängigen Strahlteiler (PBS) teilen den Laserstrahl in zwei kohärente gleich polarisierte Strahlen. Über die $\lambda/2$ Plättchen wurden die Leistung des Referenzstrahls auf 0.87 mW und die Leistung des Signalstrahls auf 1.13 mW eingestellt. Die Leistungen wurden mit den Halbleiterdetektoren (D) bei ausgebauter Probe bestimmt. Der Einfallswinkel ($\alpha_0$) des Referenzstrahls beträgt -21.8°, der Einfallswinkel ($\beta_0$) des Signalstrahls

beträgt 41.8°. Die Winkel werden ausgehend von der Probennormale zur Strahlrichtung gemessen. Gemäß Figur 2 hat daher $\alpha_0$ ein negatives Vorzeichen und $\beta_0$ ein positives Vorzeichen. Am Ort der Probe (Medium) erzeugte das Interferenzfeld der zwei überlappenden Strahlen ein Gitter heller und dunkler Streifen die senkrecht zur Winkelhalbierenden der zwei auf die Probe einfallenden Strahlen liegen (Reflexionshologramm). Der Streifenabstand A, auch Gitterperiode genannt, im Medium beträgt ~225 nm (der Brechungsindex des Mediums zu ~1.504 angenommen).

Figur 2 zeigt die Geometrie eines Holographie Media Testers (HMT) bei $\lambda$ = 532 nm (DPSS Laser): M = Spiegel, S = Verschluss, SF = Raumfilter, CL = Kollimatorlinse, $\lambda/2$ = $\lambda/2$ Platte, PBS = polarisationsempfindlicher Strahlteiler, D = Detektor, I = Irisblende, $\alpha_0$ = -21.8°, $\beta_0$ = 41.8° sind die Einfallswinkel der kohärenten Strahlen außerhalb der Probe (des Mediums) gemessen. RD = Referenzrichtung des Drehtisches.

Es wurden auf folgende Weise Hologramme in das Medium geschrieben:

- Beide Shutter (S) sind für die Belichtungszeit *t* geöffnet.
- Danach wurde bei geschlossenen Shuttern (S) dem Medium 5 Minuten Zeit für die Diffusion der noch nicht polymerisierten Schreibmonomere gelassen.

Die geschriebenen Hologramme wurden nun auf folgende Weise ausgelesen. Der Shutter des Signalstrahls blieb geschlossen. Der Shutter des Referenzstrahls war geöffnet. Die Irisblende des Referenzstrahls wurde auf einen Durchmesser < 1 mm geschlossen. Damit erreichte man, dass für alle Drehwinkel ($\Omega$) des Mediums der Strahl immer vollständig im zuvor geschriebenen Hologramm lag. Der Drehtisch überstrich nun computergesteuert den Winkelbereich von $\Omega_{min}$ bis $\Omega_{max}$ mit einer Winkelschrittweite von 0.05°. $\Omega$ wird von der Probennormale zur Referenzrichtung des Drehtisches gemessen. Die Referenzrichtung des Drehtisches ergibt sich dann wenn beim Schreiben des Hologramms der Einfallswinkel des Referenz- und des Signalstrahls betragsmäßig gleich sind also $\alpha_0$ = -31.8° und $\beta_0$ = 31.8° gilt. Dann beträgt $\Omega_{recording}$ = 0°. Für $\alpha_0$ = -21.8° und $\beta_0$ = 41.8° beträgt $\Omega_{recording}$ daher 10°. Allgemein gilt für das Interferenzfeld beim Schreiben ("recording") des Hologramms:

$$\alpha_0 = \theta_0 + \Omega_{recording} .$$

$\theta_0$ ist der Halbwinkel im Laborsystem außerhalb des Mediums und es gilt beim Schreiben des Hologramms:

$$\theta_0 = \frac{\alpha_0 - \beta_0}{2} .$$

In diesem Fall gilt also $\theta_0$ = -31.8°. An jedem angefahrenen Drehwinkel $\Omega$ wurden die Leistungen des in der nullten Ordnung transmittierten Strahls mittels des entsprechenden Detektors D und die Leistungen des in die erste Ordnung abgebeugten Strahls mittels des Detektors D gemessen. Die Beugungseffizienz ergab sich bei jedem angefahrenen Winkel $\Omega$ als der Quotient aus:

$$\eta = \frac{P_D}{P_D + P_T}$$

$P_D$ ist die Leistung im Detektor des abgebeugten Strahls und $P_T$ ist die Leistung im Detektor des transmittierten Strahls.

Mittels des oben beschriebenen Verfahrens wurde die Braggkurve, sie beschreibt den Beugungswirkungsgrad $\eta$ in Abhängigkeit des Drehwinkels $\Omega$□ des geschriebenen Hologramms gemessen und in einem Computer gespeichert. Zusätzlich wurde auch die in die nullte Ordnung transmittierte Intensität gegen den Drehwinkel $\Omega$ aufgezeichnet und in einem Computer gespeichert.

Die maximale Beugungseffizienz (DE = $\eta_{max}$) des Hologramms, also sein Spitzenwert, wurde bei $\Omega_{reconstruction}$ ermittelt. Eventuell musste dazu die Position des Detektors des abgebeugten Strahls verändert werden, um diesen maximalen Wert zu bestimmen.

Der Brechungsindexkontrast $\Delta n$ und die Dicke d der Photopolymerschicht wurde nun mittels der Coupled Wave Theorie (siehe; H. Kogelnik, The Bell System Technical Journal, Volume 48, November 1969, Number 9 Seite 2909 - Seite 2947) an die gemessene Braggkurve und den Winkelverlauf der transmittierten Intensität ermittelt. Dabei ist zu beachten, dass wegen der durch die Photopolymerisation auftretenden Dickenschwindung der Streifenabstand $\Lambda$' des Hologramms und die Orientierung der Streifen (slant) vom Streifenabstand $\Lambda$ des Interferenzmusters und dessen

Orientierung abweichen kann. Demnach wird auch der Winkel $\alpha_0$' bzw. der entsprechende Winkel des Drehtisches $\Omega_{reconstruction}$, bei dem maximale Beugungseffizienz erreicht wird von $\alpha_0$ bzw. vom entsprechenden $\Omega_{recording}$ abweichen. Dadurch verändert sich die Bragg-Bedingung. Diese Veränderung wird im Auswerteverfahren berücksichtigt. Das Auswerteverfahren wird im Folgenden beschrieben:

Alle geometrischen Größen, die sich auf das geschriebene Hologramm beziehen und nicht auf das Interferenzmuster werden als gestrichene Größen dargestellt.

Für die Braggkurve $\eta(\Omega)$ eines Reflexionshologramms gilt nach Kogelnik:

$$\eta = \begin{cases} \dfrac{1}{1 - \dfrac{1-(\xi/\nu)^2}{\sin^2\left(\sqrt{\xi^2 - \nu^2}\right)}}, \text{für } \nu^2 - \xi^2 < 0 \\ \dfrac{1}{1 + \dfrac{1-(\xi/\nu)^2}{\sinh^2\left(\sqrt{\nu^2 - \xi^2}\right)}}, \text{für } \nu^2 - \xi^2 \geq 0 \end{cases}$$

mit:

$$\nu = \frac{\pi \cdot \Delta n \cdot d'}{\lambda \cdot \sqrt{|c_s \cdot c_r|}}$$

$$\xi = -\frac{d'}{2 \cdot c_s} \cdot DP$$

$$c_s = \cos(\vartheta') - \cos(\psi') \cdot \frac{\lambda}{n \cdot \Lambda'}$$

$$c_r = \cos(\vartheta')$$

$$DP = \frac{\pi}{\Lambda'} \cdot \left( 2 \cdot \cos(\psi' - \vartheta') - \frac{\lambda}{n \cdot \Lambda'} \right)$$

$$\psi' = \frac{\beta' + \alpha'}{2}$$

$$\Lambda' = \frac{\lambda}{2 \cdot n \cdot \cos(\psi' - \alpha')}$$

Beim Auslesen des Hologramms ("reconstruction") gilt wie analog oben dargestellt:

$$\vartheta'_0 = \theta_0 + \Omega$$

$$\sin(\vartheta'_0) = n \cdot \sin(\vartheta')$$

An der Bragg-Bedingung ist das "Dephasing" *DP* = 0. Und es folgt entsprechend:

$$\alpha'_0 = \theta_0 + \Omega_{reconstruction}$$

$$\sin(\alpha'_0) = n \cdot \sin(\alpha')$$

Der noch unbekannt Winkel β' kann aus dem Vergleich der Bragg-Bedingung des Interferenzfeldes beim Schreiben des Hologramms und der Bragg-Bedingung beim Auslesen des Hologramms ermittelt werden unter der Annahme, dass nur Dickenschwindung stattfindet. Dann folgt:

$$\sin(\beta') = \frac{1}{n} \cdot \left[\sin(\alpha_0) + \sin(\beta_0) - \sin(\theta_0 + \Omega_{reconstruction})\right]$$

v ist die Gitterstärke, ξ ist der Detuning Parameter und ψ' die Orientierung (Slant) des Brechungsindexgitters das geschrieben wurde. α' und β' entsprechen den Winkeln ao und ßo des Interferenzfeldes beim Schreiben des Hologramms, aber im Medium gemessen und für das Gitter des Hologramms gültig (nach Dickenschwindung). n ist der mittlere Brechungsindex des Photopolymers und wurde zu 1.504 gesetzt. λ ist die Wellenlänge des Laserlichts im Vakuum.

Die maximale Beugungseffizienz (DE = $\eta_{max}$) ergibt sich dann für ξ = 0 zu:

$$DE = \tanh^2(\nu) = \tanh^2\left(\frac{\pi \cdot \Delta n \cdot d'}{\lambda \cdot \sqrt{\cos(\alpha') \cdot \cos(\alpha' - 2\psi)}}\right)$$

Figur 3 zeigt die gemessene transmittierte Leistung $P_T$ (rechte *y*-Achse) als durchgezogene Linie gegen das Winkeldetuning ΔΩ aufgetragen, die gemessene Beugungseffizienz η (linke *y*-Achse) als ausgefüllte Kreise gegen das Winkeldetuning ΔΩ aufgetragen (soweit die endliche Größe des Detektors es erlaubte) und die Anpassung der Kogelnik Theorie als gestrichelte Linie (linke *y*-Achse).

Die Messdaten der Beugungseffizienz, die theoretische Braggkurve und die transmittierte Intensität werden wie in Figur 3 gezeigt gegen den zentrierten Drehwinkel $\Delta\Omega \equiv \Omega_{reconstruction} - \Omega = \alpha'_0 - \vartheta'_0$, auch Winkeldetuning genannt, aufgetragen.

Da DE bekannt ist wird die Form der theoretischen Braggkurve nach Kogelnik nur noch durch die Dicke *d'* der Photopolymerschicht bestimmt. Δn wird über DE für gegebene Dicke *d'* so nachkorrigiert, dass Messung und Theorie von DE immer übereinstimmen. *d'* wird nun solange angepasst bis die Winkelpositionen der ersten Nebenminima der theoretischen Braggkurve mit den Winkelpositionen der ersten Nebenmaxima der transmittierten Intensität übereinstimmen und zudem die volle Breite bei halber Höhe (FWHM) für die theoretische Braggkurve und für die transmittierte Intensität übereinstimmen.

Da die Richtung in der ein Reflexionshologramm bei der Rekonstruktion mittels eines Ω-Scans mitrotiert, der Detektor für das abgebeugte Licht aber nur einen endlichen Winkelbereich erfassen kann, wird die Braggkurve von breiten Holgrammen (kleines *d'*) bei einem Ω-Scan nicht vollständig erfasst, sondern nur der zentrale Bereich, bei geeigneter Detektorpositionierung. Daher wird die zur Braggkurve komplementäre Form der transmittierten Intensität zur Anpassung der Schichtdicke *d'* zusätzlich herangezogen.

Figur 3 zeigt die Darstellung der Braggkurve η nach der Coupled Wave Theorie (gestrichelte Linie), des gemessenen Beugungswirkungsgrades (ausgefüllte Kreise) und der transmittierten Leistung (schwarz durchgezogene Linie) gegen das Winkeldetuning ΔΩ.

Für eine Formulierung wurde diese Prozedur eventuell mehrfach für verschiedene Belichtungszeiten *t* an verschiedenen Medien wiederholt, um festzustellen bei welcher mittleren Energiedosis des einfallenden Laserstrahls beim Schreiben des Hologramms DE in den Sättigungswert übergeht. Die mittlere Energiedosis *E* ergibt sich wie folgt aus den Leistungen der zwei den Winkeln ao und ßo zugeordneten Teilstrahlen (Referenzstrahl mit $P_r$ = 0.87 mW und Signalstrahl mit $P_s$ = 1.13 mW), der Belichtungszeit t und dem Durchmesser der Irisblende (0.4 cm):

$$E\,(\text{mJ/cm}^2) = \frac{2 \cdot [P_r + P_s] \cdot t\,(\text{s})}{\pi \cdot 0.4^2\ \text{cm}^2}$$

Die Leistungen der Teilstrahlen wurden so angepasst, dass in dem Medium bei den verwendeten Winkeln $\alpha_0$ und $\beta_0$, die gleiche Leistungsdichte erreicht wird.

**Messergebnisse**

Es wurden vier holographische Filme hergestellt:

erfindungsgemäß:

Film 1, enthaltend Farbstoff 1, 2 und 3 sowie Borat 1

Film 2, enthaltend Farbstoff 2, Borat 1

nicht erfindungsgemäß:

Film A, enthaltend Farbstoff 1, 2 und 3 sowie Borat 2

Film B, enthaltend Farbstoff 2, Borat 2

Um ihre Langzeitstabilität zu testen, wurden die Filme unter Lichtausschluss 12 Monate bei Raumtemperatur gelagert und anschließend ein Hologramm einbelichtet.

Die Ergebnisse der holographischen Belichtung mit grünem Laser (532 nm) sind in der folgenden Tabelle 1 zusammengestellt:

Tabelle 1:

| Film | $\Delta n$ bei 1,99 mJ/cm$^2$ | $\Delta n$ bei 3,02 mJ/cm$^2$ | $\Delta n$ bei 5,97 mJ/cm$^2$ | An bei 7,96 mJ/cm$^2$ | $\Delta n$ bei 15,92 mJ/cm$^2$ | $\Delta n$ bei 31,83 mJ/cm$^2$ |
|---|---|---|---|---|---|---|
| 1 | 0,0053 | 0,0191 | | 0,0311 | 0,0339 | 0,0344 |
| A | 0,0000 | 0,0152 | | 0,0286 | 0,0327 | 0,0338 |
| 2 | | | 0,0284 | 0,0325 | 0,0312 | 0,0306 |
| B | | | 0,0268 | 0,0284 | 0,0278 | 0,0278 |

Man sieht, dass die erfindungsgemäßen Filme 1 und 2 höhere Brechungsindexkontraste $\Delta n$ liefern als die nicht erfindungsgemäßen Filme A und B. Dies wird besonders deutlich, wenn man, wie in der folgenden Tabelle 2 gezeigt, die Quotienten Q der $\Delta n$ Werte der entsprechenden Paare betrachtet, wobei $Q = \Delta n(1) / \Delta n(A)$ bzw. $Q = \Delta n(2) / \Delta n(B)$.

Tabelle 2:

| Filmvergleich | Q bei 1,99 mJ/cm$^2$ | Q bei 3,02 mJ/cm$^2$ | Q bei 5,97 mJ/cm$^2$ | Q bei 7,96 mJ/cm$^2$ | Q bei 15,92 mJ/cm$^2$ | Q bei 31,83 mJ/cm$^2$ |
|---|---|---|---|---|---|---|
| 1 über A | $\infty$ | 1,26 | | 1,09 | 1,04 | 1,02 |
| 2 über B | | | 1,06 | 1,14 | 1,12 | 1,10 |

Man sieht, dass der erfindungsgemäße Film 1 insbesondere bei niedrigen Lichtdosen dem nicht erfindungsgemäßen Film A überlegen ist. Er zeigt also eine höhere Empfindlichkeit. Der erfindungsgemäße Film 2 ist dagegen über einen großen Bereich der Lichtdosen dem nicht erfindungsgemäßen Film B gleichermaßen deutlich überlegen.

Die vier Filme wurden nach Einbelichtung eines Hologramms einem Bleichprozess unterzogen. Es wurde ein zweistufiger Bleichprozess angewandt. Zunächst wurden die Filme für 45 Sekunden der Strahlung einer Quecksilberhochdruckdampflampe vom Typ Dynax 2000-EC ausgesetzt, bei einer Leistungsdichte an der Oberfläche der Folie von 75mW/cm$^2$ und einer Dosis von 7J/cm2. Im unmittelbaren Anschluss erfolgte die flächige Beleuchtung über ein CF2000 UV-LED System der Firma Clearstone Technologies bei einer Wellenlänge von 365nm, bei einer Leistungsdichte an der Oberfläche der Folie von 40mW/cm$^2$ für 300 Sekunden und einer akkumulierten Dosis von 12J/cm$^2$.

Die Ergebnisse sind der Figur 4 zu entnehmen. Diese zeigt die Transmissionsspektren der Filme 1, 2, A und B nach dem Bleichen. Figur 5 enthält die Transmissionsspektren der Filme 1, 2, A und B vor dem Bleichen.

Man sieht, dass die erfindungsgemäßen Filme 1 und 2 nach dem Bleichen eine höhere Transmission liefern als die nicht erfindungsgemäßen Filme A und B. Dieser Unterschied ist insbesondere im Bereich kürzerer Wellenlängen ausgeprägt und führt so bei den erfindungsgemäßen Filmen zu einer geringeren "Restgilbe". Folglich weisen die Filme 1 und 2 eine deutlich verbesserte Bleichbarkeit auf.

## Patentansprüche

1. Photopolymer-Formulierung umfassend eine gegenüber Isocyanaten reaktive Komponente, eine Polyisocyanat-Komponente, ein Schreibmonomer und einen Photoinitiator, enthaltend mindestens einen Farbstoff und einen Coinitiator, **dadurch gekennzeichnet, dass** der Coinitiator wenigstens eine Substanz der Formel (Ia) enthält,

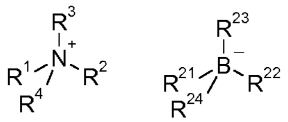

(Ia)

worin

$R^1$ für einen gegebenenfalls verzweigten $C_{14}$- bis $C_{22}$-Alkyl-Rest steht,
$R^2$ für einen gegebenenfalls verzweigten und/oder gegebenenfalls substituierten $C_8$- bis $C_{22}$-Alkyl-Rest, einen Cyclohexyl- oder Cycloheptylrest, einen $C_7$- bis $C_{10}$-Aralkyl-Rest oder einen durch nichtionische Reste substituierten Phenyl-Rest steht und
$R^3$ und $R^4$ unabhängig voneinander für einen gegebenenfalls verzweigten und/oder gegebenenfalls substituierten $C_1$- bis Cs-Alkyl-Rest stehen oder
$R^1$ für einen gegebenenfalls verzweigten $C_{14}$- bis $C_{22}$-Alkyl-Rest steht,
$R^2$ für einen gegebenenfalls verzweigten und/oder gegebenenfalls substituierten $C_8$- bis $C_{22}$-Alkyl-Rest oder einen $C_7$- bis $C_{10}$-Aralkyl-Rest steht und
$R^3$ und $R^4$ gemeinsam eine $-(CH_2)_4-$, $-(CH_2)_5-$ oder $-(CH_2)_2-O-(CH_2)_2-$Brücke bilden oder
$R^1$ für einen gegebenenfalls verzweigten $C_{14}$- bis $C_{22}$-Alkyl-Rest steht,
$R^2$, $R^3$ und $R^4$ gemeinsam mit dem $N^+$ einen Imidazol- oder Pyridin-Ring bilden, der mindestens durch einen Rest der Auswahl $C_1$- bis $C_8$-Alkyl, $C_1$- bis $C_8$-Alkoxy, $C_5$- bis $C_7$-Cycloalkyl, Benzyl oder Phenyl substituiert ist

und worin
$R^{21}$ für einen gegebenenfalls substituierten $C_1$- bis $C_{22}$-Alkyl-, $C_3$- bis $C_{22}$-Alkenyl-, $C_5$- bis $C_7$-Cycloalkyl- oder $C_7$- bis $C_{13}$-Aralkylrest steht und
$R^{22}$ bis $R^{24}$ unabhängig voneinander für einen gegebenenfalls durch mindestens einen Rest der Auswahl Halogen, $C_1$- bis $C_4$-Alkyl, Trifluormethyl, $C_1$-bis $C_4$-Alkoxy, Trifluormethoxy, Phenyl oder Phenoxy substituierten $C_6$- bis $C_{10}$-Arylrest stehen.

2. Photopolymer-Formulierung gemäß Anspruch 1, **dadurch gekennzeichnet, dass** der Coinitiator eine Glasübergangstemperatur $T_g$ von $\leq 0$ °C aufweist.

3. Photopolymer-Formulierung gemäß Anspruch 1 oder 2, **dadurch gekennzeichnet, dass** der Coinitiator weiterhin wenigstens eine Substanz der Formel (Ib) enthält,

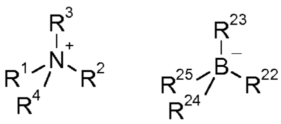

(Ib)

worin $R^1$ bis $R^4$ definiert sind wie in Anspruch 1 und
$R^{22}$ bis $R^{25}$ unabhängig voneinander für einen gegebenenfalls durch mindestens einen Rest der Auswahl Halogen, $C_1$- bis $C_4$-Alkyl, Trifluormethyl, $C_1$- bis $C_4$-Alkoxy, Trifluormethoxy, Phenyl oder Phenoxy substituierten $C_6$- bis $C_{10}$-Arylrest stehen.

**4.** Photopolymer-Formulierung gemäß Anspruch 3, **dadurch gekennzeichnet, dass** der Coinitiator die Substanzen der Formeln (Ia) und (Ib) im molaren Verhältnis von 80:20 bis 99,99:0,01 enthält.

**5.** Photopolymer-Formulierung gemäß einem der Ansprüche 1 bis 4, **dadurch gekennzeichnet, dass** der Coinitiator weiterhin auch wenigstens ein Salz der Formel (II) enthält,

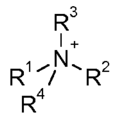 $An^-$

(II)

worin

$An^-$ für ein Anion mit einem AClogP im Bereich von 3-6 steht und
$R^1$ bis $R^4$ die in Anspruch 1 angegebene Bedeutung besitzen.

**6.** Photopolymer-Formulierung gemäß Anspruch 5, **dadurch gekennzeichnet, dass** der Coinitiator 0,01 bis 10 Gew.-%, bevorzugt 0,05 bis 7 Gew.-% und besonders bevorzugt 1 bis 5 Gew.-%, bezogen auf die Gesamtmenge an Coinitiator, an Salzen der Formel (II) enthält.

**7.** Photopolymer-Formulierung nach einem der Ansprüche 1 bis 6, **dadurch gekennzeichnet, dass** der Coinititator weiterhin Substanzen der Formeln (IIIa) und gegebenenfalls (IIIb) enthält,

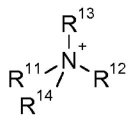 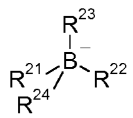

(IIIa)

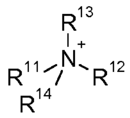 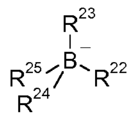

(IIIb),

worin

$R^{11}$ bis $R^{14}$ unabhängig voneinander für $C_1$- bis $C_4$-Alkyl stehen und
$R^{21}$ bis $R^{24}$ die in Anspruch 1 genannte Bedeutung und $R^{25}$ die in Anspruch 3 genannte Bedeutung besitzen.

**8.** Photopolymer-Formulierung nach Anspruch 7, **dadurch gekennzeichnet, dass** der Coinitiator die Substanzen (IIIa) und (IIIb) im gleichen Verhältnis zueinander enthält wie die Substanzen (Ia) und (Ib).

**9.** Photopolymer-Formulierung nach Anspruch 7 oder 8, **dadurch gekennzeichnet, dass** das molare Verhältnis der Substanzen (IIIa) und gegebenenfalls (IIIb) zu der Summe der Substanzen (Ia) und gegebenenfalls (Ib) $\leq$ 15:85 ist.

**10.** Holographisches Medium hergestellt unter Verwendung einer Photopolymer-Formulierung nach einem der Ansprüche 1 bis 9.

**11.** Verfahren zur Herstellung eines holographischen Mediums, bei dem

(I) eine Photopolymer-Formulierung gemäß einem der Ansprüche 1 bis 9 durch Vermischen aller Bestandteile hergestellt,
(II) die Photopolymer-Formulierung bei einer Verarbeitungstemperatur in die für das holographische Medium gewünschte Form gebracht und
(III) in der gewünschten Form unter Urethanbildung bei einer Vernetzungstemperatur oberhalb der Verarbeitungstemperatur ausgehärtet wird.

**12.** Schichtaufbau, umfassend ein Trägersubstrat, darauf aufgebracht ein holographisches Medium nach Anspruch 10 und gegebenenfalls eine auf der dem Trägersubstrat abgewandten Seite des holographischen Mediums aufgebrachte Abdeckschicht.

**13.** Verfahren zur Herstellung von Coinitiatoren umfassend eine Mischung von Substanzen der Formel (Ia) und Salzen der Formel (II), die gegebenenfalls auch Substanzen der Formel (Ib) und/oder der Formel (IIIa) und gegebenenfalls der Formel (IIIb) enthalten kann,

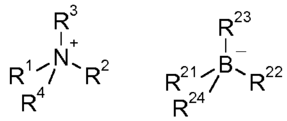
(Ia)

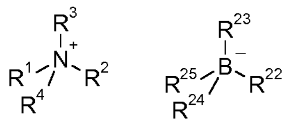
(Ib)

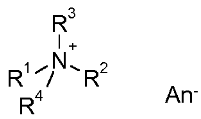
(II)

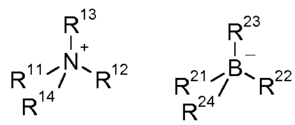
(IIIa)

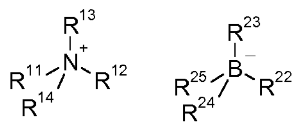
(IIIb),

worin

$R^1$ bis $R^4$ die in Anspruch 1 genannte Bedeutung besitzen,
$R^{11}$ bis $R^{14}$ die in Anspruch 7 genannte Bedeutung besitzen,
$R^{21}$ bis $R^{24}$ die in Anspruch 1 genannte Bedeutung besitzen,
$R^{25}$ die in Anspruch 3 genannte Bedeutung besitzt und
$An^-$ die in Anspruch 5 genannte Bedeutung besitzt,

**dadurch gekennzeichnet, dass** Borate der Formel (IIIa) oder eine Mischung aus Boraten der Formeln (IIIa) und (IIIb) mit einem Ammoniumsalz der Formel (VI)

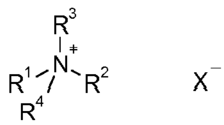
(VI),

worin

$R^1$ bis $R^4$ die in Anspruch 1 genannte Bedeutung besitzen und

$X^-$ für ein Anion, bevorzugt für ein Halogenid-Ion steht,

in Gegenwart eines Salzes der Formel (VII)

$$M^+ An^- \qquad (VII),$$

worin

$M^+$ für ein Kation, bevorzugt für ein Alkali-Ion oder ein Ammonium-Ion steht und
$An^-$ die in Anspruch 5 genannte Bedeutung besitzt,

in einem Zweiphasengemisch aus Wasser und einem Ester umgesetzt werden.

**14.** Verfahren zur Herstellung von Coinitiatoren gemäß Anspruch 13, **dadurch gekennzeichnet, dass** man die Salze der Formel (VII) relativ zu den Substanzen der Formel (Ia) oder der Summe der Substanzen der Formeln (Ia) und (Ib) im molaren Verhältnis 0,5 bis 10 zu 100, bevorzugt 1 bis 5 zu 100 einsetzt.

**15.** Verfahren zur Herstellung von Coinitiatoren gemäß einem der Ansprüche 13 oder 14, **dadurch gekennzeichnet, dass** man die Salze der Formel (II) relativ zur Summe aus den Substanzen der Formeln (Ia), (Ib) und dem Salz der Formel (VII) im molaren Verhältnis 100 bis 110 zu 100, bevorzugt 100 bis 105 zu 100, besonders bevorzugt 100 bis 102 zu 100 einsetzt.

**16.** Coinitiatoren herstellbar nach einem Verfahren gemäß einem der Ansprüche 13 bis 15.

**17.** Borate der Formel (VIII)

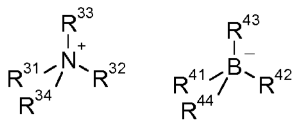

(VIII),

worin

$R^{31}$ für einen gegebenenfalls verzweigten $C_{14}$- bis $C_{22}$-Alkyl-Rest steht,
$R^{32}$ für einen $C_7$- bis $C_{10}$-Aralkyl-Rest steht,
$R^{33}$ und $R^{34}$ unabhängig voneinander für einen gegebenenfalls verzweigten und/oder gegebenenfalls substituierten $C_1$- bis Cs-Alkyl-Rest stehen,
$R^{41}$ für einen $C_2$- bis $C_{22}$-Alkyl-, $C_3$- bis $C_{22}$-Alkenyl-, $C_5$- bis $C_7$-Cycloalkyl- oder $C_7$- bis $C_{13}$-Aralkylrest steht und
$R^{42}$ bis $R^{44}$ unabhängig voneinander für einen durch mindestens einen Rest der Auswahl Halogen, $C_1$- bis $C_4$-Alkyl, Trifluormethyl, $C_1$- bis $C_4$-Alkoxy, Trifluormethoxy, Phenyl oder Phenoxy substituierten $C_6$- bis $C_{10}$-Arylrest stehen

oder worin

$R^{31}$ für einen gegebenenfalls verzweigten $C_{14}$- bis $C_{22}$-Alkyl-Rest steht,
$R^{32}$ für einen gegebenenfalls verzweigten $C_8$- bis $C_{22}$-Alkyl-Rest oder einen $C_7$- bis $C_{10}$-Aralkyl-Rest steht,
$R^{33}$ und $R^{34}$ gemeinsam eine $-(CH_2)_4-$, $-(CH_2)_5-$ oder $-(CH_2)_2-O-(CH_2)_2-$Brücke bilden,
$R^{41}$ für einen $C_2$- bis $C_{22}$-Alkyl-, $C_3$- bis $C_{22}$-Alkenyl-, $C_5$- bis $C_7$-Cycloalkyl- oder $C_7$- bis $C_{13}$-Aralkylrest steht und
$R^{42}$ bis $R^{44}$ unabhängig voneinander für einen durch mindestens einen Rest der Auswahl Halogen, $C_1$- bis $C_4$-Alkyl, Trifluormethyl, $C_1$- bis $C_4$-Alkoxy, Trifluormethoxy, Phenyl oder Phenoxy substituierten $C_6$- bis $C_{10}$-Arylrest stehen

oder worin

$R^{31}$ für einen gegebenenfalls verzweigten $C_{14}$- bis $C_{22}$-Alkyl-Rest steht,
$R^{32}$ für einen durch ein bis drei Reste aus der Gruppe gegebenenfalls verzweigter $C_3$- bis $C_8$-Alkylrest, gegebenenfalls verzweigter $C_3$- bis Cs-Alkoxyrest, Trifluormethyl, Trifluormethoxy, Cyclopentyl, Cyclohexyl, Cycloheptyl, Phenyl oder Phenoxy substituierten Phenyl-Rest steht,
$R^{33}$ und $R^{34}$ unabhängig voneinander für einen gegebenenfalls verzweigten und/oder gegebenenfalls substituierten $C_1$- bis Cs-Alkyl-Rest stehen,
$R^{41}$ für einen $C_2$- bis $C_{22}$-Alkyl-, $C_3$- bis $C_{22}$-Alkenyl-, $C_5$- bis $C_7$-Cycloalkyl- oder $C_7$- bis $C_{13}$-Aralkylrest steht und
$R^{42}$ bis $R^{44}$ unabhängig voneinander für einen durch mindestens einen Rest der Auswahl Halogen, $C_1$- bis $C_4$-Alkyl, Trifluormethyl, $C_1$- bis $C_4$-Alkoxy, Trifluormethoxy, Phenyl oder Phenoxy substituierten $C_6$- bis $C_{10}$-Arylrest stehen

oder worin

$R^{31}$ für einen gegebenenfalls verzweigten $C_{14}$- bis $C_{22}$-Alkyl-Rest steht und
$R^{32}$, $R^{33}$ und $R^{34}$ gemeinsam mit dem $N^+$-Atom einen Imidazol- oder Pyridin-Ring bilden, der mindestens durch einen Rest der Auswahl $C_1$- bis $C_8$-Alkyl, $C_1$- bis $C_8$-Alkoxy, $C_5$- bis $C_7$-Cycloalkyl, Benzyl oder Phenyl substituiert ist,
$R^{41}$ für einen $C_2$- bis $C_{22}$-Alkyl-, $C_3$- bis $C_{22}$-Alkenyl-, $C_5$- bis $C_7$-Cycloalkyl- oder $C_7$- bis $C_{13}$-Aralkylrest steht und
$R^{42}$ bis $R^{44}$ unabhängig voneinander für einen durch mindestens einen Rest der Auswahl Halogen, $C_1$- bis $C_4$-Alkyl, Trifluormethyl, $C_1$- bis $C_4$-Alkoxy, Trifluormethoxy, Phenyl oder Phenoxy substituierten $C_6$- bis $C_{10}$-Arylrest stehen.

**18.** Borate gemäß Anspruch 17, **dadurch gekennzeichnet, dass** $R^{42}$ bis $R^{44}$ für einen 4-Halogenphenylrest oder einen 4-Alkyl-3-halogenarylrest stehen.

**19.** Borate gemäß Anspruch 17 oder 18, **dadurch gekennzeichnet, dass** $R^{32}$, $R^{33}$ und $R^{34}$ gemeinsam mit dem $N^+$-Atom einen Imidazol- oder Pyridin-Ring bilden, der einer der Formeln

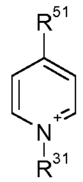 (IX), 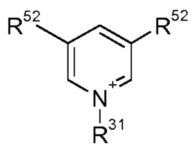 (X) oder 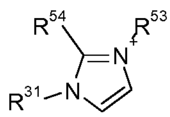 (XI)

entspricht, worin

$R^{31}$ für einen gegebenenfalls verzweigten $C_{14}$- bis $C_{22}$-Alkyl-Rest steht und
$R^{51}$ und $R^{53}$ unabhängig voneinander für einen gegebenenfalls verzweigten $C_3$- bis $C_8$-Alkylrest, Cyclopentyl, Cyclohexyl, Cycloheptyl oder Phenyl stehen und $R^{51}$ zusätzlich für einen gegebenenfalls verzweigten $C_3$- bis $C_8$-Alkoxyrest oder Phenoxy und $R^{53}$ zusätzlich für Phenyl oder Benzyl stehen,
$R^{52}$ für einen $C_1$- bis $C_4$-Alkylrest steht,
$R^{54}$ für Wasserstoff, einen $C_1$- bis $C_4$-Alkylrest oder Phenyl steht.

## Claims

**1.** Photopolymer formulation comprising a component reactive toward isocyanates, a polyisocyanate component, a writing monomer and a photoinitiator containing at least one dye and a coinitiator, **characterized in that** the coinitiator contains at least one substance of the formula (Ia)

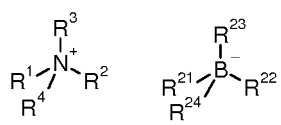 (Ia)

in which

$R^1$ is an optionally branched $C_{14}$- to $C_{22}$-alkyl radical,

$R^2$ is an optionally branched and/or optionally substituted $C_8$- to $C_{22}$-alkyl radical, a cyclohexyl or cycloheptyl radical, a $C_7$- to $C_{10}$-aralkyl radical, or a phenyl radical substituted by nonionic radicals, and
$R^3$ and $R^4$ are each independently an optionally branched and/or optionally substituted $C_1$- to $C_5$-alkyl radical or

$R^1$ is an optionally branched $C_{14}$- to $C_{22}$-alkyl radical,

$R^2$ is an optionally branched and/or optionally substituted $C_8$- to $C_{22}$-alkyl radical or a $C_7$-to $C_{10}$-aralkyl radical and
$R^3$ and $R^4$ together form a $-(CH_2)_4-$, $-(CH_2)_5-$ or $-(CH_2)_2-O-(CH_2)_2-$ bridge or
$R^1$ is an optionally branched $C_{14}$- to $C_{22}$-alkyl radical,
$R^2$, $R^3$ and $R^4$ together with the $N^+$ form an imidazole or pyridine ring substituted at least by one radical selected from $C_1$- to $C_8$-alkyl, $C_1$- to $C_8$-alkoxy, $C_5$- to $C_7$-cycloalkyl, benzyl or phenyl

and in which

$R^{21}$ is an optionally substituted $C_1$- to $C_{22}$-alkyl, $C_3$- to $C_{22}$-alkenyl, $C_5$- to $C_7$-cycloalkyl or $C_7$- to $C_{13}$-aralkyl radical and
$R^{22}$ to $R^{24}$ are each independently a $C_6$- to $C_{10}$-aryl radical optionally substituted by at least one radical selected from halogen, $C_1$- to $C_4$-alkyl, trifluoromethyl, $C_1$- to $C_4$-alkoxy, trifluoromethoxy, phenyl and phenoxy.

2. Photopolymer formulation according to Claim 1, **characterized in that** the coinitiator has a glass transition temperature $T_g$ of $\leq 0°C$.

3. Photopolymer formulation according to Claim 1 or 2, **characterized in that** the coinitiator additionally contains at least one substance of the formula (Ib)

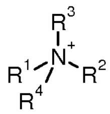 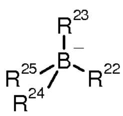 (Ib)

in which $R^1$ to $R^4$ are each as defined in Claim 1 and
$R^{22}$ to $R^{25}$ are each independently a $C_6$- to $C_{10}$-aryl radical optionally substituted by at least one radical selected from halogen, $C_1$- to $C_4$-alkyl, trifluoromethyl, $C_1$- to $C_4$-alkoxy, trifluoromethoxy, phenyl and phenoxy.

4. Photopolymer formulation according to Claim 3, **characterized in that** the coinitiator contains the substances of the formulae (Ia) and (Ib) in a molar ratio of 80:20 to 99.99:0.01.

5. Photopolymer formulation according to any of Claims 1 to 4, **characterized in that** the coinitiator additionally also contains at least one salt of the formula (II)

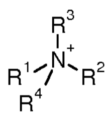 $An^-$ (II)

in which

$An^-$ is an anion having an AClogP in the range of 3-6 and
$R^1$ to $R^4$ are each as defined in Claim 1.

6. Photopolymer formulation according to Claim 5, **characterized in that** the coinitiator contains 0.01 to 10% by weight, preferably 0.05 to 7% by weight and more preferably 1 to 5% by weight, based on the total amount of coinitiator, of salts of the formula (II).

7. Photopolymer formulation according to any of Claims 1 to 6, **characterized in that** the coinitiator additionally contains substances of the formulae (IIIa) and optionally (IIIb)

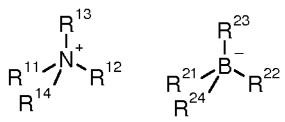

(IIIa)

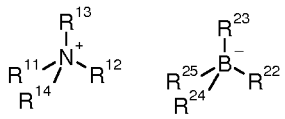

(IIIb)

in which

$R^{11}$ to $R^{14}$ are each independently $C_1$- to $C_4$-alkyl and
$R^{21}$ to $R^{24}$ are each as defined in Claim 1 and $R^{25}$ is as defined in Claim 3.

**8.** Photopolymer formulation according to Claim 7, **characterized in that** the coinitiator contains the substances (IIIa) and (IIIb) in the same ratio relative to one another as the substances (Ia) and (Ib) .

**9.** Photopolymer formulation according to Claim 7 or 8, **characterized in that** the molar ratio of the substances (IIIa) and optionally (IIIb) to the sum total of the substances (Ia) and optionally (Ib) is $\leq$ 15:85.

**10.** Holographic medium produced using a photopolymer formulation according to any of Claims 1 to 9.

**11.** Process for producing a holographic medium, in which

(I) a photopolymer formulation according to any of Claims 1 to 9 is produced by mixing all the constituents,
(II) the photopolymer formulation is converted to the form desired for the holographic medium at a processing temperature and
(III) cured in the desired form with urethane formation at a crosslinking temperature above the processing temperature.

**12.** Laminate structure comprising a carrier substrate, a holographic medium according to Claim 10 applied thereto, and optionally a covering layer applied to the opposite side of the holographic medium from the carrier substrate.

**13.** Process for preparing coinitiators comprising a mixture of substances of the formula (Ia) and salts of the formula (II), and optionally also containing substances of the formula (Ib) and/or of the formula (IIIa) and optionally of the formula (IIIb),

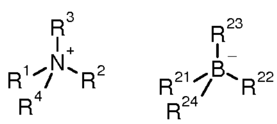

(Ia)

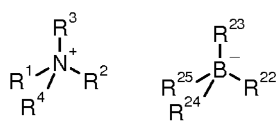

(Ib)

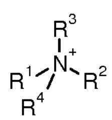

$An^-$

(II)

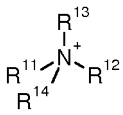 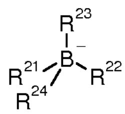

(IIIa)

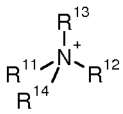 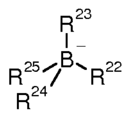

(IIIb)

in which

$R^1$ to $R^4$ are each as defined in Claim 1,
$R^{11}$ to $R^{14}$ are each as defined in Claim 7,
$R^{21}$ to $R^{24}$ are each as defined in Claim 1,
$R^{25}$ is as defined in Claim 3 and
$An^-$ is as defined in Claim 5,

**characterized in that** borates of the formula (IIIa) or a mixture of borates of the formulae (IIIa) and (IIIb) are reacted with an ammonium salt of the formula (VI)

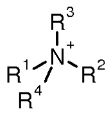 $X^-$

(VI)

in which

$R^1$ to $R^4$ are each as defined in Claim 1 and
$X^-$ is an anion, preferably a halide ion,

in the presence of a salt of the formula (VII)

$M^+$ An- (VII)

in which

$M^+$ is a cation, preferably an alkali metal ion or an ammonium ion, and
$An^-$ is as defined in Claim 5,
in a biphasic mixture of water and an ester.

**14.** Process for preparing coinitiators according to Claim 13, **characterized in that** the salts of the formula (VII) are used, relative to the substances of the formula (Ia) or the sum total of the substances of the formulae (Ia) and (Ib), in a molar ratio of 0.5 to 10:100, preferably 1 to 5:100.

**15.** Process for preparing coinitiators according to either of Claims 13 and 14, **characterized in that** the salts of the formula (II) are used, relative to the sum total of the substances of the formulae (Ia), (Ib) and the salt of the formula (VII), in a molar ratio of 100 to 110:100, preferably 100 to 105:100, more preferably 100 to 102:100.

**16.** Coinitiators preparable by a process according to any of Claims 13 to 15.

**17.** Borates of the formula (VIII)

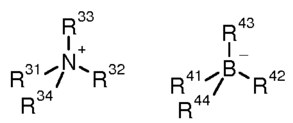

(VIII)

in which

$R^{31}$ is an optionally branched $C_{14}$- to $C_{22}$-alkyl radical,
$R^{32}$ is a $C_7$- to $C_{10}$-aralkyl radical,
$R^{33}$ and $R^{34}$ are each independently an optionally branched and/or optionally substituted $C_1$- to $C_5$-alkyl radical,
$R^{41}$ is a $C_2$- to $C_{22}$-alkyl, $C_3$- to $C_{22}$-alkenyl, $C_5$- to $C_7$-cycloalkyl or $C_7$- to $C_{13}$-aralkyl radical and
$R^{42}$ to $R^{44}$ are each independently a $C_6$- to $C_{10}$-aryl radical substituted by at least one radical selected from halogen, $C_1$- to $C_4$-alkyl, trifluoromethyl, $C_1$- to $C_4$-alkoxy, trifluoromethoxy, phenyl and phenoxy,

or in which

$R^{31}$ is an optionally branched $C_{14}$- to $C_{22}$-alkyl radical,
$R^{32}$ is an optionally branched $C_8$- to $C_{22}$-alkyl radical or a $C_7$- to $C_{10}$-aralkyl radical,
$R^{33}$ and $R^{34}$ together form a $-(CH_2)_4-$, $-(CH_2)_5-$ or $-(CH_2)_2-O-(CH_2)_2-$ bridge,
$R^{41}$ is a $C_2$- to $C_{22}$-alkyl, $C_3$- to $C_{22}$-alkenyl, $C_5$- to $C_7$-cycloalkyl or $C_7$- to $C_{13}$-aralkyl radical and
$R^{42}$ to $R^{44}$ are each independently a $C_6$- to $C_{10}$-aryl radical substituted by at least one radical selected from halogen, $C_1$- to $C_4$-alkyl, trifluoromethyl, $C_1$- to $C_4$-alkoxy, trifluoromethoxy, phenyl and phenoxy,

or in which

$R^{31}$ is an optionally branched $C_{14}$- to $C_{22}$-alkyl radical,
$R^{32}$ is a phenyl radical substituted by one to three radicals from the group of optionally branched $C_3$-to $C_8$-alkyl radical, optionally branched $C_3$- to $C_8$-alkoxy radical, trifluoromethyl, trifluoromethoxy, cyclopentyl, cyclohexyl, cycloheptyl, phenyl and phenoxy,
$R^{33}$ and $R^{34}$ are each independently an optionally branched and/or optionally substituted $C_1$- to $C_5$-alkyl radical,
$R^{41}$ is a $C_2$- to $C_{22}$-alkyl, $C_3$- to $C_{22}$-alkenyl, $C_5$- to $C_7$-cycloalkyl or $C_7$- to $C_{13}$-aralkyl radical and
$R^{42}$ to $R^{44}$ are each independently a $C_6$- to $C_{10}$-aryl radical substituted by at least one radical selected from halogen, $C_1$- to $C_4$-alkyl, trifluoromethyl, $C_1$- to $C_4$-alkoxy, trifluoromethoxy, phenyl and phenoxy,

or in which

$R^{31}$ is an optionally branched $C_{14}$- to $C_{22}$-alkyl radical and
$R^{32}$, $R^{33}$ and $R^{34}$ together with the $N^+$ atom form an imidazole or pyridine ring substituted at least by one radical selected from $C_1$- to $C_8$-alkyl, $C_1$- to $C_8$-alkoxy, $C_5$- to $C_7$-cycloalkyl, benzyl or phenyl,
$R^{41}$ is a $C_2$- to $C_{22}$-alkyl, $C_3$- to $C_{22}$-alkenyl, $C_5$- to $C_7$-cycloalkyl or $C_7$- to $C_{13}$-aralkyl radical and
$R^{42}$ to $R^{44}$ are each independently a $C_6$- to $C_{10}$-aryl radical substituted by at least one radical selected from halogen, $C_1$- to $C_4$-alkyl, trifluoromethyl, $C_1$- to $C_4$-alkoxy, trifluoromethoxy, phenyl and phenoxy.

**18.** Borates according to Claim 17, **characterized in that** $R^{42}$ to $R^{44}$ are each a 4-halophenyl radical or a 4-alkyl-3-haloaryl radical.

**19.** Borates according to Claim 17 or 18, **characterized in that** $R^{32}$, $R^{33}$ and $R^{34}$ together with the $N^+$ atom form an imidazole or pyridine ring corresponding to one of the formulae

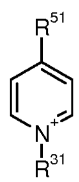

(IX),

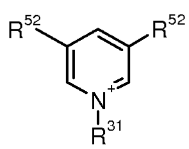

(X)

or

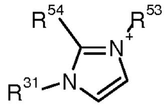

(XI)

in which

$R^{31}$ is an optionally branched $C_{14}$- to $C_{22}$-alkyl radical and
$R^{51}$ and $R^{53}$ are each independently an optionally branched $C_3$- to $C_8$-alkyl radical, cyclopentyl, cyclohexyl, cycloheptyl or phenyl, and $R^{51}$ is additionally an optionally branched $C_3$- to $C_8$-alkoxy radical or phenoxy, and $R^{53}$ is additionally phenyl or benzyl,
$R^{52}$ is a $C_1$- to $C_4$-alkyl radical,
$R^{54}$ is hydrogen, a $C_1$- to $C_4$-alkyl radical or phenyl.

**Revendications**

1. Formulation de photopolymère comprenant un composant réactif avec les isocyanates, un composant polyisocyanate, un monomère d'écriture et un photoinitiateur, contenant au moins un colorant et un co-initiateur, **caractérisée en ce que** le co-initiateur contient au moins une substance de formule (Ia)

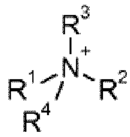

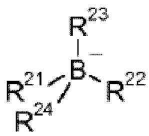

(Ia)

dans laquelle

$R^1$ représente un radical alkyle en $C_{14}$ à $C_{22}$ éventuellement ramifié,
$R^2$ représente un radical alkyle en $C_8$ à $C_{22}$ éventuellement ramifié et/ou éventuellement substitué, un radical cyclohexyle ou cycloheptyle, un radical aralkyle en $C_7$ à $C_{10}$ ou un radical phényle substitué par des radicaux non ioniques, et
$R^3$ et $R^4$ représentent indépendamment l'un de l'autre un radical alkyle en $C_1$ à $C_5$ éventuellement ramifié et/ou éventuellement substitué, ou
$R^1$ représente un radical alkyle en $C_{14}$ à $C_{22}$ éventuellement ramifié,
$R^2$ représente un radical alkyle en $C_8$ à $C_{22}$ éventuellement ramifié et/ou éventuellement substitué ou un radical aralkyle en $C_7$ à $C_{10}$, et
$R^3$ et $R^4$ forment ensemble un pont $-(CH_2)_4-$, $-(CH_2)_5$ou - $(CH_2)_2$-O-$(CH_2)_2$-, ou
$R^1$ représente un radical alkyle en $C_{14}$ à $C_{22}$ éventuellement ramifié,
$R^2$, $R^3$ et $R^4$ forment ensemble avec le $N^+$ un cycle imidazole ou pyridine, qui est substitué au moins par un radical choisi parmi alkyle en $C_1$ à $C_8$, alcoxy en $C_1$ à $C_8$, cycloalkyle en $C_5$ à $C_7$, benzyle ou phényle,

et dans laquelle

$R^{21}$ représente un radical alkyle en $C_1$ à $C_{22}$, alcényle en $C_3$ à $C_{22}$, cycloalkyle en $C_5$ à $C_7$ ou aralkyle en $C_7$ à $C_{13}$ éventuellement substitué, et
$R^{22}$ à $R^{24}$ représentent indépendamment les uns des autres un radical aryle en $C_6$ à $C_{10}$ éventuellement substitué par au moins un radical choisi parmi halogène, alkyle en $C_1$ à $C_4$, trifluorométhyle, alcoxy en $C_1$ à $C_4$, trifluorométhoxy, phényle ou phénoxy.

2. Formulation de photopolymère selon la revendication 1, **caractérisée en ce que** le co-initiateur présente une température de transition vitreuse $T_g \leq 0$ °C.

3. Formulation de photopolymère selon la revendication 1 ou 2, **caractérisée en ce que** le co-initiateur contient en outre au moins une substance de formule (Ib)

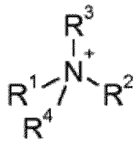 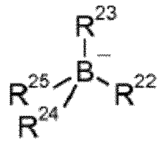

(Ib)

dans laquelle $R^1$ à $R^4$ sont tels que définis dans la revendication 1, et
$R^{22}$ à $R^{25}$ représentent indépendamment les uns des autres un radical aryle en $C_6$ à $C_{10}$ éventuellement substitué par au moins un radical choisi parmi halogène, alkyle en $C_1$ à $C_4$, trifluorométhyle, alcoxy en $C_1$ à $C_4$, trifluorométhoxy, phényle ou phénoxy.

4. Formulation de photopolymère selon la revendication 3, **caractérisée en ce que** le co-initiateur contient les substances de formule (Ia) et (Ib) en un rapport molaire de 80:20 à 99,99:0,01.

5. Formulation de photopolymère selon l'une quelconque des revendications 1 à 4, **caractérisée en ce que** le co-initiateur contient en outre au moins un sel de formule (II)

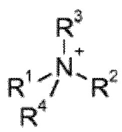 $An^-$

(II)

dans laquelle

$An^-$ représente un anion ayant un AClogP dans la plage allant de 3 à 6 et
$R^1$ à $R^4$ ont la signification indiquée dans la revendication 1.

6. Formulation de photopolymère selon la revendication 5, **caractérisée en ce que** le co-initiateur contient 0,01 à 10 % en poids, de préférence 0,05 à 7 % en poids et de manière particulièrement préférée 1 à 5 % en poids, par rapport à la quantité totale de co-initiateur, de sels de formule (II).

7. Formulation de photopolymère selon l'une quelconque des revendications 1 à 6, **caractérisée en ce que** le co-initiateur contient en outre des substances de formule (IIIa) et éventuellement (IIIb)

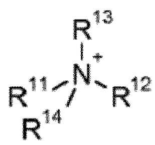 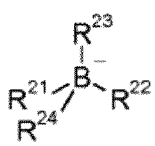

(IIIa)

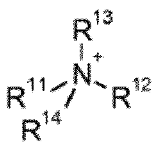 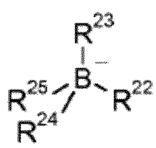

(IIIb),

dans lesquelles

$R^{11}$ à $R^{14}$ représentent indépendamment les uns des autres alkyle en $C_1$ à $C_4$, et
$R^{21}$ à $R^{24}$ ont la signification indiquée dans la revendication 1 et $R^{25}$ a la signification indiquée dans la revendication 3.

**8.** Formulation de photopolymère selon la revendication 7, **caractérisée en ce que** le co-initiateur contient les substances (IIIa) et (IIIb) en le même rapport l'une par rapport à l'autre que les substances (Ia) et (Ib).

**9.** Formulation de photopolymère selon la revendication 7 ou 8, **caractérisée en ce que** le rapport molaire entre les substances (IIIa) et éventuellement (IIIb) et la somme des substances (Ia) et éventuellement (Ib) est ≤ 15:85.

**10.** Support holographique fabriqué en utilisant une formulation de photopolymère selon l'une quelconque des revendications 1 à 9.

**11.** Procédé de fabrication d'un support holographique, selon lequel

(I) une formulation de photopolymère selon l'une quelconque des revendications 1 à 9 est fabriquée par mélange de tous les constituants,
(II) la formulation de photopolymère est mise sous la forme souhaitée pour le support holographique à une température d'usinage et
(III) durcie sous la forme souhaitée avec formation d'uréthane à une température de réticulation supérieure à la température d'usinage.

**12.** Structure en couches, comprenant un substrat support, un support holographique selon la revendication 10 appliqué sur celui-ci et éventuellement une couche de recouvrement appliquée sur le côté opposé au substrat support du support holographique.

**13.** Procédé de fabrication de co-initiateurs comprenant un mélange de substances de formule (Ia) et de sels de formule (II), qui peut éventuellement également contenir des substances de formule (Ib) et/ou de formule (IIIa) et éventuellement de formule (IIIb)

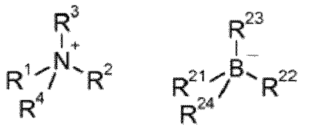

(Ia)

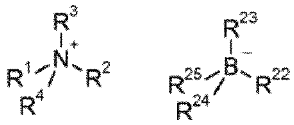

(Ib)

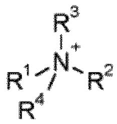

$An^-$

(II)

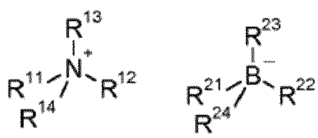

(IIIa)

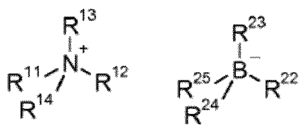

(IIIb),

dans lesquelles

$R^1$ à $R^4$ ont la signification indiquée dans la revendication 1,
$R^{11}$ à $R^{14}$ ont la signification indiquée dans la revendication 7,
$R^{21}$ à $R^{24}$ ont la signification indiquée dans la revendication 1,
$R^{25}$ a la signification indiquée dans la revendication 3, et
$An^-$ a la signification indiquée dans la revendication 5, **caractérisé en ce que** des borates de formule (IIIa) ou un mélange de borates de formule (IIIa) et (IIIb) sont mis en réaction avec un sel d'ammonium de formule (VI)

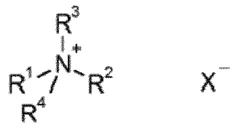

(VI),

dans laquelle

$R^1$ à $R^4$ ont la signification indiquée dans la revendication 1 et
$X^-$ représente un anion, de préférence un ion halogénure,
en présence d'un sel de formule (VII)

$M^+ An^-$ (VII)

dans laquelle

$M^+$ représente un cation, de préférence un ion alcalin ou un ion ammonium, et
$An^-$ a la signification indiquée dans la revendication 5,
dans un mélange biphasé d'eau et d'un ester.

**14.** Procédé de fabrication de co-initiateurs selon la revendication 13, **caractérisé en ce que** les sels de formule (VII) sont utilisés par rapport aux substances de formule (Ia) ou à la somme des substances de formule (Ia) et (Ib) en un rapport molaire de 0,5 à 10 sur 100, de préférence de 1 à 5 sur 100.

**15.** Procédé de fabrication de co-initiateurs selon l'une quelconque des revendications 13 ou 14, **caractérisé en ce que** les sels de formule (II) sont utilisés par rapport à la somme des substances de formule (Ia), (Ib) et du sel de formule (VII) en un rapport molaire de 100 à 110 sur 100, de préférence de 100 à 105 sur 100, de manière particulièrement préférée de 100 à 102 sur 100.

**16.** Co-initiateurs pouvant être fabriqués par un procédé selon l'une quelconque des revendications 13 à 15.

**17.** Borates de formule (VIII)

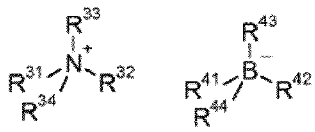

(VIII),

dans laquelle

$R^{31}$ représente un radical alkyle en $C_{14}$ à $C_{22}$ éventuellement ramifié,
$R^{32}$ représente un radical aralkyle en $C_7$ à $C_{10}$,
$R^{33}$ et $R^{34}$ représentent indépendamment l'un de l'autre un radical alkyle en $C_1$ à $C_5$ éventuellement ramifié et/ou éventuellement substitué,
$R^{41}$ représente un radical alkyle en $C_2$ à $C_{22}$, alcényle en $C_3$ à $C_{22}$, cycloalkyle en $C_5$ à $C_7$ ou aralkyle en $C_7$ à $C_{13}$, et
$R^{42}$ à $R^{44}$ représentent indépendamment les uns des autres un radical aryle en $C_6$ à $C_{10}$ substitué par au moins un radical choisi parmi halogène, alkyle en $C_1$ à $C_4$, trifluorométhyle, alcoxy en $C_1$ à $C_4$, trifluorométhoxy, phényle ou phénoxy,

ou dans laquelle

$R^{31}$ représente un radical alkyle en $C_{14}$ à $C_{22}$ éventuellement ramifié,
$R^{32}$ représente un radical alkyle en $C_8$ à $C_{22}$ éventuellement ramifié ou un radical aralkyle en $C_7$ à $C_{10}$,
$R^{33}$ et $R^{34}$ forment ensemble un pont $-(CH_2)_4-$, $-(CH_2)_5-$ ou $-(CH_2)_2-O-(CH_2)_2-$,
$R^{41}$ représente un radical alkyle en $C_2$ à $C_{22}$, alcényle en $C_3$ à $C_{22}$, cycloalkyle en $C_5$ à $C_7$ ou aralkyle en $C_7$ à $C_{13}$, et
$R^{42}$ à $R^{44}$ représentent indépendamment les uns des autres un radical aryle en $C_6$ à $C_{10}$ substitué par au moins un radical choisi parmi halogène, alkyle en $C_1$ à $C_4$, trifluorométhyle, alcoxy en $C_1$ à $C_4$, trifluorométhoxy, phényle ou phénoxy,

ou dans laquelle

$R^{31}$ représente un radical alkyle en $C_{14}$ à $C_{22}$ éventuellement ramifié,
$R^{32}$ représente un radical phényle substitué par un à trois radicaux du groupe constitué par un radical alkyle en $C_3$ à $C_8$ éventuellement ramifié, un radical alcoxy en $C_3$ à $C_8$ éventuellement ramifié, trifluorométhyle, trifluorométhoxy, cyclopentyle, cyclohexyle, cycloheptyle, phényle ou phénoxy,
$R^{33}$ et $R^{34}$ représentent indépendamment l'un de l'autre un radical alkyle en $C_1$ à $C_5$ éventuellement ramifié et/ou éventuellement substitué,
$R^{41}$ représente un radical alkyle en $C_2$ à $C_{22}$, alcényle en $C_3$ à $C_{22}$, cycloalkyle en $C_5$ à $C_7$ ou aralkyle en $C_7$ à $C_{13}$, et
$R^{42}$ à $R^{44}$ représentent indépendamment les uns des autres un radical aryle en $C_6$ à $C_{10}$ substitué par au moins un radical choisi parmi halogène, alkyle en $C_1$ à $C_4$, trifluorométhyle, alcoxy en $C_1$ à $C_4$, trifluorométhoxy, phényle ou phénoxy,

ou dans laquelle

$R^{31}$ représente un radical alkyle en $C_{14}$ à $C_{22}$ éventuellement ramifié, et
$R^{32}$, $R^{33}$ et $R^{34}$ forment ensemble avec l'atome $N^+$ un cycle imidazole ou pyridine, qui est substitué au moins par un radical choisi parmi alkyle en $C_1$ à $C_8$, alcoxy en $C_1$ à $C_8$, cycloalkyle en $C_5$ à $C_7$, benzyle ou phényle,
$R^{41}$ représente un radical alkyle en $C_2$ à $C_{22}$, alcényle en $C_3$ à $C_{22}$, cycloalkyle en $C_5$ à $C_7$ ou aralkyle en $C_7$ à $C_{13}$, et
$R^{42}$ à $R^{44}$ représentent indépendamment les uns des autres un radical aryle en $C_6$ à $C_{10}$ substitué par au moins un radical choisi parmi halogène, alkyle en $C_1$ à $C_4$, trifluorométhyle, alcoxy en $C_1$ à $C_4$, trifluorométhoxy, phényle ou phénoxy.

18. Borates selon la revendication 17, **caractérisés en ce que** $R^{42}$ à $R^{44}$ représentent un radical 4-halogénophényle ou un radical 4-alkyl-3-halogénoaryle.

19. Borates selon la revendication 17 ou 18, **caractérisés en ce que** $R^{32}$, $R^{33}$ et $R^{34}$ forment ensemble avec l'atome $N^+$ un cycle imidazole ou pyridine, qui correspond à une des formules

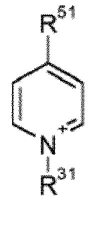
(IX),
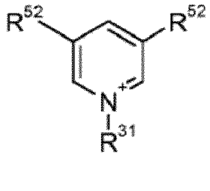
(X) ou
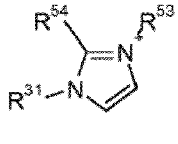
(XI)

dans lesquelles

$R^{31}$ représente un radical alkyle en $C_{14}$ à $C_{22}$ éventuellement ramifié et
$R^{51}$ et $R^{53}$ représentent indépendamment l'un de l'autre un radical alkyle en $C_3$ à $C_8$ éventuellement ramifié, cyclopentyle, cyclohexyle, cycloheptyle ou phényle, et $R^{51}$ représente en outre un radical alcoxy en $C_3$ à $C_8$ éventuellement ramifié ou phénoxy et $R^{53}$ représente en outre phényle ou benzyle,
$R^{52}$ représente un radical alkyle en $C_1$ à $C_4$,

$R^{54}$ représente hydrogène, un radical alkyle en $C_1$ à $C_4$ ou phényle.

Fig. 1

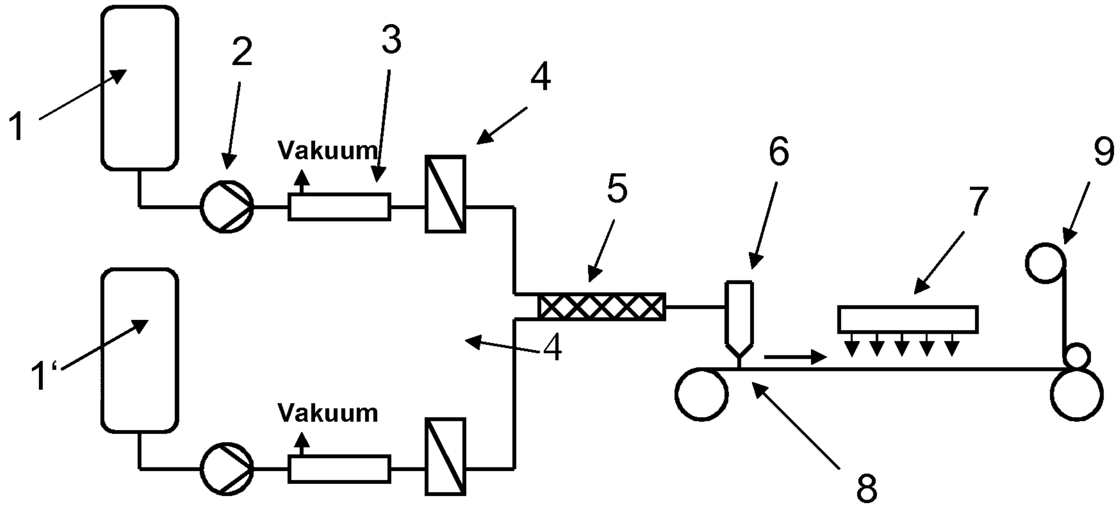
1
2
3
4
Vakuum
5
6
7
9
1'
4
Vakuum
8

Fig. 2

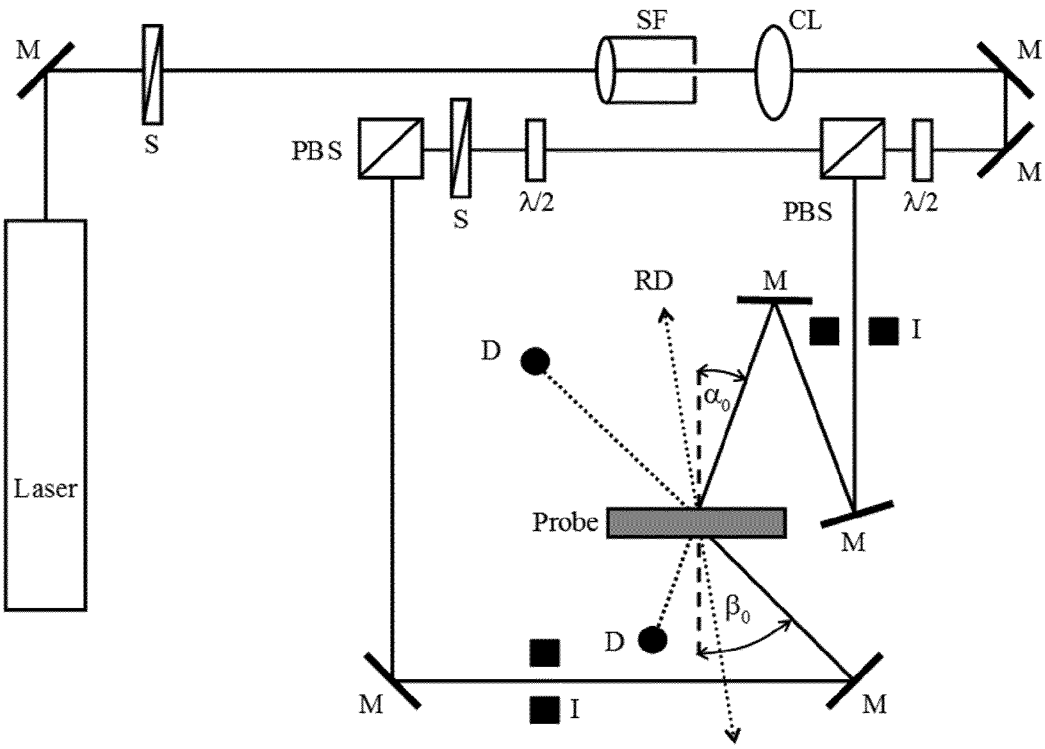
M
S
SF
CL
M
PBS
S
λ/2
PBS
λ/2
M
RD
M
I
D
$\alpha_0$
Laser
Probe
M
D
$\beta_0$
M
I
M

Fig. 3:

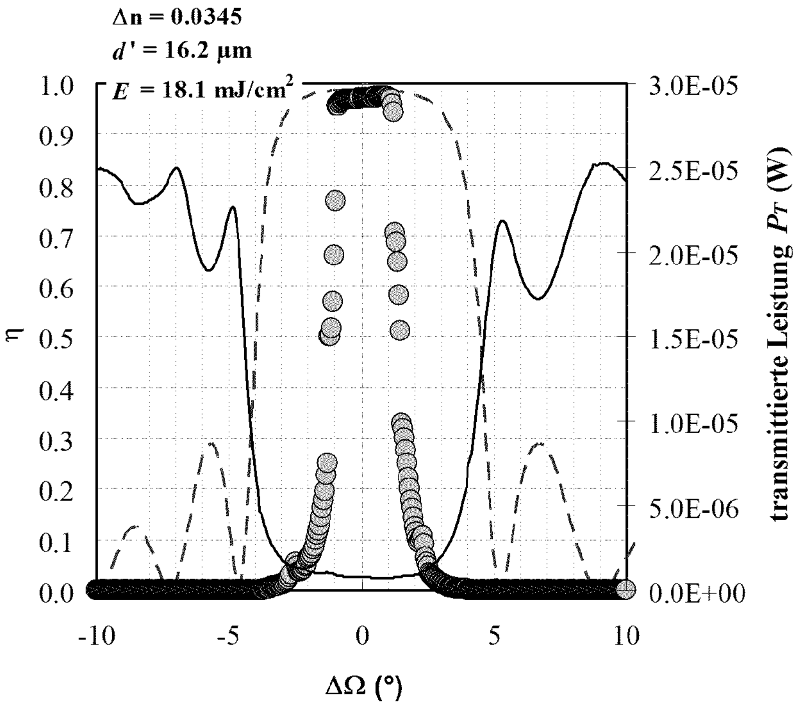
Δn = 0.0345
d' = 16.2 µm
E = 18.1 mJ/cm²
1.0
0.9
0.8
0.7
0.6
η
0.5
0.4
0.3
0.2
0.1
0.0
-10
-5
0
5
10
ΔΩ (°)
3.0E-05
2.5E-05
2.0E-05
1.5E-05
1.0E-05
5.0E-06
0.0E+00
transmittierte Leistung PT (W)

Fig. 4:

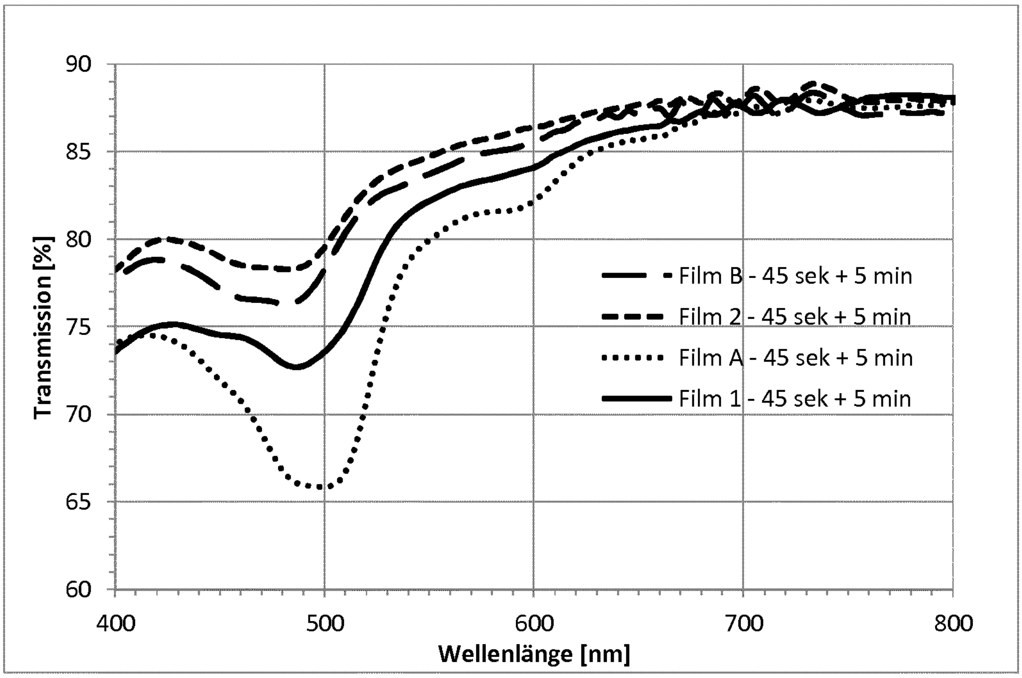
90
85
80
75
70
65
60
Transmission [%]
400
500
600
700
800
Wellenlänge [nm]
Film B - 45 sek + 5 min
Film 2 - 45 sek + 5 min
Film A - 45 sek + 5 min
Film 1 - 45 sek + 5 min

**Fig. 5**

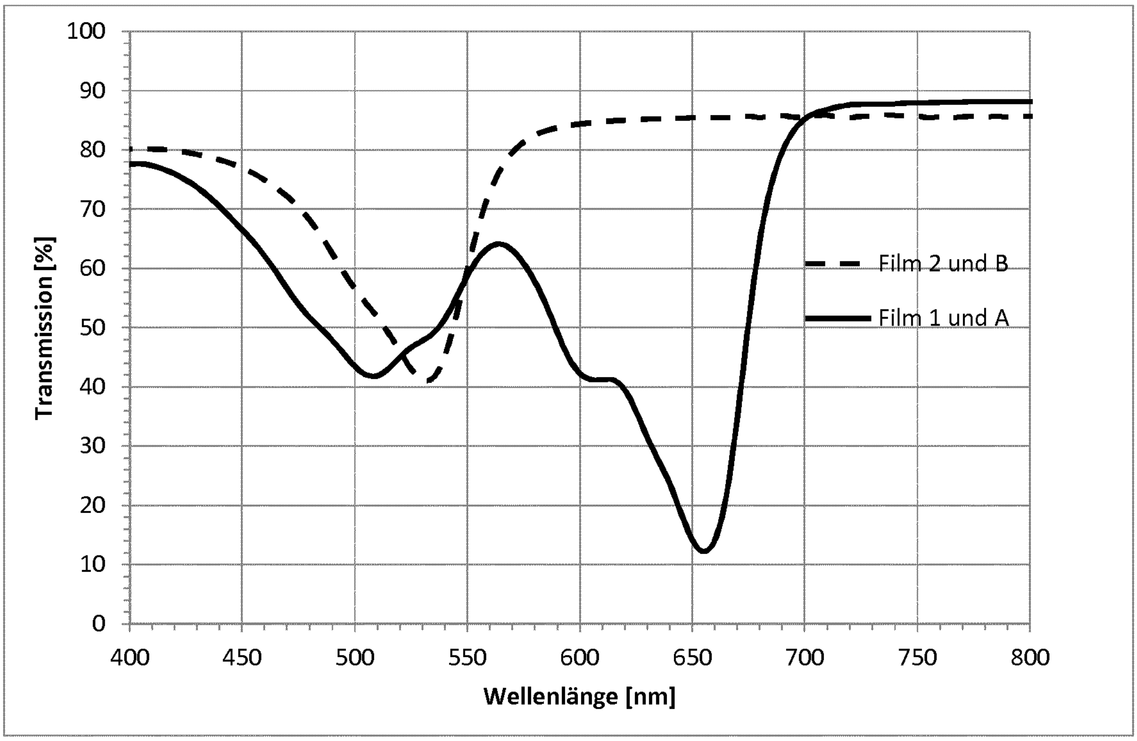
100
90
80
70
60
50
40
30
20
10
0
Transmission [%]
400
450
500
550
600
650
700
750
800
Wellenlänge [nm]
Film 2 und B
Film 1 und A

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- JP 2000267273 B
- EP 2172503 A1
- EP 0223587 A
- WO 2012062655 A
- US 6919159 B
- DE 19648282


### In der Beschreibung aufgeführte Nicht-Patentliteratur


- Azine Dyes. **H. BERNETH.** Ullmann's Encyclopedia of Industrial Chemistry. Wiley-VCH Verlag, 2008
- Methine Dyes and Pigments. **H. BERNETH.** Ullmann's Encyclopedia of Industrial Chemistry. Wiley-VCH Verlag, 2008
- Triarylmethane and Diarylmethane Dyes. **T. GESSNER ; U. MAYER.** Ullmann's Encyclopedia of Industrial Chemistry. Wiley-VCH Verlag, 2000
- *J. Comput. Aid. Mol. Des.,* 2005, vol. 19, 453, http://www.vcclab.org
- Inks & Paints. Chemistry & Technology of UV & EB Formulations For Coatings. SITA Technology, 1991, vol. 3, 61-328
- *IEEE Transactions on Electron Devices,* 1978, vol. 25 (10), ISSN 0018-9383, 1193-1200
- *J. Amer. Chem. Soc.,* 1955, vol. 77, 485
- **H. KOGELNIK.** *The Bell System Technical Journal,* November 1969, vol. 48 (9), 2909, , 2947